(19) Europäisches Patentamt
European Patent Office
Office européen des brevets

(11) Publication number: **0 395 422 B1**

(12) # EUROPEAN PATENT SPECIFICATION

(45) Date of publication of patent specification: **21.06.95**

(51) Int. Cl.[6]: **C07D 231/18**, A01N 43/56, C07D 231/12, C07D 231/16, C07D 231/22, C07D 231/38

(21) Application number: **90304566.4**

(22) Date of filing: **26.04.90**

(54) **Cyclohexane derivatives, its salts and uses as insecticides.**

(30) Priority: **28.04.89 JP 111826/89**

(43) Date of publication of application:
**31.10.90 Bulletin 90/44**

(45) Publication of the grant of the patent:
**21.06.95 Bulletin 95/25**

(84) Designated Contracting States:
**ES GB**

(56) References cited:
**EP-A- 0 207 389**
**EP-A- 0 261 674**
**EP-A- 0 292 122**

**PATENT ABSTRACTS OF JAPAN, vol. 13, no. 61 (C-567)[3409], 10th February 1989**

**PATENT ABSTRACTS OF JAPAN, vol. 13, no. 205 (C-595)[3553], 15th May 1989**

**PATENT ABSTRACTS OF JAPAN, vol. 13, no. 331 (C-622)[3679], 25th July 1989**

(73) Proprietor: **NIHON NOHYAKU CO., LTD.**
**1-2-5, Nihonbashi**
**Chuo-ku**
**Tokyo (JP)**

(72) Inventor: **Hamaguchi, Hiroshi**
**10-1-A-804, Fukakusa Hottacho**
**Fushimi-ku,**
**Kyoto (JP)**
Inventor: **Hino, Tomokazu**
**2-27, Kamihozumi-1-chome**
**Ibaraki-shi (JP)**
Inventor: **Kohno, Eiji**
**7-20, Nigawa Yurinocho**
**Nishinomiya-shi (JP)**
Inventor: **Andoh, Nobuharu**
**2-27, Kamihozumi-1-chome**
**Ibaraki-shi (JP)**
Inventor: **Nishimatsu, Tetsuyoshi**
**3-24-502, Nankadai-3-chome**
**Kawachinagano-shi (JP)**
Inventor: **Kodama, Hiroshi**
**3-21-404, Nankadai-3-chome**
**Kawachinagano-shi (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**PATENT ABSTRACTS OF JAPAN, vol. 13, no. 501 (C-652)[3849], 10th November 1989**

**PATENT ABSTRACTS OF JAPAN, vol. 13, no. 491 (C-650)[3839], 7th November 1989**

(74) Representative: **Votier, Sidney David et al**
**CARPMAELS & RANSFORD**
**43, Bloomsbury Square**
**London WC1A 2RA (GB)**

## Description

BACKGROUND OF THE INVENTION

The present invention relates to a cyclohexane derivative represented by the formula (IA),

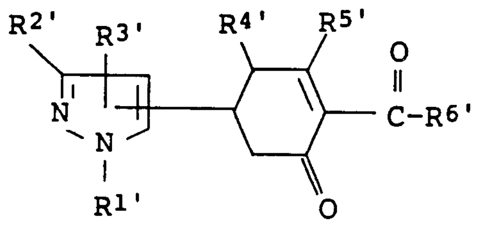

(IA)

wherein $R^{1'}$ represents a hydrogen atom, ethyl group, n-propyl group, n-butyl group, iso-butyl group or sec-butyl group, $R^{2'}$ represents a hydrogen atom, halogen atom or $C_1$-$C_5$ alkyl group, $R^{3'}$ represents a halogen atom, $C_1$-$C_5$ alkyl group, $C_1$-$C_5$ alkoxy group, $C_1$-$C_5$ alkylthio group, $C_1$-$C_5$ alkylsulfinyl group, $C_1$-$C_5$ alkylsulfonyl group or $-N(R^{7'})R^{8'}$ (in which each of $R^{7'}$ and $R^{8'}$, which may be the same or different, represents a hydrogen atom or $C_1$-$C_3$ alkyl group and $R^{7'}$ and $R^{8'}$ may be linked together through 4 to 6 methylene groups), $R^{4'}$ represents a hydrogen atom, $C_1$-$C_5$ alkyl group or $C_2$-$C_5$ alkoxycarbonyl group, $R^{5'}$ represents a hydroxy group, phenylcarbonyloxy group or phenylcarbonyloxy group of which the phenyl ring has 1 to 5 substituents, which may be the same or different, selected from the group consisting of a halogen atom, $C_1$-$C_3$ alkyl group and $C_1$-$C_3$ alkoxy group, and $R^{6'}$ represents a $C_1$-$C_5$ alkyl group, $C_3$-$C_6$ cycloalkyl group or cycloalkyl group substituted with a $C_1$-$C_3$ alkyl group, provided that when $R^{1'}$ is an ethyl group, $R^{2'}$ is a methyl group, $R^{3'}$ is a halogen atom or methylthio group, $R^{4'}$is a hydrogen atom and $R^{5'}$ is a hydroxy group at the same time, $R^{6'}$ is a group other than an ethyl and n-propyl groups,

its salts, insecticides characterized by containing as an active ingredient a cyclohexane derivative represented by the formula (IB),

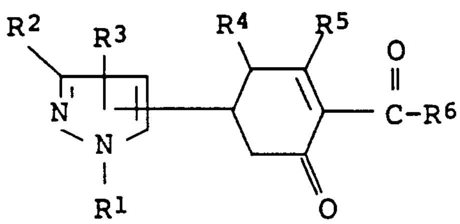

(IB)

wherein $R^1$ represents a hydrogen atom, $C_1$-$C_{16}$ alkyl group, $C_1$-$C_5$ haloalkyl group, $C_2$-$C_5$ alkenyl group, $C_2$-$C_5$ alkoxyalkyl group, phenyl group, phenyl group having 1 to 5 substituents, which may be the same or different, selected from the group consisting of a halogen atom, $C_1$-$C_3$ alkyl group and $C_1$-$C_3$ alkoxy group, benzyl group or benzyl group having 1 to 5 substituents, which may be the same or different, selected from the group consisting of a halogen atom, $C_1$-$C_3$ alkyl group and $C_1$-$C_3$ alkoxy group, $R^2$ represents a hydrogen atom, halogen atom, $C_1$-$C_5$ alkyl group, $C_1$-$C_5$ haloalkyl group, phenyl group or phenyl group having 1 to 5 substituents, which may be the same or different, selected from the group consisting of a halogen atom, $C_1$-$C_3$ alkyl group and $C_1$-$C_3$ alkoxy group, $R^3$ represents a hydrogen atom, halogen atom, $C_1$-$C_5$ alkyl group, $C_1$-$C_5$ haloalkyl group, $C_1$-$C_5$ alkoxy group, $C_1$-$C_5$ haloalkoxy group, $C_1$-$C_5$ alkylthio group, $C_1$-$C_5$ haloalkylthio group, $C_1$-$C_5$ alkylsulfinyl group, $C_1$-$C_5$ haloalkylsulfinyl group, $C_1$-$C_5$ alkylsulfonyl group, $C_1$-$C_5$ haloalkylsulfonyl group, phenoxy group, phenoxy group having 1 to 5 substituents, which may be the same or different, selected from the group consisting of a halogen atom, $C_1$-$C_3$ alkyl group and $C_1$-$C_3$ alkoxy group, phenylthio group, phenylthio group having 1 to 5 substituents, which may be the same or different, selected from the group consisting of a halogen atom, $C_1$-$C_3$ alkyl group and $C_1$-$C_3$ alkoxy group, $-N(R^7)R^8$ (in which each of $R^7$ and $R^8$, which may be the same or different, represents a hydrogen atom or $C_1$-$C_3$ alkyl group, and $R^7$ and $R^8$ may be linked together to form 4 to 6 methylene groups), or $C_2$-$C_5$ alkylcarbonyloxy group, $R^4$ represents a hydrogen atom, $C_1$-$C_5$ alkyl group or $C_2$-$C_5$ alkoxycarbonyl

group, $R^5$ represents a hydroxy group, halogen atom, $C_1$-$C_5$ alkylthio group, alkenylthio group containing up to 5 carbon atoms, -N($R^9$)$R^{10}$ (in which each of $R^9$ and $R^{10}$, which may be the same or different, represents a hydrogen atom, $C_1$-$C_3$ alkyl group or alkenyl group containing up to 3 carbon atoms), $C_2$-$C_8$ alkylcarbonyloxy group, $C_3$-$C_6$ cycloalkylcarbonyloxy group, phenylcarbonyloxy group, or phenylcarbonyloxy group of which the phenyl ring has 1 to 5 substituents, which may be the same or different, selected from the group consisting of a halogen atom, $C_1$-$C_3$ alkyl group and $C_1$-$C_3$ alkoxy group, and $R^6$ represents a $C_1$-$C_{18}$ alkyl group, $C_3$-$C_6$ cycloalkyl group, cycloalkyl group having substituents, which may be the same or different, selected from the group consisting of a halogen atom and $C_1$-$C_3$ alkyl group, $C_2$-$C_{17}$ alkenyl group, $C_2$-$C_8$ alkoxyalkyl group, $C_2$-$C_8$ alkylthioalkyl group, phenoxyalkyl group, or phenoxyalkyl group of which the phenyl ring has 1 to 5 substituents, which may be the same or different, selected from the group consisting of a halogen atom, $C_1$-$C_3$ alkyl group and $C_1$-$C_3$ alkoxy group, provided that when $R^1$ = H, $R^2$ = methyl, $R^3$ = 5-Cl $R^4$ = H and $R^5$ = OH then $R^6$ may be benzyl, or its salts and an insecticidal method with these cyclohexane derivatives or their salts.

## DETAILED DESCRIPTION OF PREFERRED EMBODIMENTS

JP-A-63 253 068 and EP-A-0 292 122 disclose that some compounds of the cyclohexane derivatives represented by the formula (IB) can be used as intermediates for the preparation of herbicides.

JP-A-1 25 762, JP-A- 1 110 674 and EP-A-0 207 389 disclose compounds of formula (IB) which themselves have herbicidal or plant growth regulating activity.

None of these documents refer to cyclohexane derivatives represented by the formula (IA) or their salts.

Neither do they disclose, or suggest, that cyclohexane derivatives represented by the formulae (IA) and (IB) or their salts possess insecticidal activity at useful levels.

The present inventors have extensively studied to develop a novel insecticide, and as a result, have found that the cyclohexane derivatives represented by the formula (IA) or their salts are a novel compound unknown to the literatures, and that the cyclohexane derivatives represented by the formula (IB) or their salts including the cyclohexane derivatives represented by the formula (IA) or their salts have a remarkable insecticidal action. The present inventors thus completed the present invention.

The cyclohexane derivatives of the present invention represented by the formula (IA) or their salts have the tautomers shown below, and the present invention includes these tautomers. When $R^{5'}$ is a hydroxy group:

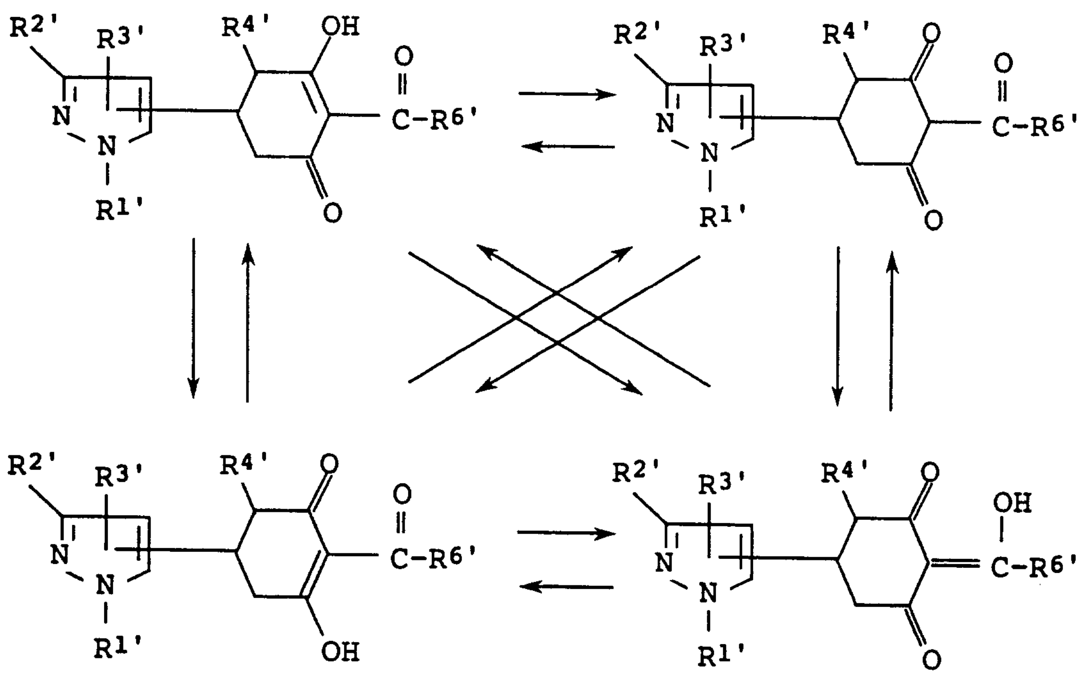

When $R^{5'}$ is a group other than a hydroxy group:

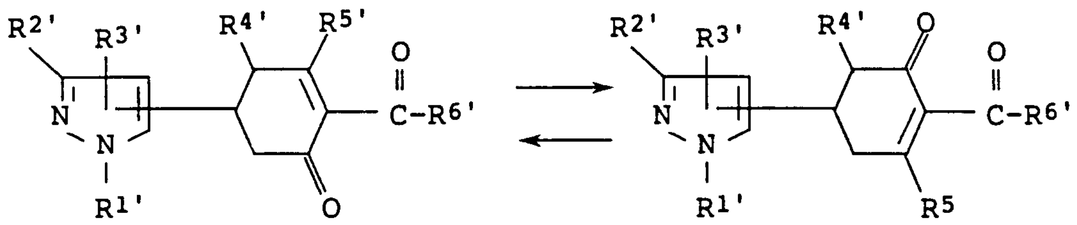

wherein $R^{1'}$, $R^{2'}$, $R^{3'}$, $R^{4'}$ and $R^{6'}$ are as defined above.

The cyclohexane derivatives represented by the formula (IB) or their salts also have tautomers.

The salts of the cyclohexane derivatives represented by the formula (IA) or (IB) include inorganic salts and organic salts. The inorganic salts include salts with an inorganic acid (e.g. hydrochloric acid, sulfuric acid, nitric acid) and salts with an alkali metal (e.g. sodium, potassium), an alkaline earth metal (e.g. calcium, magnesium, barium) or a metal (e.g. aluminum, tin, iron, nickel, zinc). The organic salts include salts with an organic base [e.g. diethylamine, triethanolamine, triethylamine, dimethylaminopyridine, pyrrole, morpholine, DBU (1,8-diazabicyclo[5.4.0]-7-undecene)], and salts with an organic acid (e.g. p-toluenesulfonic acid, trifluoroacetic acid, oxalic acid). The present invention, however, is not limited to these salts.

As a typical manufacturing method for the cyclohexane derivatives of the present invention represented by the formula (IA) or (IB) or their salts, there are mentioned those which are shown, for example, by the following flow sheets.

In explaining the manufacturing method, the one for the cyclohexane derivatives represented by the formula (IB) or their salts will be mentioned as an example because they include the cyclohexane derivatives represented by the formula (IA) or their salts.

Method (1) :

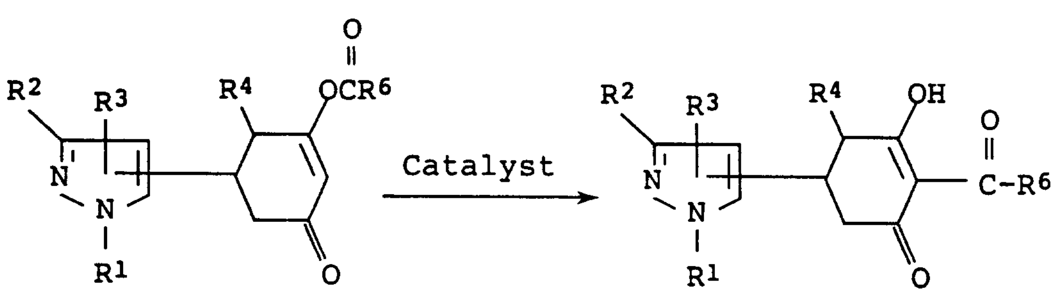


(II) (IB-1)

wherein $R^1$, $R^2$, $R^3$, $R^4$ and $R^6$ are as defined above. The cyclohexane derivative of the present invention represented by the formula (IB) wherein $R^5$ is a hydroxy group or its salts can be produced by the rearrangement of a compound represented by the formula (II) in the presence of an inert solvent and a catalyst.

As the inert solvent usable in this reaction, any of those which do not remarkably disturb the progress of this reaction may be used. For example, there are mentioned alcohols (e.g. methanol, ethanol, propanol, cyclohexanol), chlorinated hydrocarbons (e.g. dichloromethane, chloroform, carbon tetrachloride), aromatic hydrocarbons (e.g. benzene, toluene, xylene), esters (e.g. ethyl acetate), nitriles (e.g. acetonitrile), cyclic ethers (e.g. dioxane, tetrahydrofuran), etc.

As the catalyst usable in this reaction, there are mentioned for example 4-N,N-dimethylaminopyridine, acetone cyanohydrin, potassium cyanide, sodium cyanide, etc. The amount of the catalyst used is properly selected from a range of 0.01 to 10 moles, preferably 0.1 to 1 mole based on 1 mole of the compound of the formula (II).

The reaction temperature of this reaction is properly selected from a range of room temperature to the boiling point of the inert solvent used. Preferably, this reaction is carried out under heating. The reaction time depends upon the reaction amount, reaction temperature, etc., but it is selected from a range of several minutes to 48 hours. After completion of the reaction, the desired cyclohexane derivative can be separated by the usual methods, for example by adjusting the pH with an acid, isolating the desired product by extraction with a solvent and if necessary purifying the product by column chromatography, recrystallization, etc.

Method (2) :

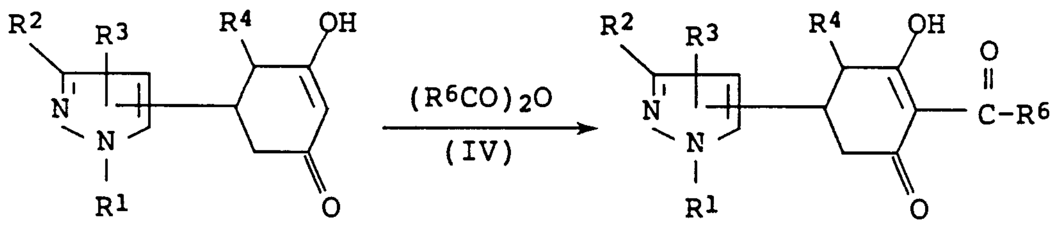


(III) (IB-1)

wherein $R^1$, $R^2$, $R^3$, $R^4$ and $R^6$ are as defined above. The cyclohexane derivative represented by the formula (IB) wherein R is a hydroxy group or its salts can be produced by an alternative method of reacting a compound represented by the formula (III) with an acid anhydride represented by the formula (IV) in the presence of an inert solvent and a base.

As the inert solvent usable in this reaction, there are mentioned for example the inert solvents used in Method (1). In addition, the acid anhydride represented by the formula (IV) also can be used as the solvent.

As the base used in Method (2), inorganic and organic bases can be used. The inorganic base includes for example the hydroxides, carbonates and alcoholates of an alkali or alkaline earth metal (e.g. sodium, potassium, magnesium, calcium). The organic base includes for example tertiary amines (e.g. triethylamine), pyridine, DBU (1,8-diazabicyclo[5.4.0]-7-undecene), etc.

This reaction is carried out using equimolar amounts of the compound represented by the formula (III) and the acid anhydride represented by the formula (IV). However, the acid anhydride may be used in excess.

The amount of the base used is properly selected from a range of an equimolar amount to excess molar amounts based on the compound represented by the formula (III).

The reaction temperature is selected from a range of 10 °C to the boiling point of the inert solvent used.

The reaction time depends upon the reaction amount, reaction temperature, etc., but it is properly selected from a range of several minutes to 48 hours.

After completion of the reaction, the desired cyclohexane derivative can be separated from the reaction solution by the usual method, for example by isolating the desired product by extraction with a solvent and if necessary purifying the product by column chromatography, recrystallization, etc.

Method (3) :

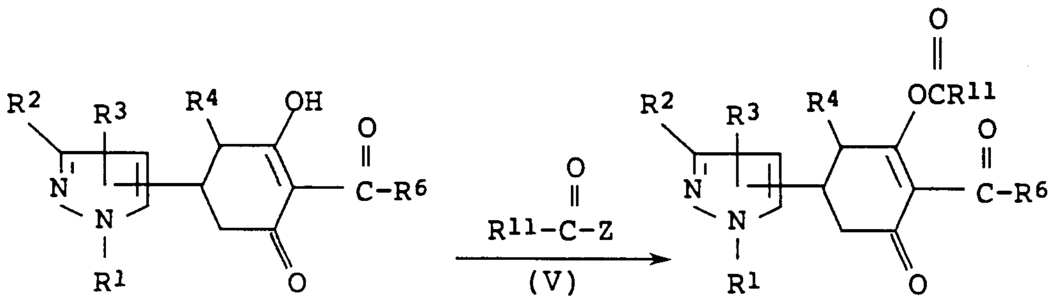

(IB-1) (IB-2)

wherein $R^1$, $R^2$, $R^3$, $R^4$ and $R^6$ are as defined above, $R^{11}$ represents a $C_2$-$C_7$ alkyl group, $C_3$-$C_6$ cycloalkyl group, phenyl group or phenyl group having 1 to 5 substituents, which may be the same or different, selected from the group consisting of a halogen atom, $C_1$-$C_3$ alkyl group and $C_1$-$C_3$ alkoxy group, and Z represents a halogen atom.

The cyclohexane derivative represented by the formula (IB) wherein $R^5$ is an acyloxy group can be produced by reacting the cyclohexane derivative of the formula (IB-1) obtained by the foregoing Method (1) or (2) with a halide represented by the formula (V) in the presence of an inert solvent and a base.

Since this reaction is an equimolar reaction, the halide represented by the formula (V) is used in an amount equimolar with the cyclohexane derivative represented by the formula (IB-1). However, the halide may be used in excess.

As the inert solvent usable in this reaction, any of those which do not remarkably disturb the progress of this reaction may be used. For example, there are mentioned chlorinated hydrocarbons (e.g. dichloromethane, chloroform, carbon tetrachloride), aromatic hydrocarbons (e.g. benzene, toluene, xylene), esters (e.g. ethyl acetate), nitriles (e.g. acetonitrile, benzonitrile), chain-like ethers (e.g. methyl cellosolve, diethyl ether), cyclic ethers (e.g. dioxane, tetrahydrofuran), sulfolane, dimethyl sulfone, dimethyl sulfoxide, water, etc. These inert solvents may be used alone or in combination.

As the base usable in this reaction, there are mentioned for example the inorganic and organic bases used in the foregoing Method (2).

The base is used in an amount properly selected from a range of an equimolar amount to excess molar amounts based on the cyclohexane derivative represented by the formula (IB-1).

The reaction temperature is selected from a range of 0 °C to the boiling point of the inert solvent used.

The reaction time depends upon the reaction amount, reaction temperature, etc., but it is properly selected from a range of several minutes to 48 hours.

After completion of the reaction, the cyclohexane derivative represented by the formula (IB-2) can be separated from the reaction solution by the usual method, for example by isolating the desired product by extraction with a solvent and if necessary purifying the product by column chromatography, recrystallization, etc.

Method (4) :

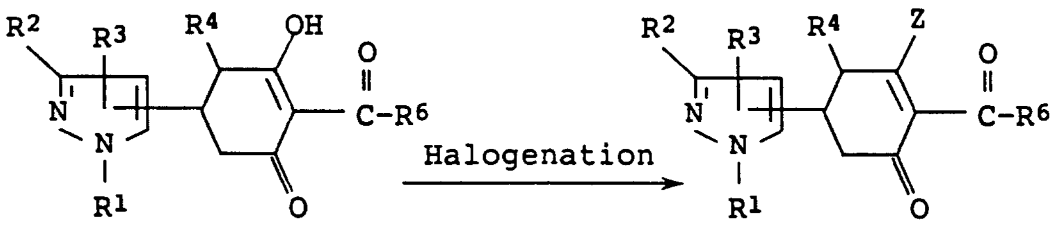


(IB-1) (IB-3)

wherein $R^1$, $R^2$, $R^3$, $R^4$ and $R^6$ are as defined above.

The cyclohexane derivative represented by the formula (IB-3) can be produced by halogenating the cyclohexane derivative represented by the formula (IB-1) with a halogenating agent in the presence or absence of an inert solvent.

As the halogenating agent used in this reaction, halogenating agents such as phosphorus trichloride, phosphorus pentachloride, phosphorus tribromide, thionyl chloride, oxalyl chloride, etc. may be used. The halogenating agent is used in a range of an equimolar amount to excess molar amounts based on the cyclohexane derivative represented by the formula (IB-1). Preferably, the halogenating agent is used in excess. Also, this reaction can be carried out in the absence of the inert solvent by using the halogenating agent in a large excess.

As the inert solvent usable in this reaction, any of those which do not remarkably disturb the progress of this reaction may be used. For example, there are mentioned chlorinated hydrocarbons such as dichloromethane, chloroform, carbon tetrachloride, etc.

The reaction temperature is selected from a range of room temperature to the boiling point of the inert solvent used.

The reaction time depends upon the reaction amount, reaction temperature, etc., but it is properly selected from a range of several minutes to 48 hours.

After completion of the reaction, the cyclohexane derivative represented by the formula (IB-3) can be separated from the reaction solution by the usual method, for example by isolating the desired product by removal of the solvent or extraction with a solvent and if necessary purifying the product by column chromatography, recrystallization, etc.

Method (5) :

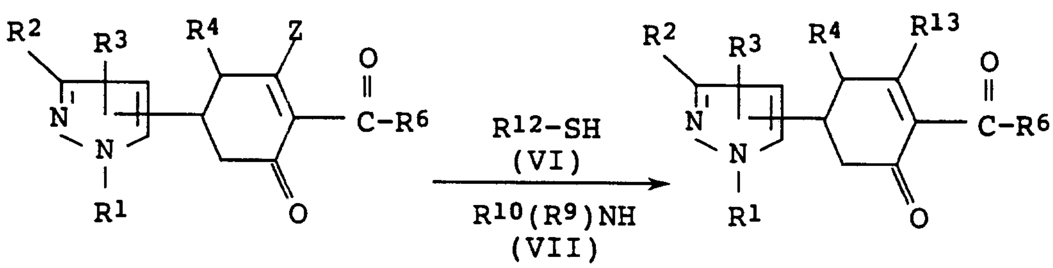


(IB-3) (IB-4)

wherein $R^1$, $R^2$, $R^3$, $R^4$, $R^6$, $R^9$, $R^{10}$ and Z are as defined above, $R^{12}$ represents a $C_1$-$C_5$ alkyl group or $C_1$-$C_5$ alkenyl group, and $R^{13}$ represents a $C_1$-$C_5$ alkylthio group, $C_1$-$C_5$ alkenylthio group or $-N(R^9)R^{10}$ (in which $R^9$ and $R^{10}$ are as defined above).

The cyclohexane derivative represented by the formula (IB-4) can be produced by reacting the cyclohexane derivative represented by the formula (IB-3) with a thiol represented by the formula (VI) or an

amine represented by the formula (VII) in the presence of an inert solvent and a base.

That is, the cyclohexane derivative represented by the formula (IB-4) can be produced by carrying out this reaction in the same manner as in Method (3).

Method (6) :

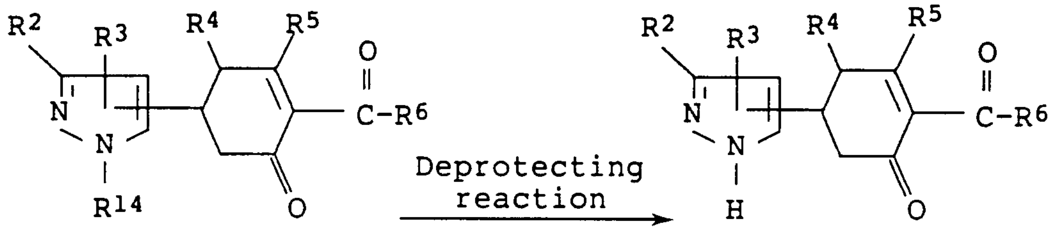


(II-1) (IB-5)

wherein $R^2$, $R^3$, $R^4$, $R^5$, and $R^6$ are as defined above, $R^{14}$ represents a lower acyl, lower alkoxycarbonyl, benzyl, sulfonyl, alkoxymethyl, alkenyl or trimethylsilyl group.

The cyclohexane derivative represented by the formula (IB-5) can be produced by reacting a cyclohexane represented by the formula (II-1) with a deprotecting agent in the presence of an inert solvent to remove a protective group $R^{14}$.

As the deprotecting agent usable in the present invention, there are mentioned for example reducing agents (e.g. $H_2$/palladium carbon, zinc), organic and inorganic acids (e.g. acetic acid, trifluoroacetic acid, p-toluenesulfonic acid, hydrochloric acid, hydrobromic acid, sulfuric acid), inorganic bases (e.g. hydroxides and carbonates of an alkali or alkaline earth metal), organic bases ( e.g. triethylamine, DBU) and potassium fluoride, tetrabutylammonium fluoride, etc.

As the inert solvent usable in this reaction, there are mentioned the inert solvents usable in Method (1), water and mixtures of the both.

The reaction temperature is selected from a range of 0 °C to the boiling point of the inert solvent used.

The reaction time depends upon the reaction temperature, reaction scale, etc., but it is selected from a range of several minutes to 48 hours.

After completion of the reaction, the cyclohexane derivative represented by the formula (IB-5) can be separated by the same treatment as in Method (1).

A cyclohexane represented by the formula (II-1) can be produced in the same manner as in the production of a cyclohexane represented by the formula (II).

Method (7) :

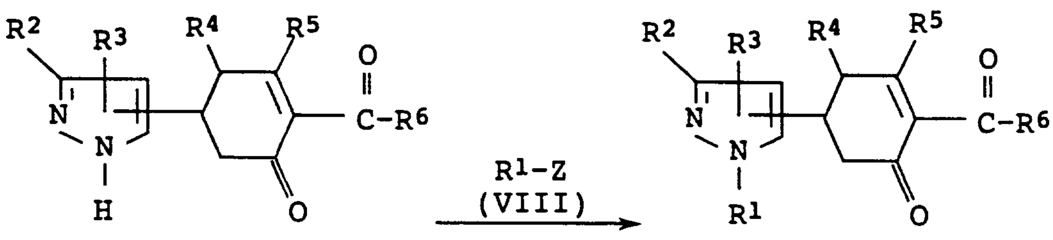


(IB-5) (IB)

wherein $R^1$, $R^2$, $R^3$, $R^4$, $R^5$, $R^6$ and Z are as defined above. The cyclohexane derivative represented by the formula (IB) can be produced by reacting the cyclohexane derivative represented by the formula (IB-5) with a halide represented by the formula (VIII) in the presence of an inert solvent and a base.

The cyclohexane derivative represented by the formula (IB-5) can be produced by carrying out this reaction in the same manner as in Method (3).

Method (8) :

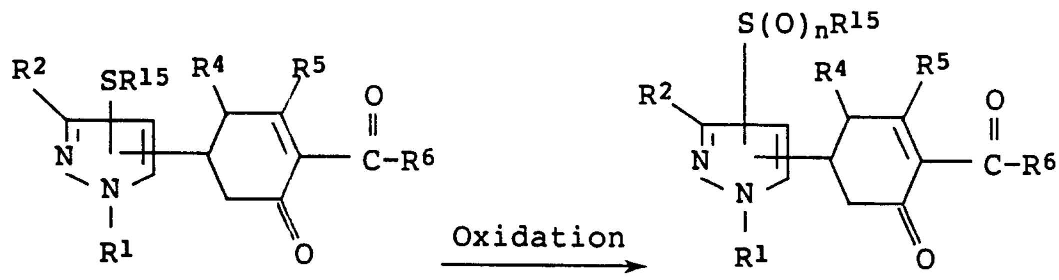


(IB-6) (IB-7)

wherein $R^1$, $R^2$, $R^4$, $R^5$, and $R^6$ are as defined above, $R^{15}$ represents a $C_1$-$C_5$ alkyl or $C_1$-$C_5$ haloalkyl group, and n represents an integer of 1 to 2.

The cyclohexane derivative represented by the formula (IB-7) can be produced by oxidizing the cyclohexane derivative represented by the formula (IB-6) with a suitable oxidizing agent in the presence of an inert solvent.

As the inert solvent usable in this reaction, there are mentioned the inert solvents usable in Method (1).

As the oxidizing agent usable in this reaction, there are mentioned for example nitric acid, hydrogen peroxide, perbenzoic acid, m-chloroperbenzoic acid, sodium metaperiodate, potassium peroxymonosulfate, etc. Since this reaction is an equimolar reaction, the oxidizing agent is used in an equimolar amount, but it may be used in excess.

The reaction temperature is selected from a range of 0 °C to the boiling point of the inert solvent used. Preferably, however, it is selected from a range of 10 °C to 150 °C.

The reaction time depends upon the reaction scale, reaction temperature, etc., but it is selected from a range of several minutes to 48 hours.

After completion of the reaction, the cyclohexane derivative represented by the formula (IB-7) can be separated in the same manner as in Method (1).

(9) Salts :

The salts of the cyclohexane derivatives represented by the formula (IB) can be produced by treating the cyclohexane derivatives produced in Methods (1) to (8) with an inorganic acid, inorganic base, organic acid, organic base, alkali metal or metal salt.

It is a matter of course that the cyclohexane derivatives represented by the formula (IA) or their salts can be produced by the foregoing Methods (1) to (9).

Typical examples of the cyclohexane derivatives of the present invention represented by the formulae (IA) and (IB) and their salts will be shown in Tables 1 and 2, but the present invention is not limited to these examples.

Formulae (IA) and (IB):

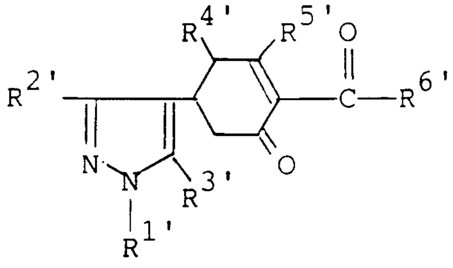

(IA)

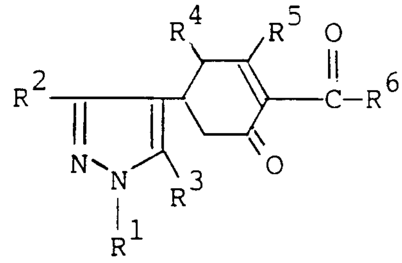

(IB)

Table 1

| No. | $R^{1}$'($R^{1}$) | $R^{2}$'($R^{2}$) | $R^{3}$'($R^{3}$) | $R^{4}$'($R^{4}$) | $R^{5}$'($R^{5}$) | $R^{6}$'($R^{6}$) | Physical property |
|---|---|---|---|---|---|---|---|
| 1 | H | $CH_3$ | 5-$SCH_3$ | H | OH | c-$C_3H_5$ | mp. 165.0°C |
| 2 | H | $CH_3$ | 5-$SOCH_3$ | H | OH | c-$C_3H_5$ | mp. 72.0°C |
| 3 | H | $CH_3$ | 5-$CH_3$ | H | OH | $C_2H_5$ | mp. 75.0°C |
| 4 | H | $CH_3$ | 5-$CH_3$ | H | OH | c-$C_3H_5$ | mp. 188.0 - 191.3°C |
| 5 | H | $CH_3$ | 5-$CH_3$ | H | OH | c-$C_3H_5$ | mp. 280.9 - 288.2°C (HCl salt) |
| 6 | H | $CH_3$ | 5-$CH_3$ | H | OH | c-$C_3H_5$ | mp>300°C (Na salt) |
| 7 | $CH_3$ | H | 5-Cl | H | OH | $C_2H_5$ | mp. 60.2 - 64.9°C |

- Cont'd -

Table 1 (Cont'd)

| | | | | | | | |
|---|---|---|---|---|---|---|---|
| 8 | $CH_3$ | H | 5-Cl | H | OH | c-$C_3H_5$ | nD 1.5788 (20.5°C) |
| 9 | $CH_3$ | H | 5-Cl | H | OH | 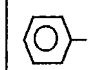 $CH_2$ | nD 1.5811 (28.1°C) |
| 10 | $CH_3$ | H | 5-F | H | OH | $C_2H_5$ | nD 1.5297 (28.5°C) |
| 11 | $CH_3$ | H | 5-$SCH_3$ | H | OH | $C_2H_5$ | nD 1.5730 (28.0°C) |
| 12 | $CH_3$ | H | 5-$SCH_3$ | H | OH | n-$C_3H_7$ | nD 1.5687 (23.8°C) |
| 13 | $CH_3$ | H | 5-$SCH_3$ | H | OH | c-$C_3H_5$ | nD 1.5793 (21.7°C) |
| 14 | $CH_3$ | H | 5-$SOCH_3$ | H | OH | $C_2H_5$ | nD 1.5688 (15.9°C) |

- Cont'd -

Table 1 (Cont'd)

| | | | | | | | |
|---|---|---|---|---|---|---|---|
| 15 | $CH_3$ | H | 5-$SOCH_3$ | H | OH | c-$C_3H_5$ | nD 1.5823 (22.5°C) |
| 16 | $CH_3$ | Cl | 5-$SCH_3$ | H | OH | c-$C_3H_5$ | Viscous product |
| 17 | $CH_3$ | Cl | 5-$SOCH_3$ | H | OH | c-$C_3H_5$ | Viscous product |
| 18 | $CH_3$ | $CH_3$ | 5-H | H | OH | $CH_3$ | mp. 91.9 - 93.0°C |
| 19 | $CH_3$ | $CH_3$ | 5-H | H | OH | $C_2H_5$ | mp. 90.0°C |
| 20 | $CH_3$ | $CH_3$ | 5-H | H | OH | n-$C_3H_7$ | mp. 59.6 - 62.3°C |
| 21 | $CH_3$ | $CH_3$ | 5-Cl | H | OH | $CH_3$ | nD 1.5551 (25.1°C) |

- Cont'd -

Table 1 (Cont'd)

| | | | | | | | |
|---|---|---|---|---|---|---|---|
| 22 | $CH_3$ | $CH_3$ | 5-Cl | H | OH | $C_2H_5$ | nD 1.5462 (25.1°C) |
| 23 | $CH_3$ | $CH_3$ | 5-Cl | H | OH | n-$C_3H_7$ | mp. 56.0°C |
| 24 | $CH_3$ | $CH_3$ | 5-Cl | H | OH | c-$C_3H_5$ | mp. 104.5 - 106.0°C |
| 25 | $CH_3$ | $CH_3$ | 5-Cl | H | OH | i-$C_3H_7$ | mp. 86.5°C |
| 26 | $CH_3$ | $CH_3$ | 5-Cl | H | OH | n-$C_9H_{19}$ | nD 1.5332 (16.5°C) |
| 27 | $CH_3$ | $CH_3$ | 5-Cl | H | OH | n-$C_{17}H_{35}$ | mp. 45 - 46°C |
| 28 | $CH_3$ | $CH_3$ | 5-Cl | H | OH | $CH_3(CH_2)_7CH=CH(CH_2)_7$ | nD 1.5243 (21.2°C) |

- Cont'd -

Table 1 (Cont'd)

| | | | | | | | |
|---|---|---|---|---|---|---|---|
| 29 | $CH_3$ | $CH_3$ | 5-Cl | $CH_3$ | OH | $C_2H_5$ | nD 1.5231 (19.8°C) |
| 30 | $CH_3$ | $CH_3$ | 5-Cl | $CH_3$ | OH | n-$C_3H_7$ | nD 1.5065 (19.0°C) |
| 31 | $CH_3$ | $CH_3$ | 5-Cl | $CO_2CH_3$ | OH | $C_2H_5$ | Viscous product |
| 32 | $CH_3$ | $CH_3$ | 5-Cl | $CO_2CH_3$ | OH | n-$C_3H_7$ | Viscous product |
| 33 | $CH_3$ | $CH_3$ | 5-Cl | $CO_2C_2H_5$ | OH | $CH_3$ | nD 1.5310 (16.0°C) |
| 34 | $CH_3$ | $CH_3$ | 5-Cl | $CO_2C_2H_5$ | OH | $C_2H_5$ | nD 1.5273 (18.6°C) |
| 35 | $CH_3$ | $CH_3$ | 5-Cl | $CO_2C_2H_5$ | OH | n-$C_3H_7$ | nD 1.5272 (16.0°C) |

- Cont'd -

Table 1 (Cont'd)

| | | | | | | | |
|---|---|---|---|---|---|---|---|
| 36 | $CH_3$ | $CH_3$ | 5-F | H | OH | $C_2H_5$ | nD 1.5403 (18.6°C) |
| 37 | $CH_3$ | $CH_3$ | 5-F | H | OH | n-$C_3H_7$ | nD 1.5317 (19.0°C) |
| 38 | $CH_3$ | $CH_3$ | 5-$CH_3$ | H | OH | $CH_3$ | nD 1.5265 (20.7°C) |
| 39 | $CH_3$ | $CH_3$ | 5-$CH_3$ | H | OH | $C_2H_5$ | mp. 112.0°C |
| 40 | $CH_3$ | $CH_3$ | 5-$CH_3$ | H | OH | n-$C_3H_7$ | mp. 105.5°C |
| 41 | $CH_3$ | $CH_3$ | 5-$CH_3$ | H | OH | c-$C_3H_5$ | nD 1.5622 (12.9°C) |
| 42 | $CH_3$ | $CH_3$ | 5-$OCH_3$ | H | OH | $CH_3$ | nD 1.5183 (20.8°C) |

- Cont'd -

Table 1 (Cont'd)

| | | | | | | | |
|---|---|---|---|---|---|---|---|
| 43 | $CH_3$ | $CH_3$ | 5-$OCH_3$ | H | OH | $C_2H_5$ | mp. 64.3 - 65.2°C |
| 44 | $CH_3$ | $CH_3$ | 5-$OCH_3$ | H | OH | n-$C_3H_7$ | mp. 73.2°C |
| 45 | $CH_3$ | $CH_3$ | 5-$OC_2H_5$ | H | OH | c-$C_3H_5$ | nD 1.5448 (25.4°C) |
| 46 | $CH_3$ | $CH_3$ | 5-O- 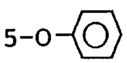 | H | OH | $C_2H_5$ | nD 1.5587 (11.9°C) |
| 47 | $CH_3$ | $CH_3$ | 5-O- 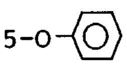 | H | OH | n-$C_3H_7$ | nD 1.5509 (11.9°C) |
| 48 | $CH_3$ | $CH_3$ | 5-O- 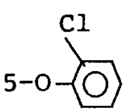 | H | OH | $C_2H_5$ | nD 1.5600 (23.5°C) |
| 49 | $CH_3$ | $CH_3$ | 5-O- 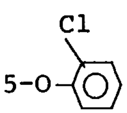 | H | OH | n-$C_3H_7$ | nD 1.5550 (24.0°C) |

- Cont'd -

Table 1 (Cont'd)

| | | | | | | | |
|---|---|---|---|---|---|---|---|
| 50 | $CH_3$ | $CH_3$ | 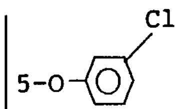 | H | OH | $C_2H_5$ | nD 1.5075 (23.0°C) |
| 51 | $CH_3$ | $CH_3$ | 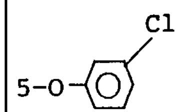 | H | OH | $n-C_3H_7$ | nD 1.5683 (23.0°C) |
| 52 | $CH_3$ | $CH_3$ | 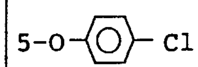 | H | OH | $C_2H_5$ | nD 1.5684 (17.7°C) |
| 53 | $CH_3$ | $CH_3$ | 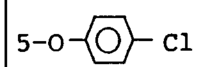 | H | OH | $n-C_3H_7$ | nD 1.5614 (18.0°C) |
| 54 | $CH_3$ | $CH_3$ | 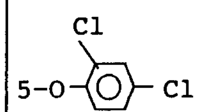 | H | OH | $C_2H_5$ | Viscous product |
| 55 | $CH_3$ | $CH_3$ | 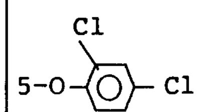 | H | OH | $n-C_3H_7$ | nD 1.5528 (25.9°C) |

- Cont'd -

Table 1 (Cont'd)

| | | | | | | | |
|---|---|---|---|---|---|---|---|
| 56 | $CH_3$ | $CH_3$ | 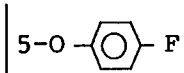 | H | OH | n-$C_3H_7$ | nD 1.5413 (24.1°C) |
| 57 | $CH_3$ | $CH_3$ | 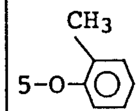 | H | OH | $C_2H_5$ | nD 1.5539 (26.2°C) |
| 58 | $CH_3$ | $CH_3$ | 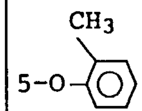 | H | OH | n-$C_3H_7$ | nD 1.5389 (26.2°C) |
| 59 | $CH_3$ | $CH_3$ | 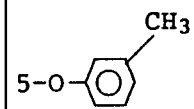 | H | OH | $CH_3$ | nD 1.5990 (17.5°C) |
| 60 | $CH_3$ | $CH_3$ | 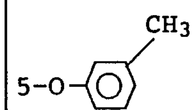 | H | OH | $C_2H_5$ | nD 1.5998 (18.2°C) |
| 61 | $CH_3$ | $CH_3$ | 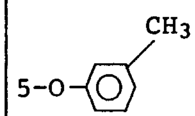 | H | OH | n-$C_3H_7$ | nD 1.5493 (17.7°C) |

- Cont'd -

Table 1 (Cont'd)

| | | | | | | | |
|---|---|---|---|---|---|---|---|
| 62 | $CH_3$ | $CH_3$ | 5-O- 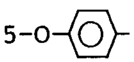 $CH_3$ | H | OH | $CH_3$ | nD 1.5705 (23.0°C) |
| 63 | $CH_3$ | $CH_3$ | 5-O- 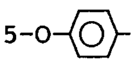 $CH_3$ | H | OH | $C_2H_5$ | nD 1.5538 (24.6°C) |
| 64 | $CH_3$ | $CH_3$ | 5-O- 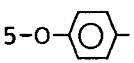 $CH_3$ | H | OH | n-$C_3H_7$ | nD 1.5446 (24.7°C) |
| 65 | $CH_3$ | $CH_3$ | 5-O- 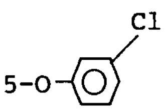 | $CO_2C_2H_5$ | OH | $C_2H_5$ | nD 1.5557 (17.0°C) |
| 66 | $CH_3$ | $CH_3$ | 5-$SCH_3$ | H | OH | $CH_3$ | mp. 107.1°C |
| 67 | $CH_3$ | $CH_3$ | 5-$SCH_3$ | H | OH | $C_2H_5$ | mp. 67.2 - 67.5°C |
| 68 | $CH_3$ | $CH_3$ | 5-$SCH_3$ | H | OH | n-$C_3H_7$ | nD 1.5506 (20.0°C) |

- Cont'd -

Table 1 (Cont'd)

| | | | | | | | |
|---|---|---|---|---|---|---|---|
| 69 | $CH_3$ | $CH_3$ | 5-$SCH_3$ | H | OH | i-$C_3H_7$ | nD 1.5471 (31.2°C) |
| 70 | $CH_3$ | $CH_3$ | 5-$SCH_3$ | H | OH | c-$C_3H_5$ | nD 1.5772 (26.2°C) |
| 71 | $CH_3$ | $CH_3$ | 5-$SCH_3$ | H | OH | i-$C_4H_9$ | nD 1.5588 (26.7°C) |
| 72 | $CH_3$ | $CH_3$ | 5-$SCH_3$ | H | OH | 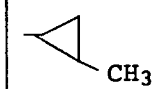 | nD 1.5633 (20.0°C) |
| 73 | $CH_3$ | $CH_3$ | 5-$SCH_3$ | H | OH | n-$C_5H_{11}$ | nD 1.5508 (28.2°C) |
| 74 | $CH_3$ | $CH_3$ | 5-$SCH_3$ | H | OH | c-$C_6H_{11}$ | mp. 89.5 - 91.0°C |
| 75 | $CH_3$ | $CH_3$ | 5-$SCH_3$ | H | OH | neo-$C_5H_{11}$ | nD 1.5472 (19.9°C) |

- Cont'd -

Table 1 (Cont'd)

| | | | | | | | |
|---|---|---|---|---|---|---|---|
| 76 | $CH_3$ | $CH_3$ | 5-$SCH_3$ | H | OH | $CH_3OCH_2$ | mp. 129 - 131.5°C |
| 77 | $CH_3$ | $CH_3$ | 5-$SCH_3$ | H | OH | 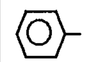 $OCH_2$ | nD 1.5845 (16.2°C) |
| 78 | $CH_3$ | $CH_3$ | 5-$SCH_3$ | H | OH | $CH_3S(CH_2)_2$ | nD 1.5617 (12.3°C) |
| 79 | $CH_3$ | $CH_3$ | 5-$SCH_3$ | H | Cl | $C_2H_5$ | mp. 141.2 - 141.3°C |
| 80 | $CH_3$ | $CH_3$ | 5-$SCH_3$ | H | $SCH_3$ | $C_2H_5$ | mp. 141.3 - 142.5°C |
| 81 | $CH_3$ | $CH_3$ | 5-$SCH_3$ | H | $SC_3H_7$-i | $C_2H_5$ | mp. 167.2 - 168.5°C |
| 82 | $CH_3$ | $CH_3$ | 5-$SCH_3$ | H | $SCH_2CH=CH_2$ | $C_2H_5$ | nD 1.5992 (11.7°C) |

- Cont'd -

Table 1 (Cont'd)

| | | | | | | | |
|---|---|---|---|---|---|---|---|
| 83 | $CH_3$ | $CH_3$ | 5-$SCH_3$ | H | $N(CH_3)_2$ | $C_2H_5$ | mp. 136.2 - 138.6°C |
| 84 | $CH_3$ | $CH_3$ | 5-$SCH_3$ | H | $N(C_2H_5)_2$ | $C_2H_5$ | nD 1.5729 (20.1°C) |
| 85 | $CH_3$ | $CH_3$ | 5-$SCH_3$ | H | $N(CH_2CH=CH_2)_2$ | $C_2H_5$ | mp. 87.0 - 89.2°C |
| 86 | $CH_3$ | $CH_3$ | 5-$SCH_3$ | H | O-CO-$C_3H_5$-c | $C_2H_5$ | nD 1.5546 (10.6°C) |
| 87 | $CH_3$ | $CH_3$ | 5-$SCH_3$ | H | O-CO-$C_3H_5$-c | c-$C_3H_5$ | nD 1.5446 (10.6°C) |
| 88 | $CH_3$ | $CH_3$ | 5-$SCH_3$ | $CH_3$ | OH | $C_2H_5$ | nD 1.5626 (23.2°C) |
| 89 | $CH_3$ | $CH_3$ | 5-$SOCH_3$ | $CH_3$ | OH | $C_2H_5$ | nD 1.5588 (23.6°C) |

- Cont'd -

Table 1 (Cont'd)

| | | | | | | | |
|---|---|---|---|---|---|---|---|
| 90 | $CH_3$ | $CH_3$ | 5-$SCH_3$ | $CH_3$ | OH | c-$C_3H_5$ | nD 1.5631 (23.8°C) |
| 91 | $CH_3$ | $CH_3$ | 5-$SOCH_3$ | $CH_3$ | OH | c-$C_3H_5$ | nD 1.5639 (22.9°C) |
| 92 | $CH_3$ | $CH_3$ | 5-$SCH_3$ | $CO_2C_2H_5$ | OH | $C_2H_5$ | nD 1.5529 (10.0°C) |
| 93 | $CH_3$ | $CH_3$ | 5-$SC_2H_5$ | H | OH | $CH_3$ | mp. 105.4 - 106.8°C |
| 94 | $CH_3$ | $CH_3$ | 5-$SC_2H_5$ | H | OH | $C_2H_5$ | mp. 60.7 - 60.9°C |
| 95 | $CH_3$ | $CH_3$ | 5-$SC_2H_5$ | H | OH | n-$C_3H_7$ | nD 1.5519 (21.1°C) |
| 96 | $CH_3$ | $CH_3$ | 5-$SC_2H_5$ | H | OH | c-$C_3H_5$ | nD 1.5654 (26.9°C) |

- Cont'd -

Table 1 (Cont'd)

| | | | | | | | |
|---|---|---|---|---|---|---|---|
| 97 | $CH_3$ | $CH_3$ | 5-$SC_3H_7$-i | H | OH | $CH_3$ | mp. 85.2°C |
| 98 | $CH_3$ | $CH_3$ | 5-$SC_3H_7$-i | H | OH | $C_2H_5$ | nD 1.5611 (22.4°C) |
| 99 | $CH_3$ | $CH_3$ | 5-$SC_3H_7$-i | H | OH | n-$C_3H_7$ | nD 1.5563 (22.4°C) |
| 100 | $CH_3$ | $CH_3$ | 5-$SCHF_2$ | H | OH | $C_2H_5$ | mp. 70.8 - 72.0°C |
| 101 | $CH_3$ | $CH_3$ | 5-$SOCH_3$ | H | OH | $CH_3$ | mp. 125.4 - 129.2°C |
| 102 | $CH_3$ | $CH_3$ | 5-$SOCH_3$ | H | OH | $C_2H_5$ | mp. 113 - 119.2°C |
| 103 | $CH_3$ | $CH_3$ | 5-$SOCH_3$ | H | OH | n-$C_3H_7$ | nD 1.5711 (17.8°C) |

- Cont'd -

Table 1 (Cont'd)

| | | | | | | | |
|---|---|---|---|---|---|---|---|
| 104 | $CH_3$ | $CH_3$ | 5-$SOCH_3$ | H | OH | c-$C_3H_5$ | mp. 186.3°C |
| 105 | $CH_3$ | $CH_3$ | 5-$SOCH_3$ | H | O-$COCH_3$ | c-$C_3H_5$ | Viscous product |
| 106 | $CH_3$ | $CH_3$ | 5-$SOCH_3$ | H | O-$COC_3H_7$-n | c-$C_3H_5$ | Viscous product |
| 107 | $CH_3$ | $CH_3$ | 5-$SOCH_3$ | H | O-$COC_3H_5$-c | c-$C_3H_5$ | nD 1.5403 (26.0°C) |
| 108 | $CH_3$ | $CH_3$ | 5-$SOCH_3$ | H | O-$COC_3H_7$-i | c-$C_3H_5$ | Viscous product |
| 109 | $CH_3$ | $CH_3$ | 5-$SOCH_3$ | H | O-$COC_7H_{15}$ | c-$C_3H_5$ | Viscous product |
| 110 | $CH_3$ | $CH_3$ | 5-$SOCH_3$ | H | O-CO 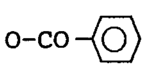 | c-$C_3H_5$ | Viscous product |

- Cont'd -

Table 1 (Cont'd)

| | | | | | | | |
|---|---|---|---|---|---|---|---|
| 111 | $CH_3$ | $CH_3$ | 5-$SOC_2H_5$ | H | OH | $CH_3$ | mp. 117.8 - 120.2°C |
| 112 | $CH_3$ | $CH_3$ | 5-$SOC_2H_5$ | H | OH | $C_2H_5$ | nD 1.5685 (16.2°C) |
| 113 | $CH_3$ | $CH_3$ | 5-$SOC_2H_5$ | H | OH | n-$C_3H_7$ | nD 1.5706 (16.9°C) |
| 114 | $CH_3$ | $CH_3$ | 5-$SOC_2H_5$ | H | OH | c-$C_3H_5$ | nD 1.5692 (27.1°C) |
| 115 | $CH_3$ | $CH_3$ | 5-$SOC_3H_7$-i | H | OH | $CH_3$ | mp. 108 - 110.1°C |
| 116 | $CH_3$ | $CH_3$ | 5-$SOC_3H_7$-i | H | OH | $C_2H_5$ | mp. 48.0 - 59.1°C |
| 117 | $CH_3$ | $CH_3$ | 5-$SOC_3H_7$-i | H | OH | n-$C_3H_7$ | nD 1.5542 (16.7°C) |

- Cont'd -

Table 1 (Cont'd)

| | | | | | | | |
|---|---|---|---|---|---|---|---|
| 118 | $CH_3$ | $CH_3$ | 5-$SO_2CH_3$ | H | OH | $C_2H_5$ | mp. 149.5 - 156.2°C |
| 119 | $CH_3$ | $CH_3$ | 5-$SO_2CH_3$ | H | OH | c-$C_3H_5$ | mp. 298°C |
| 120 | $CH_3$ | $CH_3$ | 5-S- 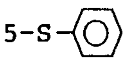 | H | OH | $CH_3$ | nD 1.5975 (19.8°C) |
| 121 | $CH_3$ | $CH_3$ | 5-S- 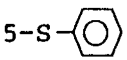 | H | OH | $C_2H_5$ | nD 1.5820 (19.8°C) |
| 122 | $CH_3$ | $CH_3$ | 5-S- 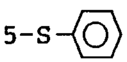 | H | OH | n-$C_3H_7$ | nD 1.5781 (20.0°C) |
| 123 | $CH_3$ | $CH_3$ | 5-$N(CH_3)_2$ | H | OH | $C_2H_5$ | nD 1.5437 (16.4°C) |
| 124 | $CH_3$ | $CH_3$ | 5-$N(CH_3)_2$ | H | OH | n-$C_3H_7$ | nD 1.5381 (16.5°C) |

- Cont'd -

Table 1 (Cont'd)

| | | | | | | | |
|---|---|---|---|---|---|---|---|
| 125 | $CH_3$ | $CH_3$ | 5-$N(CH_3)_2$ | H | OH | c-$C_3H_5$ | nD 1.5508 (25.8°C) |
| 126 | $CH_3$ | $C_2H_5$ | 5-Cl | H | OH | $CH_3$ | mp. 93.1°C |
| 127 | $CH_3$ | $C_2H_5$ | 5-Cl | H | OH | $C_2H_5$ | mp. 80.0°C |
| 128 | $CH_3$ | $C_2H_5$ | 5-Cl | H | OH | n-$C_3H_7$ | mp. 85.4°C |
| 129 | $CH_3$ | $C_2H_5$ | 5-Cl | H | OH | c-$C_3H_5$ | nD 1.5606 (26.5°C) |
| 130 | $CH_3$ | $C_2H_5$ | 5-$SCH_3$ | H | OH | $CH_3$ | mp. 101.8 - 103.1°C |
| 131 | $CH_3$ | $C_2H_5$ | 5-$SCH_3$ | H | OH | $C_2H_5$ | nD 1.5582 (28.0°C) |

- Cont'd -

Table 1 (Cont'd)

| | | | | | | | |
|---|---|---|---|---|---|---|---|
| 132 | $CH_3$ | $C_2H_5$ | 5-$SCH_3$ | H | OH | n-$C_3H_7$ | nD 1.5538 (27.8°C) |
| 133 | $CH_3$ | $C_2H_5$ | 5-$SCH_3$ | H | OH | c-$C_3H_5$ | nD 1.5732 (21.2°C) |
| 134 | $CH_3$ | $C_2H_5$ | 5-$SOCH_3$ | H | OH | $C_2H_5$ | nD 1.4619 (23.6°C) |
| 135 | $CH_3$ | $C_2H_5$ | 5-$SOCH_3$ | H | OH | c-$C_3H_5$ | mp. 148.1 - 149.5°C |
| 136 | $CH_3$ | i-$C_3H_7$ | 5-Cl | H | OH | $C_2H_5$ | Viscous product |
| 137 | $CH_3$ | i-$C_3H_7$ | 5-$SCH_3$ | H | OH | $CH_3$ | mp. 134.0 - 135.5°C |
| 138 | $CH_3$ | i-$C_3H_7$ | 5-$SCH_3$ | H | OH | $C_2H_5$ | mp. 67 - 68°C |

- Cont'd -

Table 1 (Cont'd)

| | | | | | | | |
|---|---|---|---|---|---|---|---|
| 139 | $CH_3$ | $i-C_3H_7$ | $5-SCH_3$ | H | OH | $n-C_3H_7$ | mp. 93 - 94°C |
| 140 | $CH_3$ | $t-C_4H_9$ | 5-Cl | H | OH | $C_2H_5$ | Viscous product |
| 141 | $CH_3$ | $t-C_4H_9$ | $5-SCH_3$ | H | OH | $C_2H_5$ | nD 1.5478 (25.5°C) |
| 142 | $CH_3$ | $CF_3$ | 5-Cl | H | OH | $C_2H_5$ | mp. 121.7°C |
| 143 | $CH_3$ | 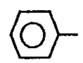 | 5-Cl | H | OH | $C_2H_5$ | mp. 82.4°C |
| 144 | $CH_3$ | 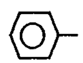 | 5-Cl | H | OH | $c-C_3H_5$ | Viscous product |
| 145 | $CH_3$ | 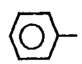 | $5-OCH_3$ | H | OH | $C_2H_5$ | mp. 141.5°C |

- Cont'd -

Table 1 (Cont'd)

| | | | | | | | |
|---|---|---|---|---|---|---|---|
| 146 | $C_2H_5$ | H | 5-Cl | H | OH | $C_2H_5$ | nD 1.5473 (24.0°C) |
| 147 | $C_2H_5$ | H | 5-Cl | H | OH | $C_2H_5$ | nD 1.5606 (22.5°C) |
| 148 | $C_2H_5$ | H | 5-$SCH_3$ | H | OH | c-$C_3H_5$ | nD 1.5640 (21.8°C) |
| 149 | $C_2H_5$ | H | 5-$SCH_3$ | H | OH | $C_2H_5$ | nD 1.5712 (21.8°C) |
| 150 | $C_2H_5$ | H | 5-$SOCH_3$ | H | OH | c-$C_3H_5$ | mp. 95.1 - 98.2°C |
| 151 | $C_2H_5$ | H | 5-$SOCH_3$ | H | OH | $C_2H_5$ | nD 1.5733 (22.0°C) |
| 152 | $C_2H_5$ | Cl | 5-$SOCH_3$ | H | OH | c-$C_3H_5$ | mp. 140 - 141°C |

- Cont'd -

Table 1 (Cont'd)

| | | | | | | | |
|---|---|---|---|---|---|---|---|
| 153 | $C_2H_5$ | $CH_3$ | 5-Cl | H | OH | $C_2H_5$ | nD 1.5456 (26.5°C) |
| 154 | $C_2H_5$ | $CH_3$ | 5-Cl | H | OH | $n-C_3H_7$ | nD 1.5388 (26.3°C) |
| 155 | $C_2H_5$ | $CH_3$ | 5-Cl | H | OH | $c-C_3H_5$ | nD 1.5168 (24.2°C) |
| 156 | $C_2H_5$ | $CH_3$ | $5-CH_3$ | H | OH | $C_2H_5$ | nD 1.5415 (15.0°C) |
| 157 | $C_2H_5$ | $CH_3$ | $5-CH_3$ | H | OH | $c-C_3H_5$ | nD 1.5490 (15.0°C) |
| 158 | $C_2H_5$ | $CH_3$ | $5-CH_3$ | H | O-CO 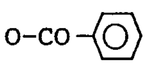 | $c-C_3H_5$ | nD 1.5508 (22.0°C) |
| 159 | $C_2H_5$ | $CH_3$ | $5-OCH_3$ | H | OH | $C_2H_5$ | mp. 65.0 - 67.4°C |

- Cont'd -

Table 1 (Cont'd)

| | | | | | | | |
|---|---|---|---|---|---|---|---|
| 160 | $C_2H_5$ | $CH_3$ | 5-$OCH_3$ | H | OH | c-$C_3H_5$ | mp. 86.9 - 89.1°C |
| 161 | $C_2H_5$ | $CH_3$ | 5-$OCH_3$ | H | OH | $C_2H_5$ | nD 1.5618 (20.9°C) |
| 162 | $C_2H_5$ | $CH_3$ | 5-$OCH_3$ | H | OH | $CH_3$ | nD 1.5718 (27.5°C) |
| 163 | $C_2H_5$ | $CH_3$ | 5-$OCH_3$ | H | OH | n-$C_3H_7$ | nD 1.5513 (21.2°C) |
| 164 | $C_2H_5$ | $CH_3$ | 5-$OCH_3$ | H | OH | i-$C_3H_7$ | nD 1.5467 (18.1°C) |
| 165 | $C_2H_5$ | $CH_3$ | 5-$OCH_3$ | H | OH | c-$C_3H_5$ | mp. 93.1 - 93.8°C |
| 166 | $C_2H_5$ | $CH_3$ | 5-$OCH_3$ | H | OH | 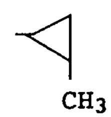 | nD 1.5599 (27.2°C) |

- Cont'd -

Table 1 (Cont'd)

| | | | | | | | |
|---|---|---|---|---|---|---|---|
| 167 | $C_2H_5$ | $CH_3$ | 5-$SCH_3$ | $CH_3$ | OH | c-$C_3H_5$ | nD 1.5702 (14.3°C) |
| 168 | $C_2H_5$ | $CH_3$ | 5-$SCH_3$ | $CO_2CH_3$ | OH | c-$C_3H_5$ | nD 1.5544 (25.4°C) |
| 169 | $C_2H_5$ | $CH_3$ | 5-$SCH_3$ | $CO_2C_2H_5$ | OH | $C_2H_5$ | nD 1.5328 (22.2°C) |
| 170 | $C_2H_5$ | $CH_3$ | 5-$SCH_3$ | $CO_2C_2H_5$ | OH | c-$C_3H_5$ | Viscous product |
| 171 | $C_2H_5$ | $CH_3$ | 5-$SC_2H_5$ | H | OH | c-$C_3H_5$ | nD 1.5728 (17.2°C) |
| 712 | $C_2H_5$ | $CH_3$ | 5-$SOCH_3$ | H | OH | $CH_3$ | mp. 125.1 - 125.5°C |
| 173 | $C_2H_5$ | $CH_3$ | 5-$SOCH_3$ | H | OH | $C_2H_5$ | mp. 85.2 - 87.2°C |

- Cont'd -

Table 1 (Cont'd)

| | | | | | | | |
|---|---|---|---|---|---|---|---|
| 174 | $C_2H_5$ | $CH_3$ | 5-$SOCH_3$ | H | OH | n-$C_3H_7$ | mp. 136.1 - 136.5°C |
| 175 | $C_2H_5$ | $CH_3$ | 5-$SOCH_3$ | H | OH | i-$C_3H_7$ | nD 1.5506 (18.7°C) |
| 176 | $C_2H_5$ | $CH_3$ | 5-$SOCH_3$ | H | OH | c-$C_3H_5$ | mp. 130.1 - 131.1°C |
| 177 | $C_2H_5$ | $CH_3$ | 5-$SOCH_3$ | H | OH | c-$C_3H_5$ | Glassy product (Na salt) |
| 178 | $C_2H_5$ | $CH_3$ | 5-$SOCH_3$ | H | OH | c-$C_3H_5$ | Viscous product (DBU salt) |
| 179 | $C_2H_5$ | $CH_3$ | 5-$SOCH_3$ | H | OH | c-$C_3H_5$ | mp. 122 - 123°C (Fe salt) |
| 180 | $C_2H_5$ | $CH_3$ | 5-$SOCH_3$ | H | OH | c-$C_3H_5$ | mp. 159 - 184°C (Cu salt) |

- Cont'd -

Table 1 (Cont'd)

| | | | | | | | |
|---|---|---|---|---|---|---|---|
| 181 | $C_2H_5$ | $CH_3$ | 5-$SOCH_3$ | H | OH | c-$C_3H_5$ | mp. 270 - 280°C (Cu salt) |
| 182 | $C_2H_5$ | $CH_3$ | 5-$SOCH_3$ | H | OH | c-$C_3H_5$ | mp. 189 - 193°C (K salt) |
| 183 | $C_2H_5$ | $CH_3$ | 5-$SOCH_3$ | H | OH | 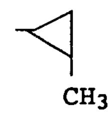 | nD 1.5554 (27.2°C) |
| 184 | $C_2H_5$ | $CH_3$ | 5-$SOCH_3$ | H | O-CO- 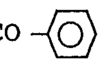 | c-$C_3H_5$ | nD 1.5608 (23.5°C) |
| 185 | $C_2H_5$ | $CH_3$ | 5-$SOCH_3$ | H | O-CO- 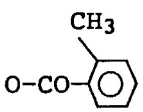 | c-$C_3H_5$ | nD 1.5719 (27.5°C) |
| 186 | $C_2H_5$ | $CH_3$ | 5-$SOCH_3$ | H | O-CO- 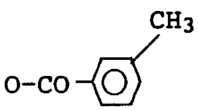 | c-$C_3H_5$ | nD 1.5732 (27.3°C) |
| 187 | $C_2H_5$ | $CH_3$ | 5-$SOCH_3$ | H | O-CO- 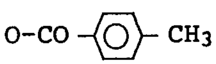 | c-$C_3H_5$ | mp. 86.3 - 88.6°C |

- Cont'd -

Table 1 (Cont'd)

| | | | | | | | |
|---|---|---|---|---|---|---|---|
| 188 | $C_2H_5$ | $CH_3$ | $5\text{-}SOCH_3$ | H | 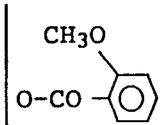 | $c\text{-}C_3H_5$ | nD 1.5730 (27.8°C) |
| 189 | $C_2H_5$ | $CH_3$ | $5\text{-}SOCH_3$ | H | 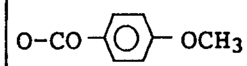 | $c\text{-}C_3H_5$ | mp. 85.0 - 88.5°C |
| 190 | $C_2H_5$ | $CH_3$ | $5\text{-}SOCH_3$ | H | 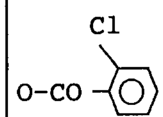 | $c\text{-}C_3H_5$ | nD 1.5819 (28.2°C) |
| 191 | $C_2H_5$ | $CH_3$ | $5\text{-}SOCH_3$ | H | 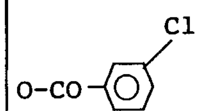 | $c\text{-}C_3H_5$ | nD 1.5664 (28.5°C) |
| 912 | $C_2H_5$ | $CH_3$ | $5\text{-}SOCH_3$ | H | 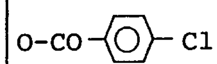 | $c\text{-}C_3H_5$ | mp. 82.0 - 85.0°C |
| 193 | $C_2H_5$ | $CH_3$ | $5\text{-}SOCH_3$ | H | 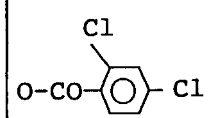 | $c\text{-}C_3H_5$ | nD 1.5761 (27.4°C) |

- Cont'd -

Table 1 (Cont'd)

| | | | | | | | |
|---|---|---|---|---|---|---|---|
| 194 | $C_2H_5$ | $CH_3$ | 5-$SOCH_3$ | $CH_3$ | OH | c-$C_3H_5$ | nD 1.5723 (15.4°C) |
| 195 | $C_2H_5$ | $CH_3$ | 5-$SOCH_3$ | $CO_2CH_3$ | OH | c-$C_3H_5$ | Viscous product |
| 196 | $C_2H_5$ | $CH_3$ | 5-$SOCH_3$ | $CO_2C_2H_5$ | OH | c-$C_3H_5$ | Viscous product |
| 197 | $C_2H_5$ | $CH_3$ | 5-$SO_2CH_3$ | H | OH | c-$C_3H_5$ | mp. 194.1 - 195.5°C |
| 198 | $C_2H_5$ | $CH_3$ | 5-$SOC_2H_5$ | H | OH | c-$C_3H_5$ | mp. 113.4 - 115.7°C |
| 199 | $C_2H_5$ | $CH_3$ | 5-$SO_2C_2H_5$ | H | OH | c-$C_3H_5$ | mp. 138.2 - 139.5°C |
| 200 | $C_2H_5$ | $CH_3$ | 5-$N(CH_3)_2$ | H | OH | $C_2H_5$ | mp. 69.2 - 69.5°C |

- Cont'd -

Table 1 (Cont'd)

| | | | | | | | |
|---|---|---|---|---|---|---|---|
| 201 | $C_2H_5$ | $CH_3$ | 5-$N(CH_3)_2$ | H | OH | c-$C_3H_5$ | nD 1.5480 (20.8°C) |
| 202 | $C_2H_5$ | Cl | 5-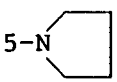 | H | OH | c-$C_3H_5$ | mp. 65.0 - 67.0°C |
| 203 | n-$C_3H_7$ | Cl | 5-$CH_3$ | H | OH | $C_2H_5$ | nD 1.5441 (24.9°C) |
| 204 | n-$C_3H_7$ | $CH_3$ | 5-$CH_3$ | H | OH | c-$C_3H_5$ | nD 1.5238 (24.8°C) |
| 205 | n-$C_3H_7$ | $CH_3$ | 5-Cl | H | OH | c-$C_3H_5$ | nD 1.5598 (20.7°C) |
| 206 | n-$C_3H_7$ | $CH_3$ | 5-$CH_3$ | H | OH | $C_2H_5$ | nD 1.5305 (16.0°C) |
| 207 | n-$C_3H_7$ | $CH_3$ | 5-$CH_3$ | H | OH | c-$C_3H_5$ | nD 1.5432 (15.0°C) |

- Cont'd -

Table 1 (Cont'd)

| | | | | | | | |
|---|---|---|---|---|---|---|---|
| 208 | n-$C_3H_7$ | $CH_3$ | 5-$CH_3$ | H | OH | c-$C_3H_5$ | Glassy product (Na salt) |
| 209 | n-$C_3H_7$ | $CH_3$ | 5-$CH_3$ | H | OH | c-$C_3H_5$ | mp. 214.1 - 215.4°C (K salt) |
| 210 | n-$C_3H_7$ | $CH_3$ | 5-$CH_3$ | H | OH | c-$C_3H_5$ | mp. 165 - 170°C (Zn salt) |
| 211 | n-$C_3H_7$ | $CH_3$ | 5-$CH_3$ | H | OH | c-$C_3H_5$ | mp. 260 - 270°C (Ca salt) |
| 212 | n-$C_3H_7$ | $CH_3$ | 5-$CH_3$ | H | OH | c-$C_3H_5$ | mp. 245 - 260°C Decomposition (Ba salt) |
| 213 | n-$C_3H_7$ | $CH_3$ | 5-$CH_3$ | H | OH | c-$C_3H_5$ | mp>250°C (Cu salt) |
| 214 | n-$C_3H_7$ | $CH_3$ | 5-$CH_3$ | H | OH | c-$C_3H_5$ | Glassy product (Fe salt) |

- Cont'd -

Table 1 (Cont'd)

| | | | | | | | |
|---|---|---|---|---|---|---|---|
| 215 | n-$C_3H_7$ | $CH_3$ | 5-$CH_3$ | H | OH | c-$C_3H_5$ | mp>250°C (Mg salt) |
| 216 | n-$C_3H_7$ | $CH_3$ | 5-$CH_3$ | H | OH | c-$C_3H_5$ | Viscous product $(CH_3)_2N$ 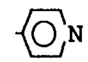 salt |
| 217 | n-$C_3H_7$ | $CH_3$ | 5-$CH_3$ | H | OH | c-$C_3H_5$ | Viscous product (DBU salt) |
| 218 | n-$C_3H_7$ | $CH_3$ | 5-$CH_3$ | H | OH | c-$C_3H_5$ | mp. 120 - 121°C $(C_2H_5)_2NH$ salt |
| 219 | n-$C_3H_7$ | $CH_3$ | 5-$CH_3$ | H | OH | c-$C_3H_5$ | nD 1.5350 (12.0°C) $(C_2H_4OH)_3N$ salt |
| 220 | n-$C_3H_7$ | $CH_3$ | 5-$CH_3$ | H | OH | c-$C_3H_5$ | 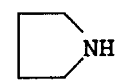 salt |
| 221 | n-$C_3H_7$ | $CH_3$ | 5-$CH_3$ | H | OH | c-$C_3H_5$ | nD 1.5452 (12.0°C) 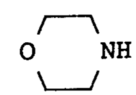 salt |

- Cont'd -

Table 1 (Cont'd)

| | | | | | | | |
|---|---|---|---|---|---|---|---|
| 222 | n-$C_3H_7$ | $CH_3$ | 5-$OCH_3$ | H | O-CO 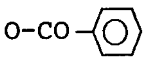 | c-$C_3H_5$ | mp. 73.0°C |
| 223 | n-$C_3H_7$ | $CH_3$ | 5-$OCH_3$ | H | OH | c-$C_3H_5$ | nD 1.5511 (20.8°C) |
| 224 | n-$C_3H_7$ | $CH_3$ | 5-$SCH_3$ | H | OH | $C_2H_5$ | nD 1.5516 (24.3°C) |
| 225 | n-$C_3H_7$ | $CH_3$ | 5-$SCH_3$ | H | OH | c-$C_3H_5$ | nD 1.5671 (24.5°C) |
| 226 | n-$C_3H_7$ | $CH_3$ | 5-$SOCH_3$ | H | OH | $C_2H_5$ | nD 1.5558 (24.3°C) |
| 227 | n-$C_3H_7$ | $CH_3$ | 5-$SOCH_3$ | H | OH | c-$C_3H_5$ | nD 1.5579 (24.9°C) |
| 228 | n-$C_3H_7$ | $CH_3$ | 5-$SO_2CH_3$ | H | OH | c-$C_3H_5$ | mp. 176 - 177.2°C |

- Cont'd -

Table 1 (Cont'd)

| | | | | | | | |
|---|---|---|---|---|---|---|---|
| 229 | n-$C_3H_7$ | $CH_3$ | 5-$N(CH_3)_2$ | H | OH | c-$C_3H_5$ | nD 1.5479 (21.2°C) |
| 230 | i-$C_3H_7$ | $CH_3$ | 5-Cl | H | OH | $C_2H_5$ | mp. 74.2°C |
| 231 | i-$C_3H_7$ | $CH_3$ | 5-Cl | H | OH | n-$C_3H_7$ | mp. 96.6°C |
| 232 | i-$C_3H_7$ | $CH_3$ | 5-$CH_3$ | H | OH | c-$C_3H_5$ | mp. 97.2 - 99.6°C |
| 233 | i-$C_3H_7$ | $CH_3$ | 5-$SCH_3$ | H | OH | $C_2H_5$ | Glassy product |
| 234 | i-$C_3H_7$ | $CH_3$ | 5-$SCH_3$ | H | OH | n-$C_3H_7$ | Glassy product |
| 235 | i-$C_3H_7$ | $CH_3$ | 5-$SCH_3$ | H | OH | c-$C_3H_5$ | Glassy product |

- Cont'd -

Table 1 (Cont'd)

| | | | | | | | |
|---|---|---|---|---|---|---|---|
| 236 | $i-C_3H_7$ | $CH_3$ | $5-SOCH_3$ | H | OH | $C_2H_5$ | mp. 120.1 - 122.5°C |
| 237 | $i-C_3H_7$ | $CH_3$ | $5-SOCH_3$ | H | OH | $c-C_3H_5$ | Glassy product |
| 238 | $n-C_4H_9$ | Cl | $5-CH_3$ | H | OH | $C_2H_5$ | nD 1.5329 (19.5°C) |
| 239 | $n-C_4H_9$ | Cl | $5-CH_3$ | H | OH | $c-C_3H_5$ | nD 1.5503 (17.8°C) |
| 240 | $n-C_4H_9$ | $CH_3$ | $5-CH_3$ | H | OH | $C_2H_5$ | nD 1.5342 (10.0°C) |
| 241 | $n-C_4H_9$ | $CH_3$ | $5-CH_3$ | H | OH | $c-C_3H_5$ | nD 1.5420 (9.0°C) |
| 242 | $n-C_4H_9$ | $CH_3$ | $5-CH_3$ | H | O-CO 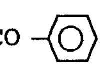 | $c-C_3H_5$ | mp. 120.8 - 122.5°C |

- Cont'd -

Table 1 (Cont'd)

| | | | | | | | |
|---|---|---|---|---|---|---|---|
| 243 | n-$C_4H_9$ | $CH_3$ | 5-$SCH_3$ | H | OH | c-$C_3H_5$ | nD 1.5481 (20.1°C) |
| 244 | n-$C_4H_9$ | $CH_3$ | 5-$SOCH_3$ | H | OH | $C_2H_5$ | nD 1.5526 (18.3°C) |
| 245 | n-$C_4H_9$ | $CH_3$ | 5-$SOCH_3$ | H | OH | c-$C_3H_5$ | nD 1.5363 (18.9°C) |
| 246 | s-$C_4H_9$ | $CH_3$ | 5-$CH_3$ | H | OH | c-$C_3H_5$ | mp. 68.0°C |
| 247 | i-$C_4H_9$ | Cl | 5-$CH_3$ | H | OH | c-$C_3H_5$ | mp.123.1 - 124.5°C |
| 248 | i-$C_4H_9$ | $CH_3$ | 5-$CH_3$ | H | OH | c-$C_3H_5$ | nD 1.5485 (27.0°C) |
| 249 | i-$C_4H_9$ | $CH_3$ | 5-$SCH_3$ | H | OH | c-$C_3H_5$ | nD 1.5622 (17.6°C) |

- Cont'd -

Table 1 (Cont'd)

| | | | | | | | |
|---|---|---|---|---|---|---|---|
| 250 | i-$C_4H_9$ | $CH_3$ | 5-$SOCH_3$ | H | OH | c-$C_3H_5$ | nD 1.5669 (18.3°C) |
| 251 | t-$C_4H_9$ | $CH_3$ | 5-Cl | H | OH | $CH_3$ | Viscous product |
| 252 | n-$C_5H_{11}$ | $CH_3$ | 5-$CH_3$ | H | OH | $C_2H_5$ | nD 1.5330 (17.0°C) |
| 253 | n-$C_5H_{11}$ | $CH_3$ | 5-$CH_3$ | H | OH | c-$C_3H_5$ | nD 1.5470 (20.0°C) |
| 254 | n-$C_5H_{11}$ | $CH_3$ | 5-$CH_3$ | H | O-CO 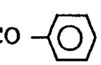 | c-$C_3H_5$ | mp. 46.0°C |
| 255 | n-$C_8H_{17}$ | $CH_3$ | 5-Cl | H | OH | $C_2H_5$ | nD 1.5252 (15.3°C) |
| 256 | n-$C_8H_{17}$ | $CH_3$ | 5-Cl | H | OH | c-$C_3H_5$ | nD 1.5181 (18.3°C) |

- Cont'd -

Table 1 (Cont'd)

| | | | | | | | |
|---|---|---|---|---|---|---|---|
| 257 | n-$C_8H_{17}$ | $CH_3$ | 5-$SCH_3$ | H | OH | $C_2H_5$ | nD 1.5341 (19.2°C) |
| 258 | n-$C_8H_{17}$ | $CH_3$ | 5-$SCH_3$ | H | OH | c-$C_3H_5$ | nD 1.5388 (19.8°C) |
| 259 | n-$C_8H_{17}$ | $CH_3$ | 5-$SOCH_3$ | H | OH | $C_2H_5$ | nD 1.5336 (20.6°C) |
| 260 | n-$C_8H_{17}$ | $CH_3$ | 5-$SOCH_3$ | H | OH | c-$C_3H_5$ | nD 1.5435 (21.1°C) |
| 261 | n-$C_{16}H_{33}$ | $CH_3$ | 5-$CH_3$ | H | OH | $C_2H_5$ | mp. 48°C |
| 262 | n-$C_{16}H_{33}$ | $CH_3$ | 5-$CH_3$ | H | OH | c-$C_3H_5$ | nD 1.5220 (16.0°C) |
| 263 | n-$C_{16}H_{33}$ | $CH_3$ | 5-$CH_3$ | H | O-CO 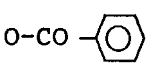 | c-$C_3H_5$ | nD 1.5250 (16.0°C) |

- Cont'd -

Table 1 (Cont'd)

| | | | | | | | |
|---|---|---|---|---|---|---|---|
| 264 | $CHF_2$ | $CH_3$ | 5-$CH_3$ | H | OH | c-$C_3H_5$ | mp. 155 - 156°C |
| 265 | $ClCH_2CH_2$ | $CH_3$ | 5-$CH_3$ | H | OH | $C_2H5$ | mp. 87.0°C |
| 266 | $ClCH_2CH_2$ | $CH_3$ | 5-$CH_3$ | H | OH | c-$C_3H_5$ | mp. 116 - 117°C |
| 267 | $CH_2$=CH | $CH_3$ | 5-$CH_3$ | H | OH | $C_2H5$ | mp. 101.0°C |
| 268 | $CH_2$=CH | $CH_3$ | 5-$CH_3$ | H | OH | c-$C_3H_5$ | nD 1.5760 (10.0°C) |
| 269 | $CH_2$=CH | $CH_3$ | 5-$SCH_3$ | H | OH | c-$C_3H_5$ | mp. 105.5 - 106.9°C |
| 270 | $CH_2$=$CHCH_2$ | Cl | 5-$CH_3$ | H | OH | $C_2H5$ | nD 1.5633 (11.8°C) |

- Cont'd -

Table 1 (Cont'd)

| | | | | | | | |
|---|---|---|---|---|---|---|---|
| 271 | $CH_2=CHCH_2$ | Cl | 5-$CH_3$ | H | OH | c-$C_3H_5$ | nD 1.5693 (15.2°C) |
| 272 | $CH_2=CHCH_2$ | $CH_3$ | 5-$CH_3$ | H | OH | c-$C_3H_5$ | nD 1.5630 (20.0°C) |
| 273 | $CH_2=CHCH_2$ | $CH_3$ | 5-$SCH_3$ | H | OH | $C_2H_5$ | nD 1.5673 (12.5°C) |
| 274 | $CH_2=CHCH_2$ | $CH_3$ | 5-$SCH_3$ | H | OH | c-$C_3H_5$ | nD 1.5509 (25.1°C) |
| 275 | $CH_2=CHCH_2$ | $CH_3$ | 5-$SOCH_3$ | H | OH | $C_2H_5$ | nD 1.5421 (13.6°C) |
| 276 | $CH_2=CHCH_2$ | $CH_3$ | 5-$SOCH_3$ | H | OH | c-$C_3H_5$ | nD 1.5692 (25.2°C) |
| 277 | $CH=C=CH_2$ | $CH_3$ | 5-$CH_3$ | H | OH | c-$C_3H_5$ | mp. 91.0°C |

- Cont'd -

Table 1 (Cont'd)

| | | | | | | | |
|---|---|---|---|---|---|---|---|
| 278 | $CH_3OCH_2$ | $CH_3$ | 5-$CH_3$ | H | OH | c-$C_3H_5$ | mp. 88.0°C |
| 279 | $CH_3OCH_2$ | $CH_3$ | 5-$SCH_3$ | H | OH | c-$C_3H_5$ | Viscous product |
| 280 | $CH_3OCH_2$ | $CH_3$ | 5-$SOCH_3$ | H | OH | c-$C_3H_5$ | Viscous product |
| 281 | 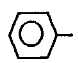 | $CH_3$ | 5-H | H | OH | $C_2H_5$ | Glassy product |
| 282 | 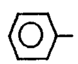 | $CH_3$ | 5-H | H | OH | n-$C_3H_7$ | nD 1.5864 (28.6°C) |
| 283 | 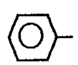 | $CH_3$ | 5-Cl | H | OH | $C_2H_5$ | nD 1.5880 (17.7°C) |
| 284 | 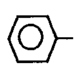 | $CH_3$ | 5-Cl | H | OH | n-$C_3H_7$ | mp. 88.9°C |

- Cont'd -

Table 1 (Cont'd)

| | | | | | | | |
|---|---|---|---|---|---|---|---|
| 285 | 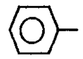 | $CH_3$ | 5-$OCH_3$ | H | OH | $C_2H_5$ | nD 1.5719 (21.7°C) |
| 286 | 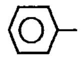 | $CH_3$ | 5-$OCH_3$ | H | OH | n-$C_3H_7$ | mp. 91.2°C |
| 287 | 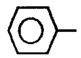 | $CH_3$ | 5-$OC_2H_5$ | H | OH | $C_2H_5$ | mp. 70.2 - 71.3°C |
| 288 | 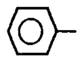 | $CH_3$ | 5-$SCH_3$ | H | OH | $C_2H_5$ | mp. 113.7°C |
| 289 | 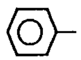 | $CH_3$ | 5-$SCH_3$ | H | OH | n-$C_3H_7$ | mp. 88.8 - 94.7°C |
| 290 | 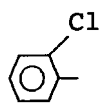 | $CH_3$ | 5-Cl | H | OH | $C_2H_5$ | mp. 98.1°C |
| 291 | 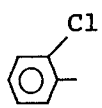 | $CH_3$ | 5-$OCH_3$ | H | OH | $C_2H_5$ | mp. 93.7°C |

- Cont'd -

Table 1 (Cont'd)

| | | | | | | | |
|---|---|---|---|---|---|---|---|
| 292 | 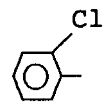 | $CH_3$ | 5-$OCH_3$ | H | OH | n-$C_3H_7$ | nD 1.5653 (21.0°C) |
| 293 | 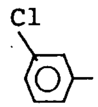 | $CH_3$ | 5-$OCH_3$ | H | OH | $C_2H_5$ | nD 1.5847 (19.0°C) |
| 294 | 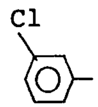 | $CH_3$ | 5-$OCH_3$ | H | OH | n-$C_3H_7$ | mp. 150.6°C |
| 295 | 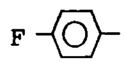 | $CH_3$ | 5-Cl | H | OH | $C_2H_5$ | nD 1.5754 (30.0°C) |
| 296 | 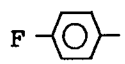 | $CH_3$ | 5-Cl | H | OH | c-$C_3H_5$ | Glassy product |
| 297 | 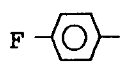 | $CH_3$ | 5-$OCH_3$ | H | OH | $C_2H_5$ | nD 1.5560 (30.2°C) |
| 298 | 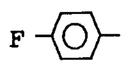 | $CH_3$ | 5-$SCH_3$ | H | OH | $C_2H_5$ | mp. 132.7°C |

- Cont'd -

Table 1 (Cont'd)

| | | | | | | | |
|---|---|---|---|---|---|---|---|
| 299 | 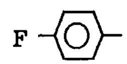 | $CH_3$ | 5-$SCH_3$ | H | OH | c-$C_3H_5$ | Glassy product |
| 300 | 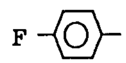 | $CH_3$ | 5-$SOCH_3$ | H | OH | $C_2H_5$ | Glassy product |
| 301 | 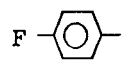 | $CH_3$ | 5-$SOCH_3$ | H | OH | c-$C_3H_5$ | Glassy product |
| 302 | 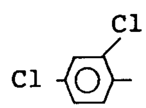 | $CH_3$ | 5-Cl | H | OH | $C_2H_5$ | mp. 162.1°C |
| 303 | 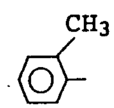 | $CH_3$ | 5-$OCH_3$ | H | OH | $C_2H_5$ | mp. 110.1°C |
| 304 | 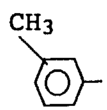 | $CH_3$ | 5-Cl | H | OH | $C_2H_5$ | nD 1.5980 (20.0°C) |

- Cont'd -

Table 1 (Cont'd)

| | | | | | | | |
|---|---|---|---|---|---|---|---|
| 305 | 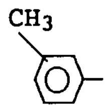 | $CH_3$ | 5-Cl | H | OH | n-$C_3H_7$ | nD 1.5843 (20.0°C) |
| 306 | 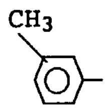 | $CH_3$ | 5-O-$COC_3H_7$-n | H | OH | n-$C_3H_7$ | mp. 127.7°C |
| 307 | 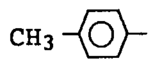 | $CH_3$ | 5-Cl | H | OH | $C_2H_5$ | mp. 150.6°C |
| 308 | 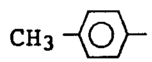 | $CH_3$ | 5-Cl | H | OH | n-$C_3H_7$ | mp. 95.3°C |
| 309 | 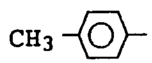 | $CH_3$ | 5-$OCH_3$ | H | OH | $C_2H_5$ | nD 1.5760 (19.0°C) |
| 310 | 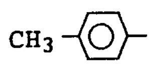 | $CH_3$ | 5-$OCH_3$ | H | OH | n-$C_3H_7$ | nD 1.5668 (27.0°C) |
| 311 | 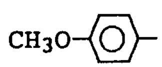 | $CH_3$ | 5-Cl | H | OH | $C_2H_5$ | mp. 139.9°C |

- Cont'd -

Table 1 (Cont'd)

| | | | | | | | |
|---|---|---|---|---|---|---|---|
| 312 | 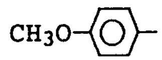 | $CH_3$ | 5-Cl | H | OH | n-$C_3H_7$ | nD 1.5857 (24.0°C) |
| 313 | 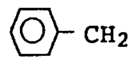 | $CH_3$ | 5-$SCH_3$ | H | OH | $C_2H_5$ | nD 1.5930 (12.3°C) |
| 314 | 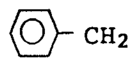 | $CH_3$ | 5-$SCH_3$ | H | OH | c-$C_3H_5$ | nD 1.5642 (14.4°C) |
| 315 | 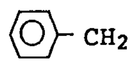 | $CH_3$ | 5-$SOCH_3$ | H | OH | $C_2H_5$ | mp. 163.1 - 164.5°C |
| 316 | 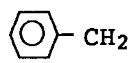 | $CH_3$ | 5-$SOCH_3$ | H | OH | c-$C_3H_5$ | mp. 170.2 - 171.2°C |
| 317 | 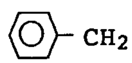 | Cl | 5-$CH_3$ | H | OH | $C_2H_5$ | mp. 63.2 - 64.8°C |
| 318 | 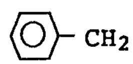 | Cl | 5-$CH_3$ | H | OH | c-$C_3H_5$ | mp. 136.1 - 137.5°C |

- Cont'd -

Table 1 (Cont'd)

| | | | | | | | |
|---|---|---|---|---|---|---|---|
| 319 | $CH_3$ | H | 4-H | H | OH | $C_2H_5$ | nD 1.5387 (19.3°C) |
| 320 | $CH_3$ | $CH_3$ | 4-H | H | OH | $C_2H_5$ | nD 1.5331 (12.1°C) |
| 321 | $CH_3$ | $CH_3$ | 4-H | H | OH | n-$C_3H_7$ | nD 1.5545 (16.8°C) |
| 322 | $CH_3$ | $CH_3$ | 4-H | H | OH | c-$C_3H_5$ | mp. 94.4 - 97.1°C |
| 323 | $CH_3$ | $CH_3$ | 4-Cl | H | OH | $C_2H_5$ | mp. 77.0 - 79.5°C |
| 324 | $CH_3$ | $CH_3$ | 4-Br | H | OH | $C_2H_5$ | mp. 103.0 - 105.6°C |
| 325 | $CH_3$ | $CH_3$ | 4-Br | H | OH | c-$C_3H_5$ | mp. 146.8 - 148.5°C |

- Cont'd -

Table 1 (Cont'd)

| | | | | | | | |
|---|---|---|---|---|---|---|---|
| 326 | $CH_3$ | i-$C_3H_7$ | 4-H | H | OH | $C_2H_5$ | nD 1.5387 (19.3°C) |
| 327 | $CH_3$ | i-$C_3H_7$ | 4-H | H | OH | n-$C_3H_7$ | mp. 62.2 - 69.2°C |
| 328 | $CH_3$ | i-$C_3H_7$ | 4-H | H | OH | c-$C_3H_5$ | mp. 94.4 - 98.5°C |
| 329 | $CH_3$ | i-$C_3H_7$ | 4-Cl | H | OH | $C_2H_5$ | mp. 119.0 - 120.2°C |
| 330 | $CH_3$ | i-$C_3H_7$ | 4-Cl | H | OH | c-$C_3H_5$ | mp. 136.6 - 137.3°C |
| 331 | $CH_3$ | t-$C_4H_9$ | 4-H | H | OH | $CH_3$ | mp. 78.3°C |
| 332 | $CH_3$ | t-$C_4H_9$ | 4-H | H | OH | $C_2H_5$ | mp. 73.4 - 80.4°C |

- Cont'd -

Table 1 (Cont'd)

| | | | | | | | |
|---|---|---|---|---|---|---|---|
| 333 | $CH_3$ | t-C4H9 | 4-H | H | OH | c-$C_3H_5$ | mp. 94.4 - 98.5°C |
| 334 | $CH_3$ | 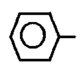 | 4-H | H | OH | n-$C_3H_7$ | mp. 112.8°C |
| 335 | i-$C_3H_7$ | $CH_3$ | 4-H | H | OH | $C_2H_5$ | mp. 92.5 - 97.5°C |
| 336 | i-$C_3H_7$ | $CH_3$ | 4-H | H | OH | c-$C_3H_5$ | mp. 136.5 - 136.7°C |

Note: A symbol, c-, in Table 1 means an alicyclic compound.

Among the compounds in Table 1, the NMR data of those of which the physical property is "Viscous product" or "Glassy product" are shown in Table 3.

Table 2

| Compound No. | NMR value ($CDCl_3$/TMS, δvalue, ppm) |
|---|---|
| 16 | 1.12-1.20 (m, 2H), 1.30-1.38 (m, 2H), 2.27 (s, 3H), 2.54-2.67 (m, 2H), 3.12-3.22 (m, 1H), 3.32-3.43 (m, 1H), 3.48-3.66 (m, 2H), 3.93 (s, 3H). |
| 17 | 1.12-1.20 (m, 2H), 1.30-1.38 (m, 2H), 2.50-2.76 (m, 2H), 2.97+2.98 (s, 3H), 3.04-3.35 (m, 2H), 3.54-3.77 (m, 2H), 4.06+4.08 (s, 3H). (mixture of diastereomers) |
| 31 | 1.17 (t, 3H), 2.25 (s, 3H), 2.32-4.10 (m, 6H), 3.63 (s, 3H), 3.76 (s, 3H). |
| 32 | 1.05 (t, 3H), 1.70 (m, 2H), 2.27 (s, 3H), 2.42-4.20 (m, 6H), 3.67 (s, 3H), 3.74 (s, 3H). |
| 54 | 1.07 (t, 3H), 2.23 (s, 3H), 3.47 (s, 3H), 1.19-4.03 (m, 7H), 6.39-7.53 (m, 3H). |
| 105 | 0.77-1.49 (m, 4H), 2.16 (s, 3H), 2.30 (s, 3H), 2.97 (s, 3H), 4.03 (s, 3H), 2.00-3.95 (m, 6H). |
| 106 | 0.83-1.27 (m, 7H), 2.30 (s, 3H), 2.97 (s, 3H), 4.06 (s, 3H), 1.30-4.05 (m, 10H). |
| 108 | 0.79-1.47 (m, 10H), 2.23 (s, 3H), 3.90 (s, 3H), 4.00 (s, 3H), 2.05-4.05 (m, 7H). |

— Cont'd —

Table 2 (Cont'd)

| | |
|---|---|
| 109 | 0.67-1.90 (m, 19H), 2.23 (s, 3H), 2.90 (s, 3H), 4.00 (s, 3H), 2.00-3.95 (m, 6H). |
| 110 | 0.77-1.36 (m, 4H), 2.30 (s, 3H), 2.96 (s, 3H), 4.03 (s, 3H), 2.00-3.95 (m, 6H), 7.40-7.70 (m, 3H), 7.87-8.17 (m, 2H). |
| 136 | 1.16 (t, 3H), 1.23 (d, 6H), 2.30-3.35 (m, 8H), 3.70 (s, 3H). |
| 140 | 1.18 (t, 3H), 1.34 (s, 9H), 2.32-3.68 (m, 5H), 3.32 (q, 2H), 3.74 (s, 3H). |
| 144 | 0.92-1.42 (m, 4H), 2.41-3.78 (m, 6H), 3.83 (s, 3H), 7.32 (s, 5H). |
| 170 | 1.12-1.18 (m, 2H), 1.15 (t, 3H), 1.31-1.38 (m, 2H), 1.43 (t, 3H), 2.25 (s, 3H), 2.30 (s, 3H), 2.62-3.40 (m, 3H), 3.48-3.54 (m, 1H), 3.70-4.08 (m, 1H), 4.15 (q, 2H), 4.28 (q, 2H). |
| 177 ($D_2O$/ DSS) | 0.90-1.00 (m, 4H), 1.35 (t, 3H), 2.25-2.49 (m, 3H), 2.65-2.85 (m, 2H), 3.11 (s, 2H), 3.53-3.65 (m, 1H), 3.53-3.65 (m, 1H), 4.32 (q, 2H). |
| 178 | 0.68-0.78 (m, 2H), 0.88-0.95 (m, 2H), 1.46 (t, 3H), 1.60-1.80 (m, 8H), 1.92-2.07 (m, 2H), 2.24 (s, 3H), 2.28-3.04 (m, 8H), 2.89 (s, 3H), 3.35-3.50 (m, 4H), 4.36-4.48 (m, 2H). |
| 195 | 1.08-1.14 (m, 2H), 1.30-1.35 (m, 2H), 1.47 (t, 3H), 2.29+2.31 (s, 3H), 3.02+3.06 (s, 3H), 2.72-3.32 (m, 3H), 3.48-3.54 (m, 1H), 3.65+3.66 (s, 3H), 3.10-4.08 (m, 1H), 4.35 (q, 2H). |

— Cont'd —

Table 2 (Cont'd)

| | |
|---|---|
| 196 | 1.08-1.14 (m, 2H), 1.15 (t, 3H), 1.30-1.35 (m, 2H), 1.48 (t, 3H), 2.30+2.33 (s, 3H), 3.03+3.07 (s, 3H), 2.72-3.32 (m, 3H), 3.48-3.54 (m, 1H), 3.70-4.08 (m, 1H), 4.11 (q, 2H), 4.35 (q, 2H). |
| 208 ($D_2O$/ DSS) | 0.76 (t, 3H), 0.87-0.93 (m, 4H), 1.65 (m, 2H), 2.14 (s, 3H), 2.17 (s, 3H), 2.24-2.31 (m, 2H), 2.64-2.74 (m, 2H), 3.21 (m, 1H), 3.86 (t, 2H). |
| 216 | 0.88 (t, 3H), 1.05-1.15 (m, 2H), 1.77 (q, 2H), 2.17 (s, 3H), 2.22 (s, 3H), 2.55-2.68 (m, 2H), 2.79-2.98 (m, 2H), 2.97 (s, 3H), 3.05-3.32 (m, 1H), 3.50-3.64 (m, 1H), 3.88 (t, 2H). 6.47 (dd, 2H), 8.20 (dd, 2H). |
| 217 | 0.75-0.83 (m, 2H), 0.87 (t, 3H), 0.90-1.01 (m, 2H), 1.60-1.82 (m, 8H), 1.92-2.07 (m, 2H), 2.15 (s, 3H), 2.22 (s, 3H), 2.35-2.50 (m, 2H), 2.65-2.85 (m, 4H), 3.00-3.28 (m, 2H), 3.83-3.50 (m, 4H), 3.87 (t, 2H). |
| 233 | 1.07 (t, 3H), 1.43 (d, 6H), 2.23 (s, 3H), 2.27 (s, 3H), 2.05-3.83 (m, 7H), 4.70-5.28 (m, 1H). |
| 234 | 1.03 (t, 3H), 1.32-2.10 (m, 8H), 2.19 (s, 3H), 2.31 (s, 3H), 2.10-4.00 (m, 7H), 4.67-5.26 (m, 1H). |
| 235 | 0.73-1.27 (m, 4H), 1.47 (d, 6H), 2.19 (s, 3H), 2.31 (s, 3H), 2.01-4.00 (m, 1H), 4.70-5.24 (m, 1H). |

- Cont'd -

Table 2 (Cont'd)

| | |
|---|---|
| 237 | 1.06-1.70 (m, 10H), 2.30 (s, 3H), 2.90 (s, 3H), 2.09-3.95 (m, 6H), 4.69-5.25 (m, 1H). |
| 251 | 1.13 (t, 3H), 1.66 (s, 9H), 1.22 (s, 3H), 2.62 (s, 3H), 2.68-3.30 (m, 4H), 4.06 (q, 2H). |
| 279 | 0.85-1.44 (m, 4H), 2.25 (s, 6H), 3.38 (s, 3H), 2.13-3.84 (m, 6H), 5.42 (s, 2H). |
| 280 | 1.02-1.46 (m, 4H), 2.32 (s, 3H), 3.03 (s, 3H), 3.32 (s, 3H), 2.34-3.83 (m, 6H), 5.33 (d, 1H). |
| 281 | 1.13 (t, 3H), 2.30 (s, 3H), 2.30-3.57 (m, 7H), 7.00-7.78 (m, 6H). |
| 296 | 0.73-1.50 (m, 4H), 2.03 (s, 3H), 2.43-2.83 (m, 6H), 6.93-7.70 (m, 4H). |
| 299 | 0.80-1.53 (m, 4H), 2.03 (s, 3H), 2.36 (s, 3H), 2.27-3.83 (m, 6H), 6.93-7.77 (m, 4H). |
| 300 | 1.17 (t, 3H), 2.40 (s, 3H), 2.67-4.17 (m, 10H), 6.97-7.73 (m, 4H). |
| 301 | 0.18-1.53 (m, 4H), 2.37 (s, 3H), 2.23-4.13 (m, 9H), 7.00-7.70 (m, 4H). |

The compounds represented by the formulae (II) and (III), which are a material for producing the cyclohexane derivatives of the present invention represented by the formulae (IA) and (IB) and their salts, can be produced, for example, by the following method :

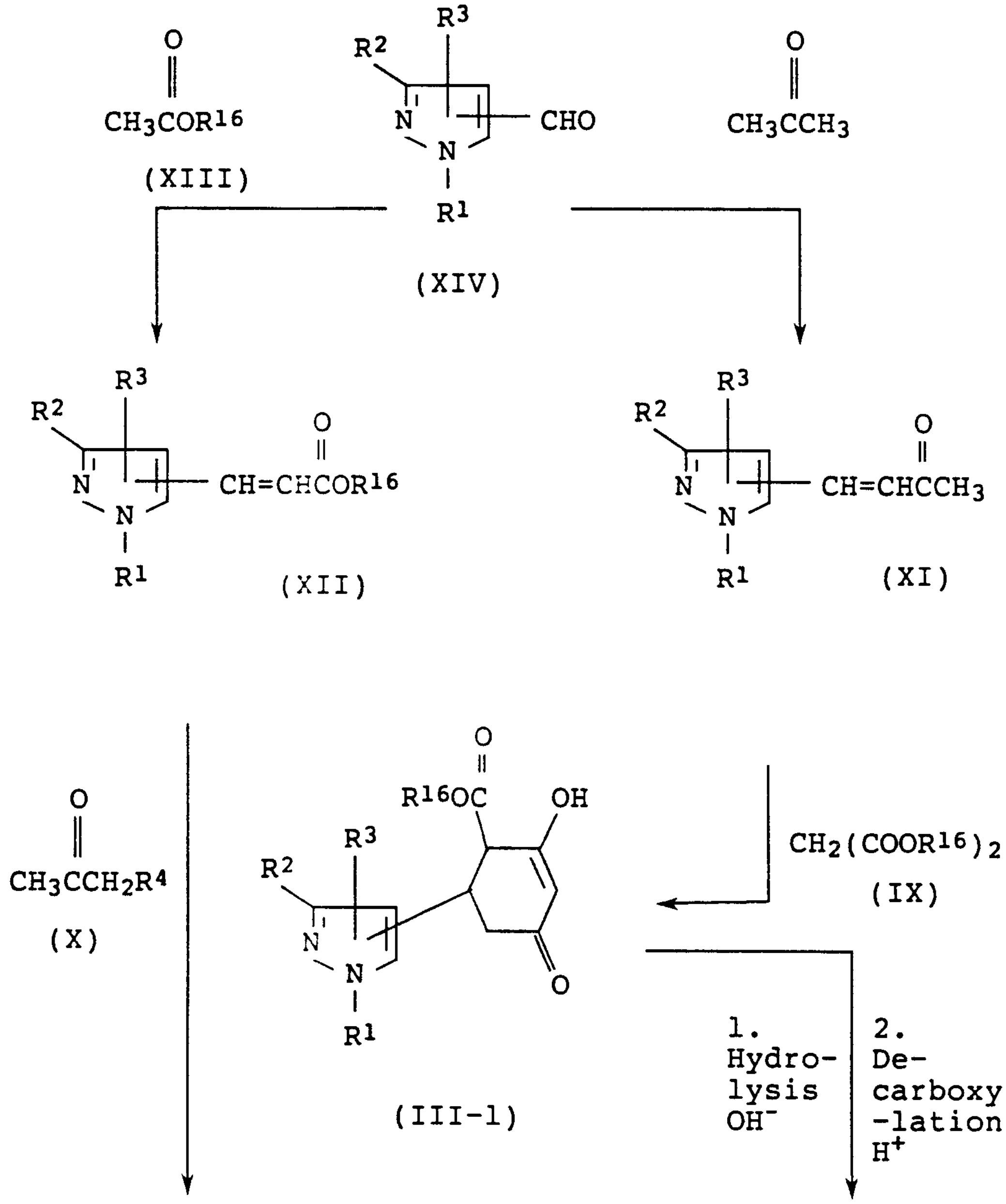


(XIII) (XIV) (XII) (XI) (X) (IX) (III-1)

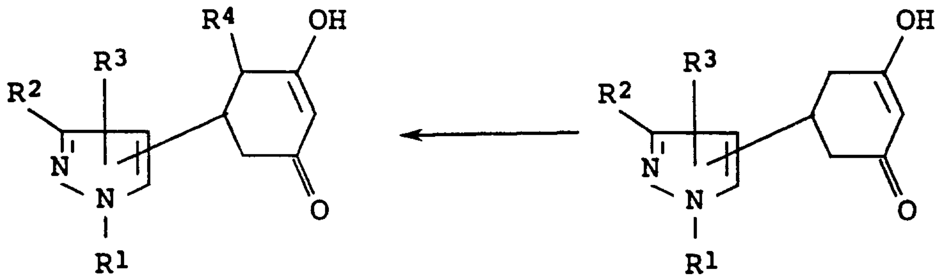

(III) (III-2)

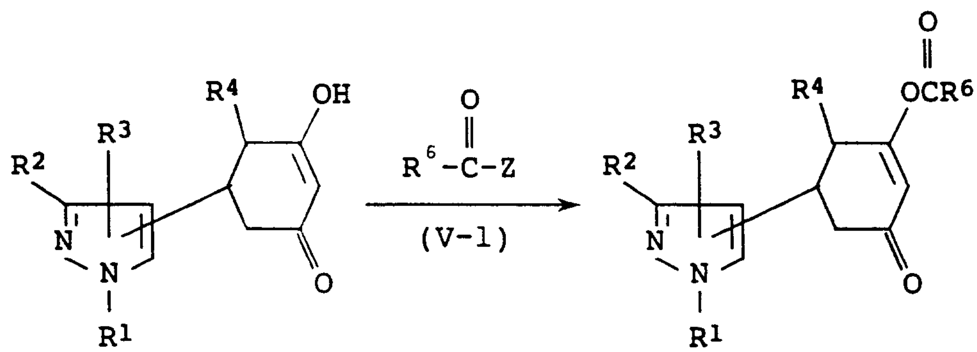


(III) (II)

wherein $R^1$, $R^2$, $R^3$, $R^4$, $R^6$ and Z are as defined above, and $R^{16}$ represents a lower alkyl group.

When $R^4$ is an alkyl group, the compound represented by the formula (III) can be produced by reacting a pyrazole represented by the formula (XIV) with an acetate represented by the formula (XIII), reacting the resulting pyrazole represented by the formula (XII) with a ketone represented by the formula (X) and then subjecting the resulting compound to ring-closure reaction.

When $R^4$ is a hydrogen atom or an alkoxy-carbonyl group, the compound represented by the formula (III-1) can be produced by reacting a pyrazole represented by the formula (XIV) with acetone, reacting the resulting pyrazole represented by the formula (XI) with a malonate represented by the formula (IX), and then subjecting the resulting compound to ring-closure reaction.

Subsequently, the compound represented by the formula (III-2) can be produced by subjecting the compound represented by the formula (III-1) to hydrolysis and decarboxylation.

The compound represented by the formula (III) can be produced by the method described above.

The compound represented by the formula (II) can be produced by reacting the compound represented by the formula (III) with an acyl halide represented by the formula (V-1).

Production examples for typical compounds of the cyclohexane derivatives of the present invention represented by the formulae (IA) and (IB) and their salts will be shown below.

Production example 1

Production of 5-(1,3-dimethyl-1H-pyrazol-4-yl)-3-hydroxy-2-propionyl-2-cyclohexen-1-one (Compound No. 19)

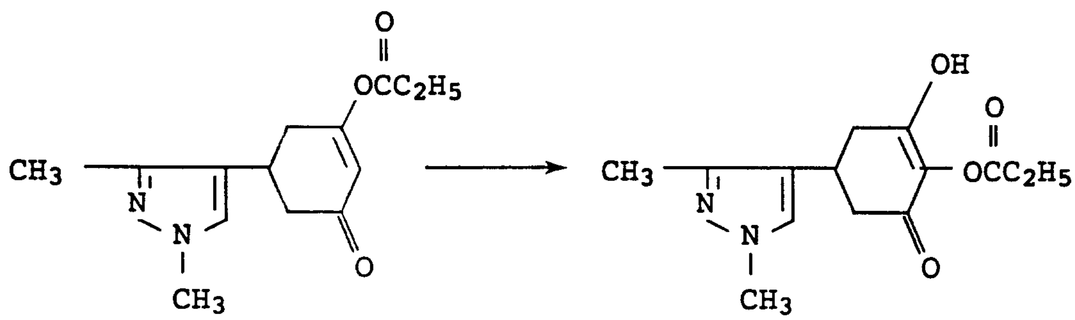

1.31 Grams (5 mmoles) of 5-(1,3-dimethyl-1H-pyrazol-4-yl)- 3-oxo-1-cyclohexenyl propionate and 0.122 g (1 mmole) of 4-N,N-dimethylaminopyridine were dissolved in 50 ml of dry tetrahydrofuran. The mixture was heated under reflux for 8 hours in a nitrogen atmosphere. After completion of the reaction, the reaction solution was allowed to cool to room temperature, and the solvent was removed under reduced pressure. After adding 50 ml of water to the residue, the pH was adjusted to 4 with a dilute hydrochloric acid, and the product was extracted with three 40-ml portions of ethyl acetate. After drying the extract, the solvent was removed and the residue was purified by column chromatography on silica gel (ethyl acetate : n-hexane) to obtain 1.19 g of the desired product.

m.p. 90.0 °C

Yield 91%

Production example 2

Production of 5-(5-chloro-1,3-dimethyl-1H-pyrazol-4-yl)-3-hydroxy-2-propionyl-2-cyclohexen-1-one (Compound No. 22)

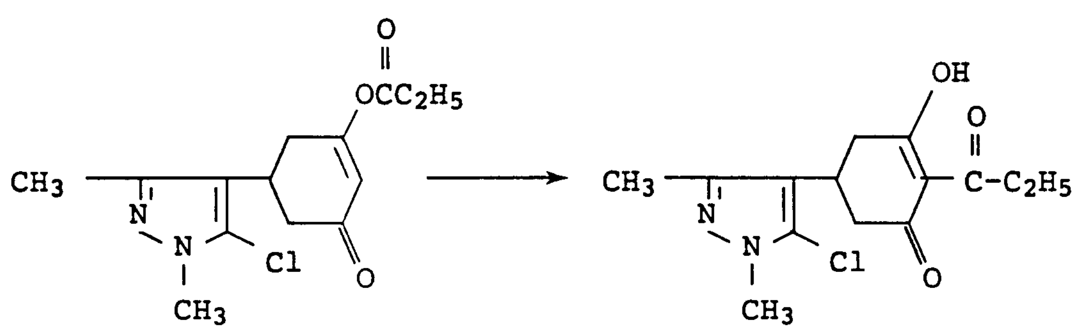

1.49 Grams (5 mmoles) of 5-(5-chloro-1,3-dimethyl-1H-pyrazol-4-yl)-3-oxo-1-cyclohexenyl propionate and 0.122 g (1 mmole) of 4-N,N-dimethylaminopyridine were dissolved in 80 ml of dry tetrahydrofuran. The mixture was heated under reflux for 8 hours in a nitrogen atmosphere. After completion of the reaction, the reaction solution was allowed to cool to room tempeature, and the solvent was removed under reduced pressure. After adding 50 ml of water to the residue, the pH was adjusted to 4 with a dilute hydrochloric acid, and the product was extracted with three 40-ml portions of ethyl acetate. After drying the extract, the solvent was removed and the residue was purified by column chromatography on silica gel (ethyl acetate : n-hexane) to obtain 1.30 g of the desired product.

nD 1.5462 (25.1 °C)

Yield 87%

Production example 3

Production of 2-isobutyryl-3-hydroxy-5-( 5-chloro-1,3-dimethyl-1H-pyrazol-4-yl)-2-cyclohexen-1-one (Compound No. 25)

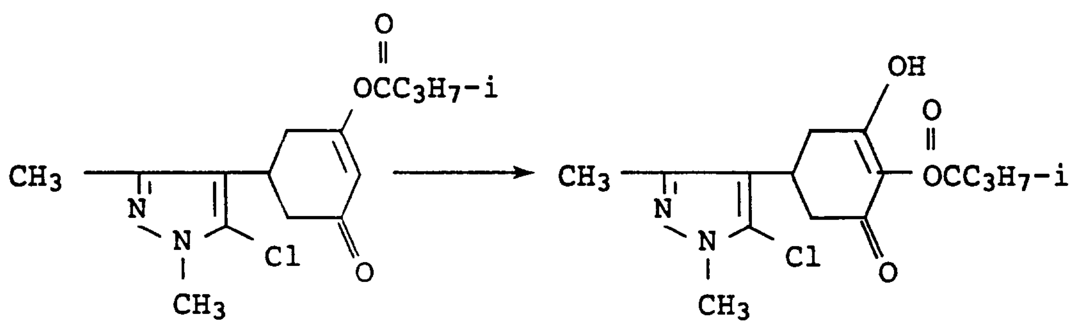

1.56 Grams (5 mmoles) of 5-(5-chloro-1,3-dimethyl-1H-pyrazol-4-yl)-3-oxo-1-cyclohexenyl isobutyrate was dissolved in 15 ml of dry acetonitrile, and 0.085 g (1 mmole) of acetone cyanohydrin and 1.01 g (10 mmoles) of triethylamine were added. The resulting mixture was heated under reflux for 1 hour. After completion of the reaction, the reaction solution was allowed to cool to room temperature, and the solvent was removed under reduced pressure. After adding 20 ml of water to the residue, the pH was adjusted to 4 with a dilute hydrochloric acid, and the desired product was extracted with three 50-ml portions of diethyl ether. After drying the extract, the solvent was removed and the residue was purified by column chromatography on silica gel (ethyl acetate : n-hexane) to obtain 1.13 g of the desired product.

m.p. 86.5 °C
Yield 72%

Production example 4

Production of 2-butyryl-3-hydroxy-5-(5-methoxy-1,3-dimethyl-1H-pyrazol-4-yl)-2-cyclohexen-1-one (Compound No. 44)

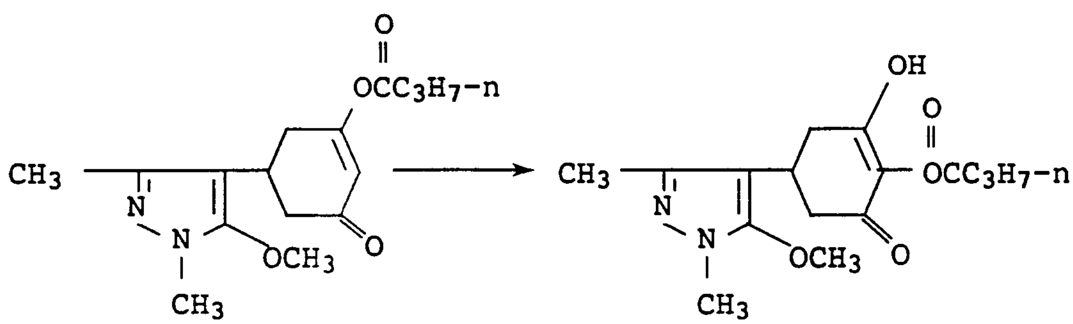

1.84 Grams (5 mmoles) of 5-(5-methoxy-1,3-dimethyl-1H-pyrazol-4-yl)-3-oxo-1-cyclohexenyl butyrate and 0.122 g (1 mmole) of 4-N,N-dimethylaminopyridine were dissolved in 80 ml of dry tetrahydrofuran. The mixture was heated under reflux for 8 hours in a nitrogen atmosphere. After completion of the reaction, the reaction solution was allowed to cool to room temperature, and the solvent was removed under reduced pressure. After adding 50 ml of water to the residue, the pH was adjusted to 4 with a dilute hydrochloric acid, and the product was extracted with three 40-ml portions of ethyl acetate. After drying the extract, the solvent was removed and the residue was purified by column chromatography on silica gel (ethyl acetate : n-hexane) to obtain 1.64 g of the desired product.

m.p. 73.2 °C
Yield 89%

Production example 5

Production of 5-(1-ethyl-3-methyl-5-methylthio-1H-pyrazol-4-yl)-3-hydroxy-2-cyclopropylcarbonyl-2-cyclohexen-1-one (Compound No. 165)

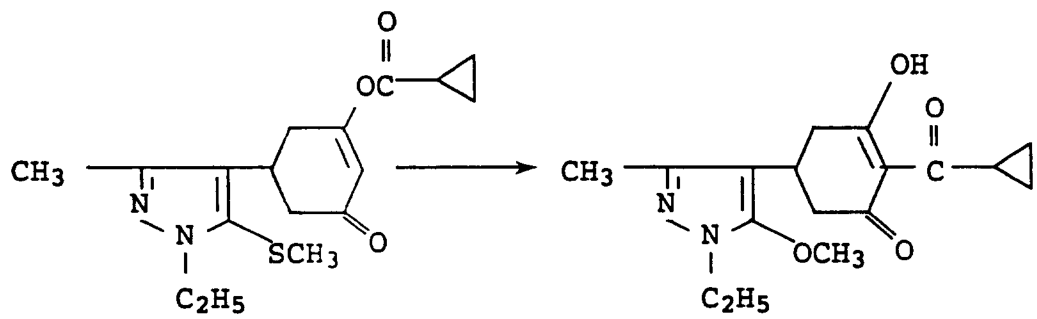

12.3 Grams (36.8 mmoles) of 5-(1-ethyl-3-methyl-5-methylthio-1H-pyrazol-4-yl)-3-oxo-1-cyclohexenyl cyclopropanecarboxylate was dissolved in 100 ml of dry acetonitrile, and 0.3 g (3.7 mmoles) of acetone cyanohydrin and 8.2 g (81.2 mmoles) of triethylamine were added. The mixture was stirred at room temperature for 8 hours in a nitrogen atmosphere.

After completion of the reaction, the solvent was removed under reduced pressure. After adding 100 ml of water to the residue obtained, the pH was adjusted to 4 with a dilute hydrochloric acid, and the desired product was extracted with three 100-ml portions of ethyl acetate. After drying the extract, the solvent was removed and the residue was purified by column chromatography on silica gel (ethyl acetate : n-hexane) to obtain 11.2 g of the desired product.

m.p. 93.1 - 93.8 °C

Yield 91.1%

Production example 6

Production of 5-(1-ethyl-3-methyl-5-pyrrolidino-1H-pyrazol-4-yl)-3-hydroxy-2-cyclopropylcarbonyl-2-cyclohexen-1-one (Compound No. 202)

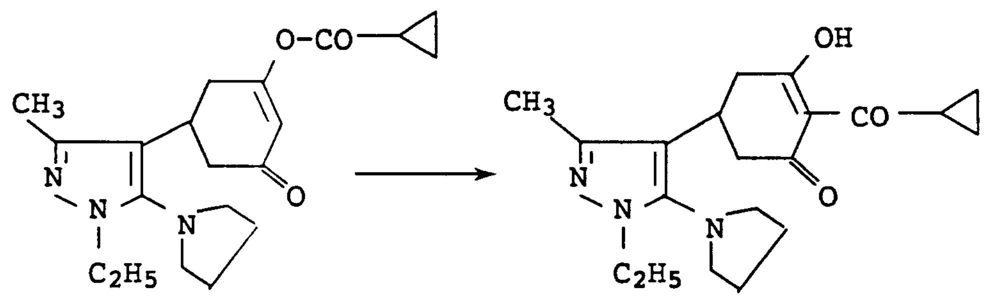

2.2 Grams (6.2 mmoles) of 5-(1-ethyl-3-methyl-5-pyrrolidino-1H-pyrazol-4-yl)-3-oxo-1-cyclohexenyl cyclopropanecarboxylate was dissolved in 50 ml of dry acetonitrile, and 0.1 g (1.2 mmoles) of acetone cyanohydrin and 1.4 g (13.6 mmoles) of triethylamine were added. The mixture was stirred at room temperature for 8 hours in a nitrogen atmosphere.

After completion of the reaction, the solvent was removed under reduced pressure. After adding 50 ml of water to the residue obtained, the pH was adjusted to 4 with a dilute hydrochloric acid, and the desired product was extracted with three 40-ml portions of ethyl acetate. After drying the extract, the solvent was removed and the residue was purified by column chromatography on silica gel (ethyl acetate : n-hexane) to obtain 1.4 g of the desired product.

m.p. 65.0 - 67.0 °C

Yield 63.6%

Production example 7

Production of 5-(3-methyl-5-methylsulfinyl-1-octyl-1H-pyrazol-4-yl)-2-cyclopropyl-carbonyl-3-hydroxy-2-cyclohexen-1-one (Compound No. 260)

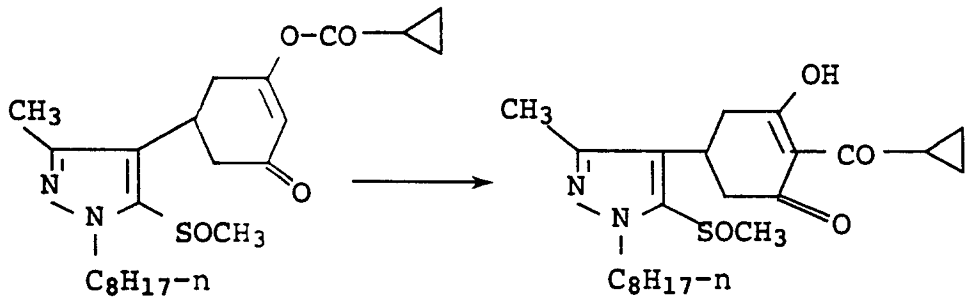

1.7 Grams (3.9 mmoles) of 5-(3-methyl-5-methylsulfinyl-1-octyl-1H-pyrazol-4-yl)-3-oxo-1-cyclohexenyl cyclopropanecarboxylate was dissolved in 20 ml of dry acetonitrile, and 0.1 g (1.2 mmoles) of acetone cyanohydrin and 0.9 g (8.9 mmoles) of triethylamine were added. The mixture was stirred at room temperature for 8 hours in a nitrogen atmosphere.

After completion of the reaction, the solvent was removed under reduced pressure. After adding 150 ml of water to the residue obtained, the pH was adjusted to 4 with a dilute hydrochloric acid, and the desired product was extracted with three 40-ml portions of ethyl acetate. After drying the extract, the solvent was removed and the residue was purified by column chromatography on silica gel (ethyl acetate : n-hexane) to obtain 1.6 g of the desired product.

nD 1.5435 (21.1 ° C)

Yield 94.1%

Production example 8

Production of 5-(3,5-dimethyl-1-n-propyl-1H-pyrazol-4-yl)-2-cyclopropylcarbonyl-3-hydroxy-2-cyclohexen-1-one (Compound No. 207)

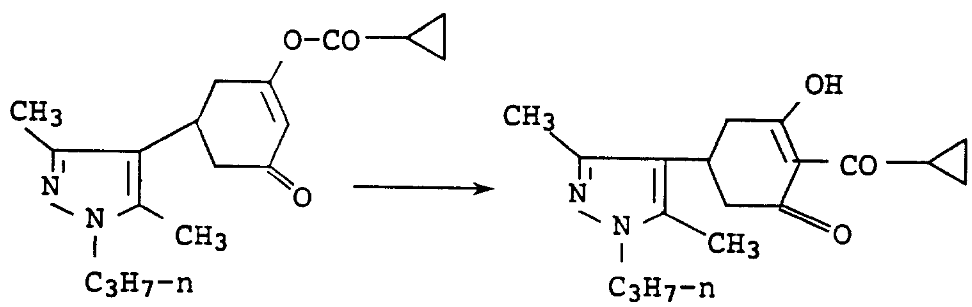

1.77 Grams (5.6 mmoles) of 5-(3,5-dimethyl-1-n-propyl-1H-pyrazol-4-yl)-3-oxo-1-cyclohexenyl cyclopropanecarboxylate, 0.1 g (1.1 mmoles) of acetone cyanohydrin and 1.24 g (12.3 mmoles) of triethylamine were dissolved in 20 ml of dry acetonitrile. The mixture was stirred at room temperature for 8 hours in a nitrogen atmosphere.

After completion of the reaction, the solvent was removed under reduced pressure. After adding 50 ml of water to the residue obtained, the pH was adjusted to 4 with a dilute hydrochloric acid, and the desired product was extracted with three 40-ml portions of ethyl acetate. After drying the extract, the solvent was removed and the residue was purified by column chromatography on silica gel (ethyl acetate : n-hexane) to obtain 1.51 g of the desired product.

nD 1.5432 (15 ° C)

Yield 85.3%

The product obtained was further purified to obtain 1.48 g of crystals.
m.p. 79.7-79.9 °C
Yield 83.6%

Production example 9

Production of 5-(1,3-dimethyl-1H-pyrazol-5-yl)-2-propionyl-2-cyclohexen-1-one (Compound No. 320)

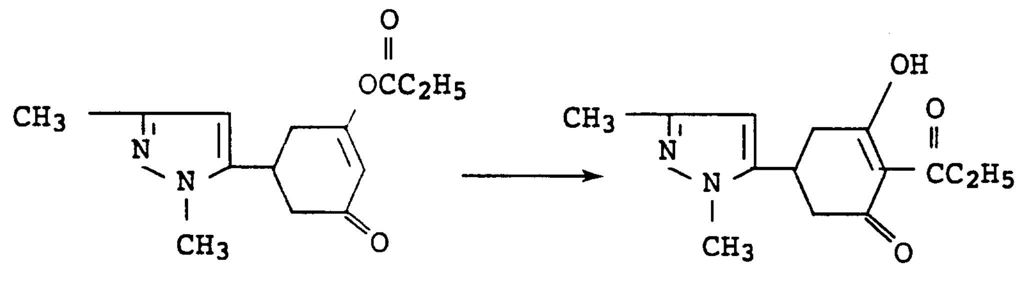

A mixture of 17.5 g (66.7 mmoles) of 5-(1,3-dimethyl-1H-pyrazol-5-yl)-3-oxo-1-cyclohexenyl propionate and 0.85 g (7.0 mmoles) of 4-N,N-dimethylaminopyridine was dissolved in 200 ml of tetrahydrofuran. The mixture was heated under reflux for 2 hours.

After completion of the reaction, the reaction solution was cooled and the solvent was removed under reduced pressure. The residue obtained was purified by column chromatography on silica gel (ethyl acetate : n-hexane) to obtain 16.40 g of the desired product.
nD 1.5331 (12.1 °C)
Yield 93.7%

Production example 10

Production of 5-(1,3-dimethyl-5-methylsulfinyl-1H-pyrazol-4-yl)-3-oxo-2-cyclopropylcarbonyl-1-cyclohexenyl benzoate (Compound No. 110)

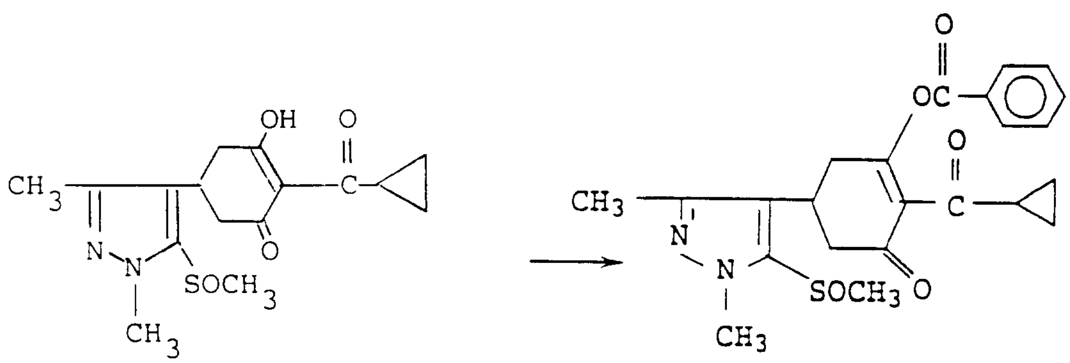

0.6 Gram (1.8 mmoles) of 5-(1,3-dimethyl-5-methylsulfinyl-1H-pyrazol-4-yl)-3-hydroxy-2-cyclopropylcarbonyl-2-cyclohexen-1-one and 0.082 g (2.1 mmoles) of 63% sodium hydride were added to 30 ml of dry tetrahydrofuran, and the resulting mixture was stirred for 30 minutes. Thereafter, to the reaction solution was added 0.30 g (2.1 mmoles) of benzoyl chloride, and stirring was continued overnight at room temperature. After completion of the reaction, water was added to the reaction solution, and the product was extracted with ether. The extract was washed with an aqueous potassium carbonate solution and then with water, dried and concentrated. The residue obtained was purified by column chromatography on silica gel (ethyl acetate) to obtain 0.20 g of the desired product.
Physical property : viscous
Yield 25%
NMR ($CDCl_3$/TMS, $\delta$ value, ppm)

0.77-1.36 (m, 4H), 2.30 (s, 3H), 2.96 (s, 3H), 4.03 (s, 3H), 2.00-3.95 (m, 6H), 7.40-7.70 (m, 3H), 7.87-8.17 (m, 2H).

Production example 11

Production of 3-chloro-5-(1,3-dimethyl-5-methylthio-1H-pyrazol-4-yl)-2-propionyl-2-cyclohexen-1-one (Compound No. 79)

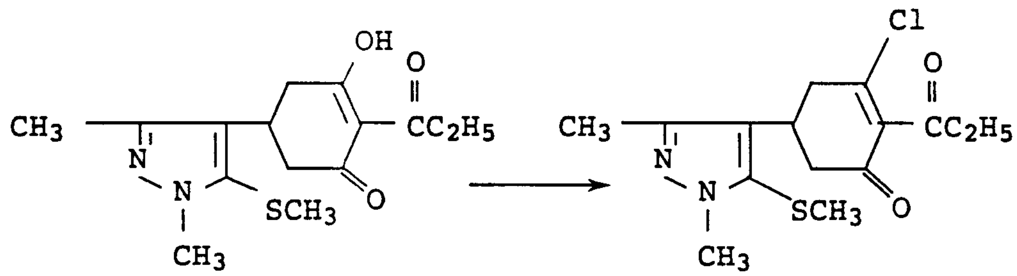

10 Grams (32.5 mmoles) of 3-hydroxy-5-(1,3-dimethyl-5-methylthio-1H-pyrazol-4-yl)-2-propionyl-2-cyclohexen-1-one was dissolved in 29 g (230 mmoles) of oxalyl chloride. The mixture was stirred at room temperature for 1 hour. After completion of the reaction, the reaction solution was poured into 100 ml of ice water, the pH was adjusted to 7 with 1N sodium hydroxide, and the desired product was extracted with three 100-ml portions of ethyl acetate. After drying the extract, the solvent was removed, and the residue obtained was purified by column chromatography on silica gel (ethyl acetate : n-hexane) to obtain 9.5 g of the desired product.

m.p. 141.2 - 142.3 °C

Yield 89.5%

Production example 12

Production of 3-diethylamino-5(1,3-dimethyl-5-methylthio-1H-pyrazol-4-yl)-2-propionyl-2-cyclohexen-1-one (Compound No. 84)

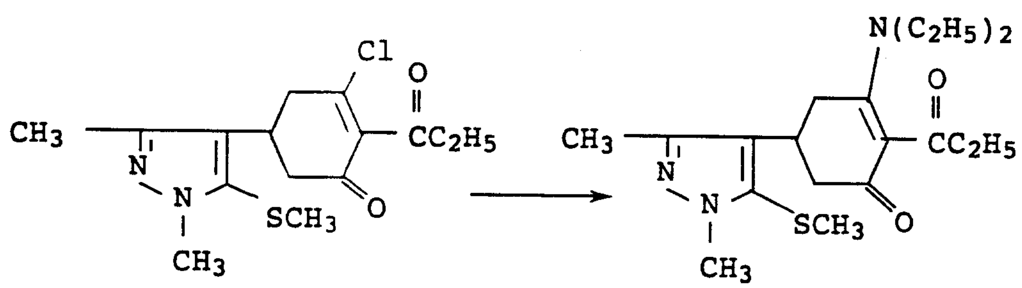

0.6 Gram (1.8 mmoles) of 3-chloro-5-(1,3-dimethyl-5-methylthio-1H-pyrazol-4-yl)-2-propionyl-2-cyclohexen-1-one and 0.16 g (2.2 mmoles) of diethylamine were dissolved in 30 ml of dry tetrahydrofuran, and 0.22 g (2.2 mmoles) of triethylamine was added. The mixture was stirred at room temperature for 4 hours with stirring. After completion of the reaction, the solvent was removed under reduced pressure. After adding 50 ml of water to the residue obtained, the pH was adjusted to 5 with a dilute hydrochloric acid, and the product was extracted with three 40-ml portions of ethyl acetate. After drying the extract, the solvent was removed and the residue was purified by column chromatography on silica gel (ethyl acetate : n-hexane) to obtain 0.4 g of the desired product.

nD 1.5729 (20.1 °C)

Yield 61.2%

Production example 13

Production of 5-(3,5-dimethyl-1H-pyrazol-4-yl)-3-hydroxy-2-cyclopropylcarbonyl-2-cyclohexen-1-one (Compound No. 4)

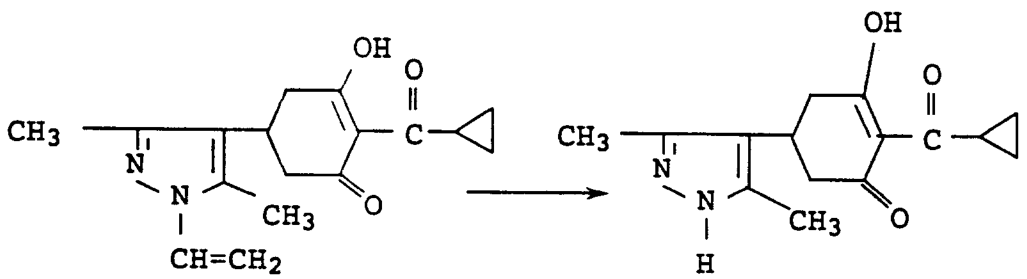

5.8 Grams (19.3 mmoles) of 5-(3,5-dimethyl-1-vinyl-1H-pyrazol-4-yl)-3-hydroxy-2-cyclopropylcarbonyl-2-cyclohexen-1-one was dissolved in a mixed solution of 50 ml of 1,4-dioxane and 50 ml of water, and 4.0 g (41.6 mmoles) of a 38% hydrochloric acid was added dropwise thereto. After completion of the addition, the mixture was heated under reflux for 3 hours. After completion of the reaction, the reaction solution was allowed to cool to room temperature, and the solvent was removed under reduced pressure. After adding 100 ml of water to the residue obtained, the pH was adjusted to 4 with an aqueous dilute sodium hydroxide solution, and the product was extracted with three 100-ml portions of ethyl acetate. After drying the extract, the solvent was removed under reduced pressure. The residue obtained was purified by column chromatography on silica gel (ethyl acetate) to obtain 4.5 g of the desired product.

m.p. 188.0 - 191.3 °C

Yield 85.1%

Production example 14

Production of 5-(3,5-dimethyl-1-n-propyl-1H-pyrazol-4-yl)-2-cyclopropylcarbonyl-3-hydroxy-2-cyclohexen-1-one (Compound No. 207)

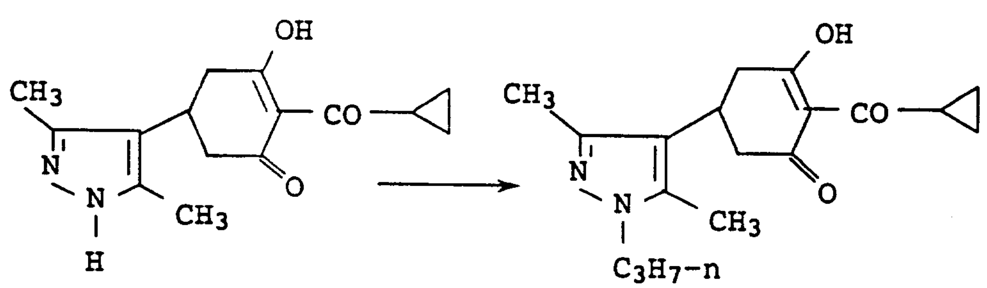

1.0 Gram (3.7 mmoles) of 5-(3,5-dimethyl-1H-pyrazol-4-yl)-2-cyclopropylcarbonyl-3-hydroxy-2-cyclohexen-1-one and 0.9 g (8.0 mmoles) of potassium tert-butoxide were dissolved in 20 ml of dry dimethyl sulfoxide, and 0.8 g (4.4 mmoles) of n-propyl iodide was added dropwise thereto. After completion of the addition, the mixture was stirred at room temperature for 10 hours.

After completion of the reaction, 100 ml of water was added to the reaction solution, and the resulting aqueous solution was washed with two 50-ml portions of ether. The aqueous layer was adjusted to a pH of 4 with an dilute hydrochloric acid, and the product was extracted with three 100-ml portions of ethyl acetate. After drying the extract, the solvent was removed and the residue was purified by column chromatography on silica gel (ethyl acetate : n-hexane) to obtain 1.0 g of the desired product.

m.p. 79.7 - 79.9 °C

Yield 85.5%

Production example 15

Production of 5-(3,5-dimethyl-1-iso-propyl-1H-pyrazol-4-yl)-2-cyclopropylcarbonyl-3-hydroxy-2-cyclohexen-1-one (Compound No. 232)

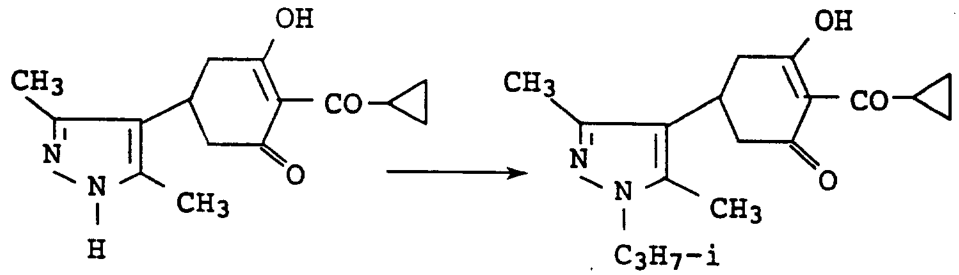

1.0 Gram (3.7 mmoles) of 5-(3,5-dimethyl-1H-pyrazol-4-yl)-2-cyclopropylcarbonyl-3-hydroxy-2-cyclohexen-1-one and 0.9 g (8.0 mmoles) of potassium tert-butoxide were dissolved in 20 ml of dry dimethyl sulfoxide, and 0,8 g (4.4 mmoles) of isopropyl iodide was added dropwise thereto. After completion of the addition, the mixture was stirred at room temperature for 9 hours.

After completion of the reaction, 100 ml of water was added to the reaction solution, and the resulting aqueous solution was washed with two 50-ml portions of ether. The aqueous layer was adjusted to a pH of 4 with a dilute hydrochloric acid, and the desired product was extracted with three 100-ml portions of ethyl acetate. After drying the extract, the solvent was removed and the residue was purified by column chromatography on silica gel (ethyl acetate : n-hexane) to obtain 1.1 g of the desired product.

m.p. 97.2 - 99.6 ° C

Yield 95.3%

Production example 16

Production of 5-(3,5-dimethyl-1-iso-butyl-1H-pyrazol-4-yl)-2-cyclopropylcarbonyl-3-hydroxy-2-cyclohexen-1-one (Compound No. 248)

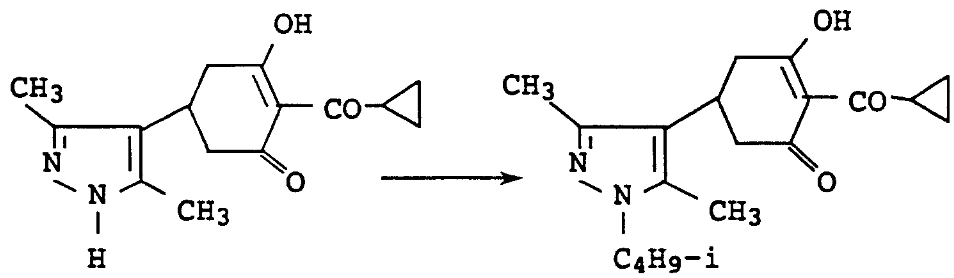

0.5 Gram (1.8 mmoles) of 5-(3,5-dimethyl-1H-pyrazol-4-yl)-2-cyclopropylcarbonyl-3-hydroxy-2-cyclohexen-1-one and 0.4 g (3.8 mmoles) of potassium tert-butoxide were dissolved in 20 ml of dry tetrahydrofuran, and 0.4 g (2.2 mmoles) of isobutyl iodide was added dropwise thereto. After completion of the addition, the mixture was heated under reflux for 8 hours.

After completion of the reaction, 100 ml of water was added to the reaction solution, and the resulting aqueous solution was washed with two 50-ml portions of ether. The aqueous layer was adjusted to a pH of 4 with a dilute hydrochloric acid, and the product was extracted with three 100-ml portions of ethyl acetate. After drying the extract, the solvent was removed and the residue was purified by column chromatography on silica gel (ethyl acetate : n-hexane) to obtain 0.2 g of the desired product.

nD 1.5485 (27.0 ° C)

Yield 33.3%

Production example 17

Production of 5-(3-chloro-1-ethyl-5-methylsulfinyl-1H-pyrazol-4-yl)-2-cyclopropylcarbonyl-3-hydroxy-2-cyclohexen-1-one (Compound No. 152)

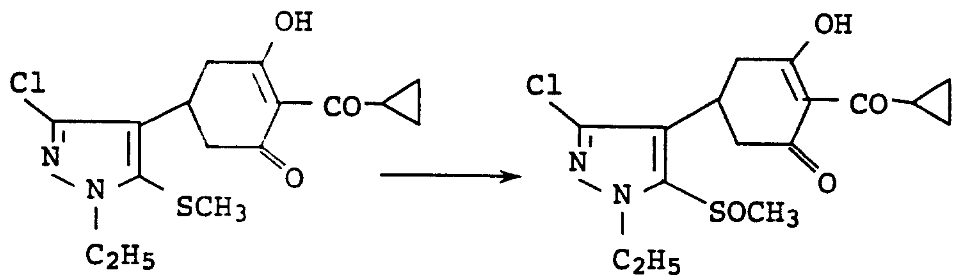

0.98 Gram (2.8 mmoles) of 5-(3-chloro-1-ethyl-5-methylthio-1H-pyrazol-4-yl)-2-cyclopropylcarbonyl-3-hydroxy-2-cyclohexen-1-one was dissolved in 10 ml of ethanol, and 1.02 g (1.7 mmoles) of potassium peroxymonosulfate previously dissolved in 5 ml of water was added thereto. The mixture was stirred at room temperature for 10 hours.

After completion of the reaction, the solvent was removed under reduced pressure, and water was added to the residue. The desired product was extracted with methylene chloride. After drying the extract, the solvent was removed and the residue was purified by column chromatography on silica gel (methanol : ethyl acetate) to obtained 0.57 g of the desired product.

m.p. 140 - 141 °C

Yield 56%

Production example 18

Production of 5-(1-ethyl-3-methyl-5-methylsulfinyl-1H-pyrazol-4-yl)-3-hydroxy-2-cyclopropylcarbonyl-2-cyclohexen-1-one (Compound No. 176)

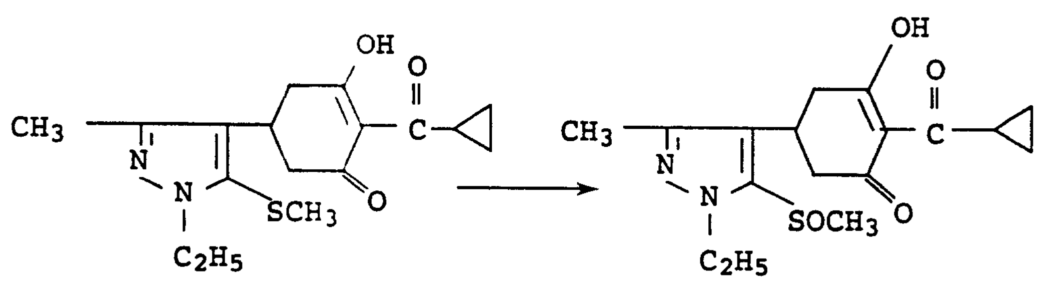

9.3 Grams (27.8 mmoles) of 5-(1-ethyl-3-methyl-5-methylthio-1H-pyrazol-4-yl)-3-hydroxy-2-cyclopropylcarbonyl-2-cyclohexen-1-one was dissolved in 300 ml of ethanol, and 9.4 g (15.3 mmoles) of potassium peroxymonosulfate previously dissolved in 300 ml of water was added thereto. The mixture was stirred at room temperature for 10 hours.

After completion of the reaction, the solvent was removed under reduced pressure, and water was added to the residue. The desired product was extracted with three 200-ml portions of methylene chloride. After drying the extract, the solvent was removed and the residue was purified by column chromatography on silica gel (methanol : ethyl acetate) to obtain 8.0 g of the desired product.

m.p. 130.1 - 131.1 °C

Yield 82.1%

Production example 19

Production of the sodium salt of 5-(1-ethyl-3-methyl-5-methylsulfinyl-1H-pyrazol-4-yl)-3-hydroxy-2-cyclopropylcarbonyl-2-cyclohexen-1-one (Compound No. 177)

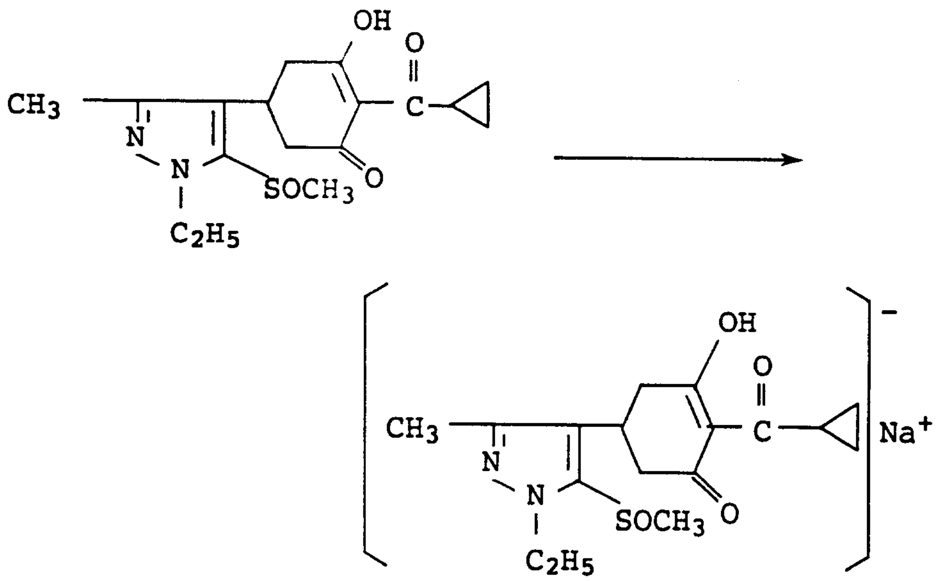

Twenty milligrams (0.87 milliatom) of sodium metal was dissolved in 10 ml of dry ethanol, and to the resulting solution was added 0.3 g (0.86 mmoles) of 5-(1-ethyl-3-methyl-5-methylsulfinyl-1H-pyrazol-4-yl)-3-hydroxy-2-cyclopropylcarbonyl-2-cyclohexen-1-one. The mixture was stirred at room temperature for 1 hour. After completion of the reaction, the solvent was removed, and the residue was dried by heating it to about 70 °C under reduced pressure using calcium chloride as a drier. Thus, 0.32 g of the desired product was obtained.

Physical property : glassy

NMR ($D_2O$/DSS, $\delta$ value, ppm)

0.90-1.00 (m, 4H), 1.35 (t, 3H), 2.44 (s, 3H), 2.25-2.49 (m, 3H), 2.65-2.85 (m, 2H), 3.11 (s, 3H), 3.53-3.65 (m, 1H), 4.39 (q, 2H).

Production example 20

Production of 5-(3,5-dimethyl-1H-pyrazol-4-yl)-2-cyclopropylcarbonyl-3-hydroxy-2-cyclohexen-1-one hydrochloride (Compound No. 5)

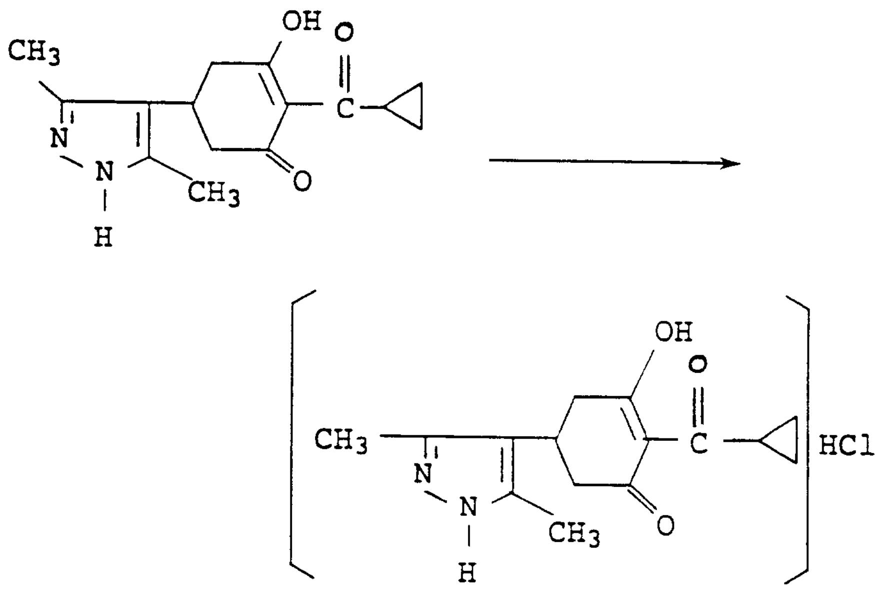

0.5 Gram (1.8 mmoles) of 5-(3,5-dimethyl-1H-pyrazol-4-yl)-2-cyclopropylcarbonyl-3-hydroxy-2-cyclohexen-1-one and 2 g (6.6 mmoles) of a 1,4-dioxane solution containing 12% of hydrochloric acid were added to 20 ml of 1,4-dioxane. The mixture was stirred at room temperature for 10 minutes.

After completion of the reaction, precipitated crystals were collected by filtration, washed with ether and dried at 50 ° C under reduced pressure to obtain 0.5 g of the desired product.

m.p. 280.9-288.2 ° C

Yield 89.5%

Insecticides containing as an active ingredient the cyclohexane derivatives of the present invention represented by the formula (IA) or (IB) or their salts, exhibit a remarkable effect particularly on insect pests belonging to Hemiptera. Among such insect pests, said insecticides are efficacious against sucking insects which parasitize on fruit trees, crops, etc. and cause growth inhibition. For example, said insecticides are efficacious against planthoppers which are typical pests of paddy rice, against whiteflies, suckers, scales and lace bugs which do damage to vegetables (e.g. eggplant, tomato, cucumber, kidney bean, soybean), industrial crops (e.g. cotton, mulberry, tea) and fruit trees (e.g. citrus, grape, pear, apple, Japanese persimmon). Among the above insect pests, Planthoppers include brown rice plant-hopper (Nilaparvata lugens), white-backed rice planthopper (Sogatella furcifera), small brown planthopper (Laodelphax striatellus), etc. Whiteflies include greenhouse whitefly (Trialeurodes vaporariorum), citrus whitefly (Dialeurodes citri), citrus spiny whitefly (Aleurocanthus spiniferus), grape whitefly (Aleurolobus taonabae), sweetpotato whitefly (Bemisia tabaci), strawberry whitefly (Trialeurodes packardi), etc. Suckers include pear sucker (Psylla pyrisuga), mulberry sucker (Anomoneura mori), citrus psylla (Diaphorina citri), apple sucker (Psylla mari), etc. Scales include horned wax scale (Ceroplastes pseudoceriferus), cotton citrus scale (Pulvinaria aurantii), camphor scale (Pseudaonidia duplex), San Jose scale (Quadraspidiotus perniciosus), arrowhead scale (Unaspis yanonensis), etc. Lace bugs include pear lace bug (Stephanitis nashi), azalea lace bug (Stephanitis pyrioides), etc.

The insecticides of the present invention specifically exhibit an insecticidal effect on planthoppers infesting paddy field as well as on whiteflies and suckers which are an insect harmful to fruit trees and vegetables. The said insecticides exhibit killing activity particularly against adult stage of those insect pests mentioned above by inhibiting their sucking action on paddy rice, fruit trees, vegetables, etc.

Consequently, the expected effect of the insecticides of the present invention can be expected by treating paddy field water for paddy rice, foliage of fruit trees and soils for vegetables with these insecticides before the generation of these insects harmful to paddy rice, fruit trees, vegetables, etc. so as

to be in time for the season when the generation can be forecast, or at a point when the generation has just been confirmed.

The present invention, however, is not limited to these embodiments alone.

When the cyclohexane derivatives of the present invention represented by the formula (IA) or (IB) or their salts are used as an insecticide, it is a common practice to use them in preparations of a suitable form for use prepared according to the customary methods for production of agricultural chemicals.

That is, prior to use as an insecticide, the cyclohexane derivatives represented by the formula (IA) or (IB) or their salts are formulated into suitable preparation forms, for example suspension formulations, emulsifiable concentrates, liquid formulations, wettable powders, granules, dusts, tablets, etc. by blending them with a suitable inert carrier and if necessary an adjuvant in proper ratios and applying to the resulting blends various treatments such as dissolution, dispersion, suspension, mixing, impregnation, adsorption, sticking, etc.

As the inert carrier usable in the present invention, any of solid carriers and liquid carriers may be used. Materials usable as solid carriers include soybean powder, cereal flour, wood powder, bark powder, saw dust, tobacco stalk powder, walnut shell powder, bran, cellulose powder, residues after extraction of vegetable essence, synthetic polymers (e.g. crushed synthetic resins), clays (e.g. kaolin, bentonite, acid clay), talcs (e.g. talc, pyrophyllite), silicas [e.g. diatomaceous earth, silica sand, mica, white carbon (synthetic highly dispersed silicic acid, also called finely divided hydrated silica or hydrated silicic acid, some of which contain calcium silicate as a main component)], powders of inorganic minerals (e.g. activated carbon, sulfur, pumice, calcined diatomaceous earth, brick, fly ash, sand, calcium carbonate, calcium phosphate), chemical fertilizers (e.g. ammonium sulfate, ammonium phosphate, ammonium nitrate, urea, ammonium chloride), compost, etc. These materials may be used alone or in combination.

Materials usable as liquid carriers are selected from the group consisting of liquids which themselves have a solvent power and liquids which do not have a solvent power, but can disperse active ingredients with the aid of adjuvants. For example, the following carriers, which may be used alone or in combination, can be given as typical examples : Water, alcohols (e.g. methanol, ethanol, isopropanol, butanol, ethylene glycol), ketones (e.g. acetone, methyl ethyl ketone, methyl isobutyl ketone, diisobutyl ketone, cyclohexanone), ethers (e.g. ethyl ether, dioxane, Cellosolve, dipropyl ether, tetrahydrofuran), aliphatic hydrocarbons (e.g. gasoline, mineral oils), aromatic hydrocarbons (e.g. benzene, toluene, xylene, solvent naphtha, alkylnaphthalenes), halogenated hydrocarbons (e.g. dichloroethane, chloroform, carbon tetrachloride, chlorinated benzenes), esters (e.g. ethyl acetate, diisopropyl phthalate, dibutyl phthalate, dioctyl phthalate), amides (e.g. dimethylformamide, diethylformamide, dimethylacetamide), nitriles (e.g. acetonitrile), dimethyl sulfoxide, etc.

As other adjuvants, the following typical ones can be given. These adjuvants are used according to objects. They are used alone or in combination, but in some cases, it is also possible not to use them at all.

For the purpose of emulsification, dispersion, solubilization and/or wetting of active ingredients, for example the following surface active agents may be used : Polyoxyethylene alkyl ethers, polyoxyethylene alkylaryl ethers, polyoxyethylene higher fatty acid esters, polyoxyethylene resinates, polyoxyethylene sorbitan monolaurate, polyoxyethylene sorbitan monooleate, alkylarylsulfonates, naphthalenesulfonic acid condensation products, ligninsulfonates, higher alcohol sulfate esters, etc.

For the purpose of stabilizing the dispersion of active ingredients, tackifying and/or binding them, for example the following adjuvants may be used : Casein, gelatin, starch, methyl cellulose, carboxymethyl cellulose, gum arabic, polyvinyl alcohol, turpentine, bran oil, bentonite, ligninsulfonates, etc.

For the purpose of improving the flowability of the solid insecticidal products, for example the following adjuvants may be used : Waxes, stearates, alkyl phosphates, etc.

Adjuvants such as for example naphthalene-sulfonic acid condensation products, polyphosphates, etc. may be used as a peptizers for dispersible insecticidal products.

Adjuvants such as for example silicon oils, etc. may be used as a defoaming agent.

The proportion of the active ingredients blended may be varied as occasion demands. For example, a proportion of 0.01 to 50 wt.% is suitable for dusts and granules, and it is also the same with emulsifiable concentrates and wettable powders.

For the purpose of controlling insect pests using insecticides containing as an active ingredient the cyclohexane derivatives represented by the formula (IA) or (IB) or their salts, it is desirable to apply insecticidally effective amounts of the insecticides themselves or in a suitable aqueous dilution form or suspension form, directly to the insects or places wherein the generation or Propagation of the insects is not desirable. For example, in order to control planthoppers which are an insect harmful to rice plant, the insecticides are applied to the foliage of rice plants or to soil or water in paddy fields wherein rice plants are planted.

The dosage rate of the insecticides containing as an active ingredient the cyclohexane derivatives represented by the formula (IA) or (IB) or their salts, depends upon various factors such as for example objects of application, insect pests to be controlled, emergence/growth states of crops, generation tendency of insect pests, weather, environmental conditions, preparation forms and how, where and when the insecticides are applied. It is desirable, however, to properly select the dosage rate according to the objects from a range of 1 g to 3 kg/10 ares as an active ingredient.

The insecticides of the present invention containing as an active ingredient the cyclohexane derivatives represented by the formula (IA) or (IB) or their salts can also be used in mixture with other insecticides or fungicides for the purpose of extending the range of insect pests to be controlled and a period of time wherein insect pests can be controlled, or for the purpose of reducing the dosage rate.

Typical formulation examples and test examples of the present invention will be shown below, but the present invention is not limited to these examples.

Formulation example 1

| | |
|---|---|
| Compound No. 1 | 50 parts |
| Xylene | 40 parts |
| Mixture of polyoxyethylene nonylphenyl ether and calcium alkylbenzenesulfonate | 10 parts |

The above ingredients are uniformly mixed to obtain an emulsifiable concentrate.

Formulation example 2

| | |
|---|---|
| Compound No. 15 | 3 parts |
| Clay powder | 82 parts |
| Diatomaceous earth powder | 15 parts |

The above ingredients are uniformly mixed to obtain a dust.

Formulation example 3

| | |
|---|---|
| Compound No. 58 | 5 parts |
| Powdery mixture of bentonite and clay | 90 parts |
| Calcium ligninsulfonate | 5 parts |

The above ingredients are uniformly mixed, kneaded with a suitable amount of wa er, granulated and dried to obtain a granule.

Formulation example 4

| | |
|---|---|
| Compound No. 72 | 20 parts |
| Mixture of kaolin and synthetic highly dispersed silicic acid | 75 parts |
| Mixture of polyoxyethylene nonylphenyl ether and calcium alkylbenzenesulfonate | 5 parts |

The above ingredients are uniformly mixed to obtain a wettable powder.

Test example 1 Insecticidal test on brown rice planthopper (Nilaparvata lugens)

Rice seedlings (var., Kimmaze) were dipped for 30 seconds in an insecticidal solution containing 200 ppm of the present compound as an active ingredient. After air-drying, the seedlings were put in a glass

test tube, and each of them was inoculated with ten third instar larvae of brown rice planthopper. The test tube was then stoppered with cotton. Eight days after inoculation, the numbers of the dead and alive of the larvae were examined, and the mortality was calculated according to the following equation. The insecticidal activity was judged based on the following standard.

The test was carried out in a constant-temperature room kept at 25 °C.

$$\text{Mortality (\%)} = \frac{\text{Number of killed larvae}}{\text{Number of inoculated larvae}} \times 100$$

Standard of judgement :

Mortality

A : 90% or more
B : 70% to less than 90%
C : 50% to less than 70%
D : Less than 50%

The results are shown in Table 4.

Table 4

| Compound No. | Concentration (ppm) | Judgement | Compound No. | Concentration (ppm) | Judgement |
|---|---|---|---|---|---|
| 1 | 200 | A | 40 | 200 | C |
| 2 | 200 | A | 41 | 200 | A |
| 3 | 200 | A | 43 | 200 | A |
| 4 | 200 | A | 45 | 200 | A |
| 5 | 200 | A | 48 | 200 | A |
| 6 | 200 | A | 52 | 200 | B |
| 7 | 200 | C | 67 | 200 | A |
| 8 | 200 | A | 68 | 200 | A |
| 9 | 200 | C | 69 | 200 | B |
| 10 | 200 | C | 70 | 200 | A |
| 11 | 200 | B | 73 | 200 | C |
| 13 | 200 | A | 74 | 200 | C |
| 14 | 200 | A | 83 | 200 | A |
| 15 | 200 | A | 84 | 200 | C |
| 16 | 200 | A | 85 | 200 | A |
| 17 | 200 | A | 86 | 200 | A |
| 19 | 200 | A | 87 | 200 | A |
| 22 | 200 | A | 92 | 200 | A |
| 24 | 200 | A | 94 | 200 | A |
| 28 | 200 | B | 95 | 200 | C |
| 34 | 200 | B | 96 | 200 | A |
| 36 | 200 | A | 98 | 200 | A |
| 38 | 200 | C | 99 | 200 | C |
| 39 | 200 | A | 100 | 200 | C |

Cont'd

Table 4 (Cont'd)

| | | | | | |
|---|---|---|---|---|---|
| 101 | 200 | B | 137 | 200 | C |
| 102 | 200 | A | 146 | 200 | B |
| 103 | 200 | A | 147 | 200 | A |
| 104 | 200 | A | 148 | 200 | B |
| 105 | 200 | A | 149 | 200 | A |
| 106 | 200 | A | 150 | 200 | B |
| 107 | 200 | A | 151 | 200 | A |
| 108 | 200 | A | 152 | 200 | A |
| 109 | 200 | A | 153 | 200 | A |
| 110 | 200 | A | 155 | 200 | A |
| 112 | 200 | A | 156 | 200 | A |
| 113 | 200 | A | 157 | 200 | A |
| 114 | 200 | A | 158 | 200 | A |
| 116 | 200 | A | 159 | 200 | A |
| 118 | 200 | A | 160 | 200 | A |
| 119 | 200 | C | 161 | 200 | A |
| 121 | 200 | A | 162 | 200 | B |
| 123 | 200 | A | 163 | 200 | A |
| 125 | 200 | A | 164 | 200 | A |
| 128 | 200 | A | 165 | 200 | A |
| 129 | 200 | A | 166 | 200 | A |
| 131 | 200 | A | 167 | 200 | A |
| 133 | 200 | A | 168 | 200 | A |
| 134 | 200 | A | 169 | 200 | A |
| 135 | 200 | A | 170 | 200 | A |
| 136 | 200 | A | 171 | 200 | A |

Cont'd

Table 4 (Cont'd)

| | | | | | |
|---|---|---|---|---|---|
| 172 | 200 | A | 198 | 200 | A |
| 173 | 200 | A | 199 | 200 | B |
| 174 | 200 | A | 200 | 200 | A |
| 175 | 200 | A | 201 | 200 | A |
| 176 | 200 | A | 202 | 200 | A |
| 177 | 200 | A | 203 | 200 | A |
| 178 | 200 | A | 204 | 200 | A |
| 179 | 200 | A | 205 | 200 | A |
| 180 | 200 | A | 206 | 200 | A |
| 181 | 200 | A | 207 | 200 | A |
| 182 | 200 | A | 208 | 200 | A |
| 183 | 200 | A | 209 | 200 | A |
| 184 | 200 | A | 210 | 200 | A |
| 185 | 200 | A | 211 | 200 | A |
| 186 | 200 | A | 212 | 200 | A |
| 187 | 200 | A | 213 | 200 | A |
| 188 | 200 | A | 214 | 200 | A |
| 198 | 200 | A | 215 | 200 | A |
| 190 | 200 | A | 216 | 200 | A |
| 191 | 200 | A | 217 | 200 | A |
| 192 | 200 | A | 222 | 200 | A |
| 193 | 200 | A | 223 | 200 | A |
| 194 | 200 | B | 224 | 200 | A |
| 195 | 200 | A | 225 | 200 | A |
| 196 | 200 | A | 226 | 200 | A |
| 197 | 200 | A | 227 | 200 | A |

Cont'd

Table 4 (Cont'd)

| | | | | | |
|---|---|---|---|---|---|
| 228 | 200 | C | 258 | 200 | A |
| 229 | 200 | A | 260 | 200 | A |
| 230 | 200 | A | 264 | 200 | A |
| 232 | 200 | A | 265 | 200 | A |
| 233 | 200 | A | 266 | 200 | A |
| 235 | 200 | A | 267 | 200 | A |
| 236 | 200 | A | 268 | 200 | B |
| 237 | 200 | A | 269 | 200 | A |
| 238 | 200 | A | 270 | 200 | A |
| 239 | 200 | A | 271 | 200 | A |
| 240 | 200 | A | 272 | 200 | A |
| 241 | 200 | A | 273 | 200 | A |
| 242 | 200 | A | 274 | 200 | A |
| 243 | 200 | A | 275 | 200 | C |
| 244 | 200 | A | 276 | 200 | A |
| 245 | 200 | A | 277 | 200 | A |
| 246 | 200 | A | 278 | 200 | A |
| 247 | 200 | A | 280 | 200 | A |
| 248 | 200 | A | 281 | 200 | A |
| 249 | 200 | A | 285 | 200 | C |
| 250 | 200 | A | 295 | 200 | A |
| 253 | 200 | A | 297 | 200 | A |
| 254 | 200 | A | 298 | 200 | A |
| 255 | 200 | A | 300 | 200 | A |
| 256 | 200 | A | 301 | 200 | A |
| 257 | 200 | A | 304 | 200 | C |

Cont'd

Table 4 (Cont'd)

| | | | | | |
|---|---|---|---|---|---|
| 314 | 200 | A | 324 | 200 | C |
| 315 | 200 | C | 325 | 200 | A |
| 316 | 200 | B | 326 | 200 | A |
| 317 | 200 | A | 328 | 200 | A |
| 318 | 200 | A | 330 | 200 | A |
| 319 | 200 | C | 332 | 200 | A |
| 320 | 200 | C | 333 | 200 | A |
| 322 | 200 | A | 334 | 200 | C |
| 323 | 200 | A | 336 | 200 | C |

Test example 2 Insecticidal test on greenhouse whitefly (Trialeurodes vaporariorum)

Small leaves of tomato (var., Ponteroza) were dipped for 30 seconds in an insecticidal solution containing 100 ppm of the present compound as an active ingredient. After air-drying, the petioles of the leaves were wrapped in wetted absorbent cotton and put in a glass cylinder of 5 cm in diameter and 20 cm in height. Ten adults of greenhouse whitefly were released on each leaf, and the upper end of the cylinder was covered with gauze. Seven days after releasement, the dead and alive of the adults were examined. The mortality was calculated according to Test example 1, and judgement was made similarly.

The test was carried out in a constant-temperature room kept at 25 °C.

The results are shown in Table 5.

Table 5

| Compound No. | Concentration (ppm) | Judgement | Compound No. | Concentration (ppm) | Judgement |
|---|---|---|---|---|---|
| 4 | 100 | A | 201 | 100 | A |
| 13 | 100 | A | 206 | 100 | C |
| 17 | 100 | A | 207 | 100 | A |
| 24 | 100 | C | 215 | 100 | A |
| 104 | 100 | C | 220 | 100 | A |
| 144 | 100 | B | 239 | 100 | A |
| 157 | 100 | C | 248 | 100 | A |
| 160 | 100 | B | 272 | 100 | A |
| 165 | 100 | A | 278 | 100 | B |
| 176 | 100 | A | 322 | 100 | B |

**Claims**

1. A cyclohexane derivative represented by the formula (IA),

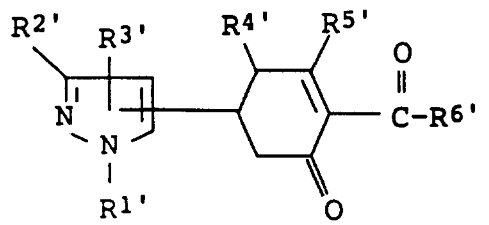

(IA)

wherein $R^{1'}$ represents a hydrogen atom, ethyl group, n-propyl group, n-butyl group, iso-butyl group or sec-butyl group, $R^{2'}$ represents a hydrogen atom, halogen atom or $C_1$-$C_5$ alkyl group, $R^{3'}$ represents a halogen atom, $C_1$-$C_5$ alkyl group, $C_1$-$C_5$ alkoxy group, $C_1$-$C_5$ alkylthio group, $C_1$-$C_5$ alkylsulfinyl group, $C_1$-$C_5$ alkylsulfonyl group or -N($R^{7'}$)$R^{8'}$ (in which each of $R^{7'}$ and $R^{8'}$, which may be the same or different, represents a hydrogen atom or $C_1$-$C_3$ alkyl group and $R^{7'}$ and $R^{8'}$ may be linked together to form 4 to 6 methylene groups), $R^{4'}$ represents a hydrogen atom, $C_1$-$C_5$ alkyl group or $C_3$-$C_5$ alkoxycarbonyl group, $R^{5'}$ represents a hydroxy group, phenylcarbonyloxy group or phenylcarbonyloxy group of which the phenyl ring has 1 to 5 substituents, which may be the same or different, selected from the group consisting of a halogen atom, $C_1$-$C_3$ alkyl group and $C_1$-$C_3$ alkoxy group, and $R^{6'}$ represents a $C_1$-$C_5$ alkyl group, $C_3$-$C_6$ cycloalkyl group or cycloalkyl group substituted with a $C_1$-$C_3$ alkyl group, provided that when $R^{1'}$ is an ethyl group, $R^{2'}$ is a methyl group, $R^{3'}$ is a halogen atom or methylthio group, $R^{4'}$is a hydrogen atom and $R^{5'}$ is a hydroxy group at the same time, $R^{6'}$ is a substituent other than an ethyl and n-propyl groups, or its salts.

2. A cyclohexane derivative according to Claim 1, wherein $R^{1'}$ represents an ethyl group or n-propyl group, $R^{2'}$ represents a hydrogen atom, halogen atom or $C_1$-$C_5$ alkyl group, $R^{3'}$ represents a $C_1$-$C_5$ alkyl group, $C_1$-$C_5$ alkylthio group, $C_1$-$C_5$ alkylsulfinyl group, $C_1$-$C_5$ alkylsulfonyl group or -N($R^{7'}$)$R^{8'}$ (in which each of $R^{7'}$ and R8', which may be the same or different, represents a hydrogen atom or $C_1$-$C_3$ alkyl group), $R^{4'}$ represents a hydrogen atom, $R^{5'}$ represents a hydroxy group, and $R^{6'}$ represents a $C_1$-$C_5$ alkyl group, $C_3$-$C_6$ cycloalkyl group or cycloalkyl group substituted with a $C_1$-$C_3$ alkyl group, provided that when $R^{1'}$ is an ethyl group, $R^{2'}$ is a methyl group, $R^{3'}$ is a methylthio group, $R^{4'}$ is a hydrogen atom and $R^{5'}$ is a hydroxy group at the same time, $R^{6'}$ is a substituent other than an ethyl and n-propyl groups, or its salts.

3. A method for producing a cyclohexane derivative represented by the formula,

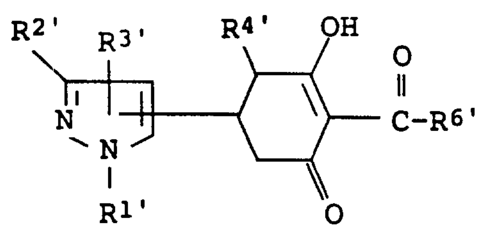

wherein $R^{1'}$ represents a hydrogen atom, ethyl group, n-propyl group, n-butyl group, iso-butyl group or sec-butyl group, $R^{2'}$ represents a hydrogen atom, halogen atom or $C_1$-$C_5$ alkyl group, $R^{3'}$ represents a halogen atom, $C_1$-$C_5$ alkyl group, $C_1$-$C_5$ alkoxy group, $C_1$-$C_5$ alkylthio group, $C_1$-$C_5$ alkylsulfinyl group, $C_1$-$C_5$ alkylsulfonyl group or -N($R^{7'}$)$R^{8'}$ (in which each of $R^{7'}$ and $R^{8'}$, which may be the same or different, represents a hydrogen atom or $C_1$-$C_3$ alkyl group and $R^{7'}$ and $R^{8'}$ may be linked together to form 4 to 6 methylene groups), $R^{4'}$ represents a hydrogen atom, $C_1$-$C_5$ alkyl group or $C_2$-$C_5$ alkoxycarbonyl group, $R^{6'}$ represents a $C_1$-$C_5$ alkylgroup, $C_3$-$C_6$ cycloalkyl group or cycloalkyl group substituted with a $C_1$-$C_3$ alkyl group, provided that when $R^{1'}$ is an ethyl group, $R^{2'}$ is a methyl group, $R^{3'}$ is a halogen atom or methylthio group and $R^{4'}$ is a hydrogen atom at the same time, $R^{6'}$ is a

substitutent other than an ethyl and n-propyl groups, or its salts which comprises subjecting a cyclohexane compound represented by the formula ,

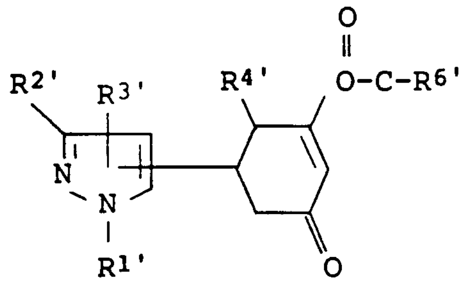

wherein $R^{1'}$, $R^{2'}$, $R^{3'}$, $R^{4'}$ and $R^{6'}$ are as defined above, to rearrangement reaction in the presence of a catalyst.

**4.** The use, as the active ingredient of an insecticide, of a cyclohexane derivative represented by the formula (IB),

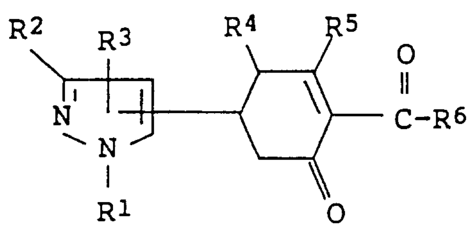

(IB)

wherein $R^1$ represents a hydrogen atom, $C_1$-$C_{16}$ alkyl group, $C_1$-$C_5$ haloalkyl group, $C_2$-$C_5$ alkenyl group, $C_2$-$C_5$ alkoxyalkyl group, phenyl group, phenyl group having 1 to 5 substituents, which may be the same or different, selected from the group consisting of a halogen atom, $C_1$-$C_3$ alkyl group and $C_1$-$C_3$ alkoxy group, benzyl group or benzyl group having 1 to 5 substituents, which may be the same or different, selected from the group consisting of a halogen atom, $C_1$-$C_3$ alkyl group and $C_1$-$C_3$ alkoxy group, $R^2$ represents a hydrogen atom, halogen atom, $C_1$-$C_5$ alkyl group, $C_1$-$C_5$ haloalkyl group, phenyl group or phenyl group having 1 to 5 substituents, which may be the same or different, selected from the group consisting of a halogen atom, $C_1$-$C_3$ alkyl group and $C_1$-$C_3$ alkoxy group, $R^3$ represents a hydrogen atom, halogen atom, $C_1$-$C_5$ alkyl group, $C_1$-$C_5$ haloalkyl group, $C_1$-$C_5$ alkoxy group, $C_1$-$C_5$ haloalkoxy group, $C_1$-$C_5$ alkylthio group, $C_1$-$C_5$ haloalkylthio group, $C_1$-$C_5$ alkylsulfinyl group, $C_1$-$C_5$ haloalkylsulfinyl group, $C_1$-$C_5$ alkylsulfonyl group, $C_1$-$C_5$ haloalkylsulfonyl group, phenoxy group, phenoxy group having 1 to 5 substituents, which may be the same or different, selected from the group consisting of a halogen atom, $C_1$-$C_3$ alkyl group and $C_1$-$C_3$ alkoxy group, phenylthio group, phenylthio group having 1 to 5 substituents, which may be the same or different, selected from the group consisting of a halogen atom, $C_1$-$C_3$ alkyl group and $C_1$-$C_3$ alkoxy group, $-N(R^7)R^8$ (in which each of $R^7$ and $R^8$, which may be the same or different, represents a hydrogen atom or $C_1$-$C_3$ alkyl group and $R^7$ and $R^8$ may be linked together to form 4 to 6 methylene groups), or $C_2$-$C_5$ alkylcarbonyloxy group, $R^4$ represents a hydrogen atom, $C_1$-$C_5$ alkyl group or $C_2$-$C_5$ alkoxycarbonyl group, $R^5$ represents a hydroxy group, halogen atom, $C_1$-$C_5$ alkylthio group, alkenylthio group containing up to 5 carbon atoms, $-N(R^9)R^{10}$ (in which each of $R^9$ and $R^{10}$, which may be the same or different, represents a hydrogen atom, $C_1$-$C_3$ alkyl group or alkenyl group containing up to 3 carbon atoms), $C_2$-$C_8$ alkylcarbonyloxy group, $C_3$-$C_6$ cycloalkylcarbonyloxy group, phenylcarbonyloxy group, or phenylcarbonyloxy group of which the phenyl ring has 1 to 5 substituents, which may be the same or different, selected from the group consisting of a halogen atom, $C_1$-$C_3$ alkyl group and $C_1$-$C_3$ alkoxy group, and $R^6$ represents a $C_1$-$C_{18}$ alkyl group, $C_3$-$C_6$ cycloalkyl group, cycloalkyl group having substituents, which may be the same or different, selected from the group consisting of a halogen atom and $C_1$-$C_3$ alkyl group, $C_2$-$C_{17}$ alkenyl group, $C_2$-$C_8$ alkoxyalkyl group, $C_2$-$C_8$ alkylthioalkyl group, phenoxyalkyl group, or phenoxyalkyl group of which the phenyl ring has 1 to 5 substituents, which may be the same

or different, selected from the group consisting of a halogen atom, $C_1$-$C_3$ alkyl group and $C_1$-$C_3$ alkoxy group, provided that when $R^1$ = H, $R^2$ = methyl, $R^3$ = 5-Cl, $R^4$ = H and $R^5$ = OH then $R^6$ may be benzyl, or its salts.

**5.** An insecticide according to Claim 4, wherein in the formula (IB), $R^1$ represents a $C_1$-$C_5$ alkyl group, $C_1$-$C_5$ haloalkyl group or $C_2$-$C_5$ alkenyl group, $R^2$ represents a hydrogen atom, halogen atom, $C_1$-$C_5$ alkyl group or $C_1$-$C_5$ haloalkyl group, $R^3$ represents a $C_1$-$C_5$ alkyl group, $C_1$-$C_5$ alkoxy group, $C_1$-$C_5$ alkylthio group, $C_1$-$C_5$ haloalkylthio group, $C_1$-$C_5$ alkylsulfinyl group, $C_1$-$C_5$ alkylsulfonyl group or -N($R^7$)$R^8$ (in which each of $R^7$ and $R^8$, which may be the same or different, represents a hydrogen atom or $C_1$-$C_3$ alkyl group), $R^4$ represents a hydrogen atom, $R^5$ represents a hydroxy group, and $R^6$ represents a $C_1$-$C_5$ alkyl group, $C_3$-$C_6$ cycloalkyl group or cycloalkyl group having substituents, which may be the same or different, selected from the group consisting of a halogen atom and $C_1$-$C_3$ alkyl group.

**6.** An insecticide according to Claim 5, wherein in the formula (IB), $R^1$ represents a $C_1$-$C_5$ alkyl group, $R^2$ represents a $C_1$-$C_5$ alkyl group, $R^3$ represents a $C_1$-$C_5$ alkyl group, $C_1$-$C_5$ alkylthio group, $C_1$-$C_5$ alkylsulfinyl group or -N($R^7$)$R^8$ (in which each of $R^7$ and $R^8$, which may be the same or different, represents a hydrogen atom $C_1$-$C_3$ alkyl group), $R^4$ represents a hydrogen atom, $R^5$ represents a hydroxy group, and $R^6$ represents a $C_3$-$C_6$ cycloalkyl group.

**7.** An insecticide according to Claim 4, 5 or 6 which is an insecticide for controlling insect pests belonging to Hemiptera.

**8.** An insecticidal method which comprises applying an insecticide containing as an active ingredient a cyclohexane derivative represented by the formula (IB),

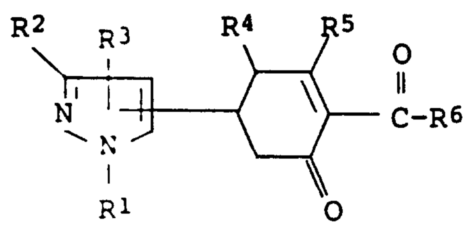

(IB)

wherein $R^1$ represents a hydrogen atom, $C_1$-$C_{16}$ alkyl group, $C_1$-$C_5$ haloalkyl group, $C_2$-$C_5$ alkenyl group, $C_2$-$C_5$ alkoxyalkyl group, phenyl group, phenyl group having 1 to 5 substituents, which may be the same or different, selected from the group consisting of a halogen atom, $C_1$-$C_3$ alkyl group and $C_1$-$C_3$ alkoxy group, benzyl group or benzyl group having 1 to 5 substituents, which may be the same or different, selected from the group consisting of a halogen atom, $C_1$-$C_3$ alkyl group and $C_1$-$C_3$ alkoxy group, $R^2$ represents a hydrogen atom, halogen atom, $C_1$-$C_5$ alkyl group, $C_1$-$C_5$ haloalkyl group, phenyl group or phenyl group having 1 to 5 substituents, which may be the same or different, selected from the group consisting of a halogen atom, $C_1$-$C_3$ alkyl group and $C_1$-$C_3$ alkoxy group, $R^3$ represents a hydrogen atom, halogen atom, $C_1$-$C_5$ alkyl group, $C_1$-$C_5$ haloalkyl group, $C_1$-$C_5$ alkoxy group, $C_1$-$C_5$ haloalkoxy group, $C_1$-$C_5$ alkylthio group, $C_1$-$C_5$ haloalkylthio group, $C_1$-$C_5$ alkylsulfinyl group, $C_1$-$C_5$ haloalkylsulfinyl group, $C_1$-$C_5$ alkylsulfonyl group, $C_1$-$C_5$ haloalkylsulfonyl group, phenoxy group, phenoxy group having 1 to 5 substituents, which may be the same or different, selected from the group consisting of a halogen atom, $C_1$-$C_3$ alkyl group and $C_1$-$C_3$ alkoxy group, phenylthio group, phenylthio group having 1 to 5 substituents, which may be the same or different, selected from the group consisting of a halogen atom, $C_1$-$C_3$ alkyl group and $C_1$-$C_3$ alkoxy group, -N($R^7$)$R^8$ (in which each of $R^7$ and $R^8$, which may be the same or different, represents a hydrogen atom or $C_1$-$C_3$ alkyl group, and $R^7$ and $R^8$ may be linked together to form 4 to 6 methylene groups), or $C_2$-$C_5$ alkylcarbonyloxy group, $R^4$ represents a hydrogen atom, $C_1$-$C_5$ alkyl group or $C_2$-$C_5$ alkoxycarbonyl group, $R^5$ represents a hydroxy group, halogen atom, $C_1$-$C_5$ alkylthio group, alkenylthio group containing up to 5 carbon atoms, -N($R^9$)$R^{10}$ (in which each of $R^9$ and $R^{10}$, which may be the same or different, represents a hydrogen atom, $C_1$-$C_3$ alkyl group or alkenyl group containing up to 3 carbon atoms), $C_2$-$C_8$ alkylcarbonyloxy group, $C_3$-$C_6$ cycloalkylcarbonyloxy group, phenylcarbonyloxy group, or phenylcarbonyloxy group of which the phenyl ring has 1 to 5 substituents, which may be the same or different, selected from the group consisting of a halogen atom, $C_1$-$C_3$ alkyl group and $C_1$-$C_3$ alkoxy group, and

$R^6$ represents a $C_1$-$C_{18}$ alkyl group $C_3$-$C_6$ cycloalkyl group, cycloalkyl group having substituents, which may be the same or different, selected from the group consisting of a halogen atom and $C_1$-$C_3$ alkyl group, $C_2$-$C_{17}$ alkenyl group, $C_2$-$C_8$ alkoxyalkyl group, $C_2$-$C_8$ alkylthioalkyl group, phenoxyalkyl group, or phenoxyalkyl group of which the phenyl ring has 1 to 5 substituents, which may be the same or different, selected from the group consisting of a halogen atom, $C_1$-$C_3$ alkyl group and $C_1$-$C_3$ alkoxy group, provided that when $R^1$ = H, $R^2$ = methyl, $R^3$ = 5-Cl $R^4$ = H and $R^5$ = OH the $R^6$ may be benzyl, or its salts to plants on which insects harmful to the plants have been generated or generation of the insects is forecast, or to places around the plants in an amount ranging from 1 g to 3 kg per 10 ares as the amount of the active ingredient.

**9.** An insecticidal method according to Claim 8, wherein in the formula (IB), $R^1$ represents a $C_1$-$C_5$ alkyl group, $C_1$-$C_5$ haloalkyl group or $R^2$ represents a hydrogen atom, halogen atom, $C_1$-$C_5$ alkyl group or $C_1$-$C_5$ haloalkyl group, $R^3$ represents a $C_1$-$C_5$ alkyl group, $C_1$-$C_5$ alkoxy group, $C_1$-$C_5$ alkylthio group, $C_1$-$C_5$ haloalkylthio group, $C_1$-$C_5$ alkylsulfinyl group, $C_1$-$C_5$ alkylsulfonyl group or -N($R^7$)$R^8$ (in which each of $R^7$ and $R^8$, which may be the same or different, represents a hydrogen atom or $C_1$-$C_3$ alkyl group), $R^4$ represents a hydrogen atom, $R^5$ represents a hydroxy group, and $R^6$ represents a $C_1$-$C_5$ alkyl group, $C_3$-$C_6$ cycloalkyl group or cycloalkyl group having substituents, which may be the same or different, selected from the group consisting of a halogen atom and $C_1$-$C_3$ alkyl group.

**10.** An insecticidal method according to Claim 9, wherein in the formula (IB), $R^1$ represents a $C_1$-$C_5$ alkyl group, $R^2$ represents a $C_1$-$C_5$ alkyl group, $R^3$ represents a $C_1$-$C_5$ alkyl group, $C_1$-$C_5$ alkylthio group, $C_1$-$C_5$ alkylsulfonyl group or -N($R^7$)$R^8$ (in which each of $R^7$ and $R^8$, which may be the same or different, represents a hydrogen atom or $C_1$-$C_3$ alkyl group), $R^4$ represents a hydrogen atom, $R^5$ represents a hydroxy group, and $R^6$ represents a $C_3$-$C_6$ cycloalkyl group.

**11.** An insecticidal method according to Claim 8, 9 or 10, wherein the insects harmful to the plants are insect pests belonging to Hemiptera.

**Patentansprüche**

**1.** Cyclohexan-Derivat, dargestellt durch die Formel (IA),

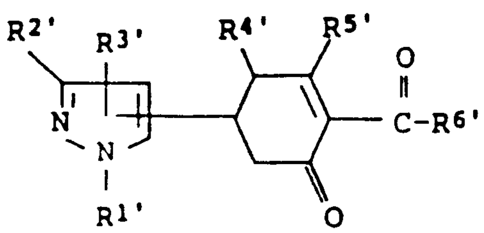

(IA)

wobei $R^{1'}$ ein Wasserstoffatom, eine Ethyl-Gruppe, n-Propyl-Gruppe, n-Butyl-Gruppe, Iso-Butyl-Gruppe oder Sec-Butyl-Gruppe darstellt, $R^{2'}$ ein Wasserstoffatom, Halogen-Atom oder eine $C_1$-$C_5$-Alkyl-Gruppe darstellt, $R^{3'}$ ein Halogen-Atom, eine $C_1$-$C_5$-Alkyl-Gruppe, $C_1$-$C_5$-Alkoxy-Gruppe, $C_1$-$C_5$-Alkyl-Thio-Gruppe, $C_1$-$C_5$-Alkylsulfinyl-Gruppe, $C_1$-$C_5$-Alkylsulfonyl-Gruppe oder -N($R^{7'}$)$R^{8'}$ (wobei $R^{7'}$ und $R^{8'}$, die gleich oder verschieden sein können, je ein Wasserstoffatom oder eine $C_1$-$C_3$-Alkyl-Gruppe und $R^{7'}$ und $R^{8'}$ zu 4 oder 6 Methylen-Gruppen gebunden sein können) darstellt, $R^{4'}$ ein Wasserstoff-Atom, $C_1$-$C_5$ eine Alkyl-Gruppe oder $C_3$-$C_5$-Alkoxycarbonyl-Gruppe darstellt, $R^{5'}$ eine Hydroxy-Gruppe, Phenylcarbonyloxy-Gruppe oder Phenylcarbonyloxy-Gruppe, deren Phenylring 1 bis 5 Substituenten enthält, die gleich oder verschieden sein können, ausgewählt aus der Gruppe bestehend aus einem Halogen-Atom, einer $C_1$-$C_3$-Alkyl-Gruppe und einer $C_1$-$C_3$-Alkoxy-Gruppe darstellt, $R^{6'}$ eine $C_1$-$C_5$-Alkyl-Gruppe, $C_3$-$C_6$ Cycloalkyl-Gruppe oder durch eine $C_1$-$C_3$-Alkyl-Gruppe substituierte Cycloalkyl-Gruppe darstellt; unter der Bedingung dass wenn $R^{1'}$ eine Ethyl-Gruppe darstellt, $R^{2'}$ eine Methyl-Gruppe darstellt, $R^{3'}$ ein Halogen-Atom oder eine Methylthio-Gruppe darstellt, $R^{4'}$ ein Wasserstoff-Atom und gleichzeitig $R^{5'}$ eine Hydroxy-Gruppe darstellt, dann ist $R^{6'}$ ein anderer Substituent als ein Ethyl und n-Propyl-Gruppen oder deren Salze.

2. Cyclohexan-Derivat nach Anspruch 1, wobei $R^{1'}$ eine Ethyl- oder n-Propyl-Gruppe darstellt, $R^{2'}$ ein Wasserstoff-Atom, Halogen-Atom oder eine $C_1$-$C_5$-Alkyl-Gruppe darstellt, $R^{3'}$ eine $C_1$-$C_5$-Alkyl-Gruppe, $C_1$-$C_5$-Alkylthio-Gruppe, $C_1$-$C_5$-Alkylsulfnyl-Gruppe, $C_1$-$C_5$ Alkylsulfonyl-Gruppe, eine C1-C3-Alkyl-Gruppe istoder-$N(R^{7'})R^{8'}$ (wobei $R^{7'}$ und $R^{8'}$, die gleich oder verschieden sein können, je ein Wasserstoffatom sind) darstellt, $R^{4'}$ ein Wasserstoff-Atom darstellt, $R^{5'}$ eine Hydroxy-Gruppe darstellt und $R^{6'}$ eine $C_1$-$C_5$-Alkyl-Gruppe, $C_3$-$C_6$ Cycloalkyl-Gruppe oder durch eine $C_1$-$C_3$-Alkyl-Gruppe substituierte Cycloalkyl-Gruppe darstellt; unter der Bedingund dass wenn $R^{1'}$ eine Ethyl-Gruppe darstellt $R^{2'}$ eine Methyl-Gruppe darstellt, $R^{3'}$ eine Methylthio-Gruppe darstellt, $R^{4'}$ ein Wasserstoff-Atom darstellt und gleichzeitig $R^{5'}$ eine Hydroxy-Gruppe darstellt, dann ist $R^{6'}$ ein anderer Substituent als ein Ethyl und n-Propyl-Gruppen oder deren Salze.

3. Verfahren zur Herstellung eine Cyclohexan-Derivats, dargestellt durch die Formel,

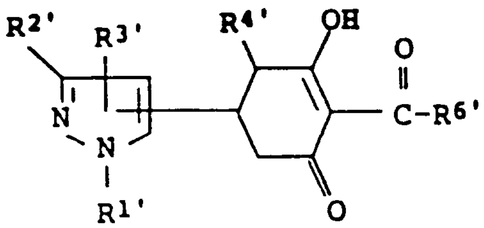

wobei $R^{1'}$ ein Wasserstoff-Atom, eine Ethyl-Gruppe, n-Propyl-Gruppe, n-Butyl-Gruppe, Iso-Butyl-Gruppe oder Sec-Butyl-Gruppe darstellt, $R^{2'}$ ein Wasserstoffatom, Halogen-Atom oder eine $C_1$-$C_5$-Alkyl-Gruppe darstellt, $R^{3'}$ ein Halogen-Atom, eine $C_1$-$C_5$-Alkyl-Gruppe, $C_1$-$C_5$-Alkoxy-Gruppe, $C_1$-$C_5$-Alkylthio-Gruppe, $C_1$-$C_5$-Alkylsulfinyl-Gruppe, $C_1$-$C_5$-Alkylsulfonyl-Gruppe oder -$N(R^{7'})R^{8'}$ (wobei $R^{7'}$ und $R^{8'}$, die gleich oder verschieden sein können, je ein Wasserstoffatom oder eine $C_1$-$C_3$-Alkyl-Gruppe und $R^{7'}$ und $R^{8'}$ zu 4 oder 6 Methylen-Gruppen gebunden sein können) darstellt, $R^{4'}$ ein Wasserstoff-Atom, eine $C_1$-$C_5$ Alkyl-Gruppe oder $C_3$-$C_5$-Alkoxycarbonyl-Gruppe darstellt, $R^{6'}$ eine $C_1$-$C_5$-Alkyl-Gruppe, $C_3$-$C_6$ Cycloalkyl-Gruppe oder durch eine $C_1$-$C_3$-Alkyl-Gruppe substituierte Cycloalkyl-Gruppe darstellt; unter der Bedingung dass wenn $R^{1'}$ eine Ethyl-Gruppe, $R^{2'}$ eine Methyl-Gruppe, $R^{3'}$ ein Halogen-Atom oder eine Methylthio-Gruppe und $R^{4'}$ ein Wasserstoff-Atom darstellt, dann ist $R^{6'}$ ein anderer Substituent als ein Ethyl und n-Propyl-Gruppen oder deren Salze, bei dem eine Cyclohexan-Verbindung, dargestellt durch die Formel,

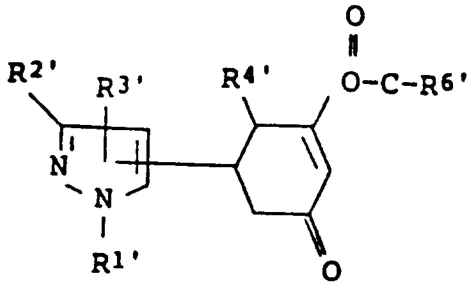

wobei $R^{1'}$, $R^{2'}$, $R^{3'}$, $R^{4'}$ und $R^{6'}$ wie oben definiert sind, in Anwesenheit eines Katalysators einer Umbildungsreaktion unterzogen wird.

**4.** Einsatz als Wirkstoff eines Insektizids eines Cyclohexan-Derivats, dargestellt durch die Formel (IB)

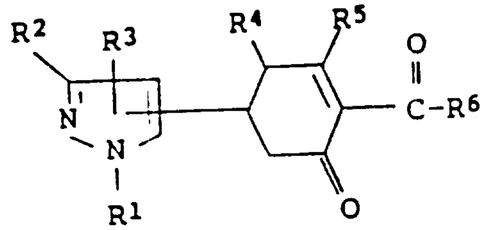

(IB)

wobei $R^{1'}$ ist ein Wasserstoffatom, eine $C_1$-$C_{16}$ Alkyl-Gruppe, $C_1$-$C_5$ Haloalkyl-Gruppe, $C_2$-$C_5$ Alkenyl-Gruppe, $C_2$-$C_5$ Alkoxyalkyl-Gruppe, Phenyl-Gruppe , Phenyl-Gruppe mit 1 bis 5 gleichen oder verschiedenen Substituenten, ausgewählt aus der Gruppe bestehend aus einem Halogen-Atom, einer $C_1$-$C_3$ Alkyl-Gruppe und $C_1$-$C_3$ Alkoxy-Gruppe, Benzyl-Gruppe oder Benzyl-Gruppe mit 1 bis 5 gleichen oder verschiedenen Substituenten, ausgewählt aus der Gruppe bestehend aus einem Wasserstoff-Atom, einer $C_1$-$C_3$ Alkyl-Gruppe und $C_1$-$C_3$ Alkoxy-Gruppe darstellt; $R^2$ ein Wasserstoff-Atom, Halogen-Atom, eine $C_1$-$C_5$ Alkyl-Gruppe, $C_1$-$C_5$ Haloalkyl-Gruppe, Phenyl-Gruppe oder Phenyl-Gruppe mit 1 bis 5 gleichen oder verschiedenen Substituenten, ausgewählt aus der Gruppe bestehend aus einem Halogen-Atom, einer $C_1$-$C_3$ Alkyl-Gruppe und $C_1$-$C_3$-Alkoxy-Gruppe darstellt; $R^3$ ein Wasserstoff-Atom, Halgoen-Atom, eine $C_1$-$C_5$ Alkyl-Gruppe, $C_1$-$C_5$ Haloalkyl-Gruppe, $C_1$-$C_5$ Alkoxy-Gruppe, $C_1$-$C_5$ Haloalkoxy-Gruppe, $C_1$-$C_5$ Alkylthio-Gruppe, $C_1$-$C_5$ Haloalkylthio-Gruppe, $C_1$-$C_5$ Alkylsulfinyl-Gruppe, $C_1$-$C_5$ Haloalkylsulfinyl-Gruppe, $C_1$-$C_5$ Alkylsulfonyl-Gruppe, $C_1$-$C_5$ Haloalkylsulfonyl-Gruppe, Phenoxy-Gruppe, Phenoxy-Gruppe mit 1 bis 5 gleichen oder verschiedenen Substituenten, ausgewählt aus der Gruppe bestehend aus einem Halogen-Atom, einer $C_1$-$C_3$ Alkyl-Gruppe und einer $C_1$-$C_3$ Alkoxy-Gruppe, Phenylthio-Gruppe, Phenylthio-Gruppe mit 1 bis 5 gleichen oder verschiedenen Substituenten, ausgewählt aus der Gruppe bestehend aus einem Halogen-Atom, aus einer $C_1$-$C_3$ Alkyl-Gruppe und $C_1$-$C_3$ Alkoxy-Gruppe, -N($R^7$)$R^8$ (wobei $R^7$ und $R^8$, die gleich oder verschieden sein können, je ein Wasserstoffatom oder eine $C_1$-$C_3$ Alkyl-Gruppe und $R^7$ und $R^8$ zu 4 bis 6 Methylen-Gruppen gebunden sein können) oder eine $C_2$-$C_5$ Alkylcarbonyloxy-Gruppe darstellt; $R^4$ ein Wasserstoff-Atom, eine $C_1$-$C_5$ Alkyl-Gruppe oder $C_2$-$C_5$ Alkoxycarbonyl-Gruppe darstellt, $R^5$ eine Hydroxy-Gruppe, ein Halogen-Atom, eine $C_1$-$C_5$ Alkylthio-Gruppe, Alkenylthio-Gruppe mit bis zu 5 Kohlenstoff-Atomen, -N($R^9$)$R^{10}$ (wobei $R^9$ und $R^{10}$, die gleich oder verschieden sein können, ein Wasserstoff-Atom, eine $C_1$-$C_3$ Alkyl-Gruppe oder Alkenyl-Gruppe mit bis zu 3 Kohlenstoffatomen sind, eine $C_2$-$C_8$ Alkylcarbonyloxy-Gruppe, $C_3$-$C_6$ Cycloalkylcarbonylory-Gruppe, Phenylcarbonyl-oxy-Gruppe oder Phenylcarbonyloxy-Gruppe deren Phenylring 1 bis 5 gleichen oder verschiedenen Substituenten hat, ausgewählt aus der Gruppe bestehend aus einem Halogen-Atom, einer $C_1$-$C_3$ Alkyl-Gruppe und $C_1$-$C_3$ Alkoxy-Gruppe darstellt,$R_6$ eine $C_1$-$C_{18}$-Alkyl-Gruppe, $C_3$-$C_6$ Cycloalkyl-Gruppe, Cycloalkyl-Gruppe mit gleichen oder verschiedenen Substituenten, ausgewählt aus der Gruppe bestehend aus einem Halogen-Atom und einer $C_1$-$C_3$ Alkyl-Gruppe, $C_2$-$C_{17}$ Alkenyl-Gruppe, $C_2$-$C_8$ Alkoxyalkyl-Gruppe, $C_2$-$C_8$ Alkylthioalkyl-Gruppe, Phenoxyalkyl-Gruppe oder Phenoxyalkyl-Gruppe, deren Phenylring 1 bis 5 gleiche oder verschiedene Substituenten hat, ausgewählt aus der Gruppe bestehend aus einem Halogen-Atom, einer $C_1$-$C_3$ Alkyl-Gruppe und $C_1$-$C_3$ Alkoxy-Gruppe darstellt unter der Bedingung dass wenn$R^1$ =H, $R^2$ = Methyl, $R^3$ = 5-Cl, $R^4$ = H und $R^5$ = OH, dann kann R6 Benzyl sein.- oder deren Salze;

**5.** Insektizid nach Anspruch 4, wobei in der Formel (IB) $R^1$ eine $C_1$-$C_5$ Alkyl-Gruppe, $C_1$-$C_5$ Haloalkyl-Gruppe oder $C_2$-$C_5$ Alkenyl-Gruppe darstellt, $R^2$ ein Wasserstoff-Atom, Halogen-Atom, eine $C_1$-$C_5$ Alkyl-Gruppe oder $C_1$-$C_5$ Haloalkyl-Gruppe darstellt, $R^3$ eine C1-$C_5$ Alkyl-Gruppe, $C_1$-$C_5$ Alkoxy-Gruppe, $C_1$-$C_5$ Alkylthio-Gruppe, $C_1$-$C_5$ Haloalkylthio-Gruppe, $C_1$-$C_5$ Alkylsulfinyl-Gruppe, $C_1$-$C_5$ Alkylsulfonyl-Gruppe oder -N($R^7$)$R^8$ (wobei $R^7$ und $R^8$, die gleich oder verschieden sein können, ein Wasserstoff-Atom oder eine $C_1$-$C_3$ Alkyl-Gruppe sind) darstellt, $R^4$ ein Wasserstoff-Atom darstellt, $R^5$ eine Hydroxy-Gruppe darstellt und $R^6$ eine $C_1$-$C_5$ Alkyl-Gruppe, $C_3$-$C_6$ Cycloalkyl-Gruppe oder Cycloalkyl-Gruppe mit Substituenten, die gleich oder verschieden sein können, ausgewählt aus der Gruppe bestehend aus einem Halogen-Atom und einer $C_1$-$C_3$ Alkyl-Gruppe, darstellt.

**6.** Insektizid nach Anspruch 5, wobei in der Formel (IB) gilt: $R^1$ ist eine $C_1$-$C_5$ Alkyl-Gruppe, $R^2$ eine $C_1$-$C_5$ Alkyl-Gruppe, $R^3$ eine $C_1$-$C_5$ Alkyl-Gruppe, $C_1$-$C_5$ Alkylthio-Gruppe, $C_1$-$C_5$ Alkylsulfinyl-Gruppe oder -N-

($R^7$) (wobei $R^7$ und $R^8$, die gleich oder verschieden sein können, ein Wasserstoff-Atom oder eine $C_1$-$C_3$ Alkyl-Gruppe ist), $R^4$ ein Wasserstoff-Atom, $R^5$ eine Hydroxy-Gruppe und $R^6$ eine $C_3$-$C_6$ Cycloalkyl-Gruppe.

**7.** Insektizid nach den Ansprüchen 4, 5 oder 6, das ein Insektizid für die Kontrolle des Befalls durchInsekten der Familie der Hemiptera ist.

**8.** Insektizid-Verfahren, das in der Anwendung eines Insektizids begreift, das als Wirkstoff ein Cyclohexan-Derivat enthält, dargestellt durch die Formel (IB),

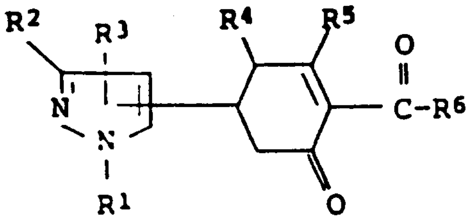

(IB)

wobei $R^1$ ein Wasserstoff-Atom, eine $C_1$-$C_{16}$ Alkyl-Gruppe, $C_1$-$C_5$ Haloalkyl-Gruppe, $C_2$-$C_5$ Alkenyl-Gruppe, $C_2$-$C_5$ Alkoxyalkyl-Gruppe, Phenyl-Gruppe, Phenyl-Gruppe mit 1 bis 5 Substituenten, die gleich oder verschieden sein können, ausgewählt aus der Gruppe bestehend aus einem Halogen-Atom, einer $C_1$-$C_3$ Alkyl-Gruppe und $C_1$-$C_3$ Alkoxy-Gruppe, Benzyl-Gruppe oder Benzyl-Gruppe mit 1 bis 5 Substituenten, die gleich oder verschieden sein können, ausgewählt aus der Gruppe bestehend aus einem Halogen-Atom, einer $C_1$-$C_3$ Alkyl-Gruppe und $C_1$-$C_3$ Alkoxy-Gruppe darstellt; $R^2$ ein Wasserstoff-Atom, Halogen-Atom, eine $C_1$-$C_5$ Alkyl-Gruppe, $C_1$-$C_5$ Haloalkyl-Gruppe, Phenyl-Gruppe oder Phenyl-Gruppe mit 1 bis 5 Substituenten, die gleich oder verschieden sein können, ausgewählt aus der Gruppe bestehend aus einem Halogen-Atom, einer $C_1$-$C_3$ Alkyl-Gruppe und $C_1$-$C_3$ Alkoxy-Gruppe darstellt; $R^3$ ein Wasserstoff-Atom, Halogen-Atom, eine $C_1$-$C_5$ Alkyl-Gruppe, $C_1$-$C_5$ Haloalkyl-Gruppe, $C_1$-$C_5$ Alkoxy-Gruppe, $C_1$-$C_5$ Haloalkoxy-Gruppe, $C_1$-$C_5$ Alkylthio-Gruppe, $C_1$-$C_5$ Haloalkylthio-Gruppe, $C_1$-$C_5$ Alkylsulfinyl-Gruppe, $C_1$-$C_5$ Haloalkylsulfinyl-Gruppe, $C_1$-$C_5$ Alkylsulfonyl-Gruppe, $C_1$-$C_5$ Haloalkylsulfonyl-Gruppe, Phenoxy-Gruppe, Phenoxy-Gruppe mit 1 bis 5 Substituenten, die gleich oder verschieden sein können, ausgewählt aus der Gruppe bestehend aus einem Halogen-Atom, einer $C_1$-$C_3$ Alkyl-Gruppe und $C_1$-$C_3$ Alkoxy-Gruppe, Phenylthio-Gruppe, Phenylthio-Gruppe mit 1 bis 5 Substituenten, die gleich oder verschieden sein können, ausgewählt aus der Gruppe bestehend aus einem Halogen-Atom, einer $C_1$-$C_3$ Alkyl-Gruppe und $C_1$-$C_3$ Alkoxy-Gruppe, - $N(R^7)R^8$ (wobei $R^7$ und $R^8$, die gleich oder verschieden sein können, ein Wasserstoff-Atom oder eine $C_1$-$C_3$ Alkyl-Gruppe sind und $R^7$ und $R^8$ zu 4 bis 6 Methylen-Gruppen gebunden sein können), oder eine $C_2$-$C_5$ Alkylcarbonyloxy-Gruppe darstellt; $R^4$ ein Wasserstoff-Atom, eine $C_1$-$C_5$ Alkyl-Gruppe oder $C_2$-$C_5$ Alkoxycarbonyl-Gruppe darstellt, $R^5$ eine Hydroxy-Gruppe, ein Halogen-Atom, eine $C_1$-$C_5$ Alkylthio-Grupp, Alkenylthio-Gruppe mit bis zu 5 Kohlenstoffatomen, - $N(R^9)R^{10}$ (wobei $R^9$ und $R^{10}$, die gleich oder verschieden sein können, je ein Wasserstoff-Atom, eine $C_1$-$C_3$ Alkyl-Gruppe oder Alkenyl-Gruppe mit bis zu 3 Kohlenstoff-Atomen sind), eine $C_2$-$C_8$ Alkylcarbonyloxy-Gruppe, $C_3$-$C_6$ Cycloalkylcarbonyloxy-Gruppe, Phenylcarbonyloxy-Gruppe oder Phenylcarbonyloxy-Gruppe, deren Phenylring 1 bis 5 Substituenten hat, die gleich oder verschieden sein können, ausgewählt aus der Gruppe bestehend aus einem Halogen-Atom, einer $C_1$-$C_3$ Alkyl-Gruppe und $C_1$-$C_3$ Alkoxy-Gruppe darstellt; $R^6$ eine $C_1$-$C_{18}$ Alkyl-Gruppe, $C_3$-$C_6$ Cycloalkyl-Gruppe, Cycloalkyl-Gruppe mit Substituenten, die gleich oder verschieden sein können, ausgewählt aus der Gruppe bestehend aus einem Halogen-Atom und einer $C_1$-$C_3$ Alkyl-Gruppe, $C_2$-$C_{17}$ Alkenyl-Gruppe, $C_2$-$C_8$ Alkoxyalkyl-Gruppe, $C_2$-$C_8$ Alkylthioalkyl-Gruppe, Phenoxyalkyl-Gruppe oder einer Phenoxyalkyl-Gruppe, deren Phenylring 1 bis 5 Substituenten hat, die gleich oder verschieden sein können, ausgewählt aus der Gruppe bestehend aus einem Halogen-Atom, einer $C_1$-$C_3$ Alkyl-Gruppe und $C_1$-$C_3$ Alkoxy-Gruppe darstellt; unter der Bedingung dass wenn $R^1$ = H, $R^2$ = Methyl, $R^3$ = 5-Cl, $R^4$ = H und $R^5$ = OH, dann kann $R^6$ Benzyl sein - oder dessen Salze, angewandt auf Pflanzen, an denen pflanzenschädigende Insekten entstanden sind oder voraussichtlich entstehen werden, oder aber um Umfeld von Pflanzen in Mengen von 1 g bis 3 kg je 10 Ar des Wirkstoffes.

**9.** Insektizid-Verfahren nach Anspruch 8, wobei in der Formel (IB) $R^1$ eine $C_1$-$C_5$ Alkyl-Gruppe, $C_1$-$C_5$ Haloalkyl-Gruppe darstellt, $R^2$ ein Wasserstoff-Atom, Halogen-Atom, eine $C_1$-$C_5$ Alkyl-Gruppe oder $C_1$-$C_5$ Haloalkyl-Gruppe darstellt, $R^3$ eine $C_1$-$C_5$ Alkyl-Gruppe, $C_1$-$C_5$ Alkoxy-Gruppe, $C_1$-$C_5$ Alkylthio-Gruppe, $C_1$-$C_5$ Haloalkylthio-Gruppe, $C_1$-$C_5$ Alkylsulfinyl-Gruppe, $C_1$-$C_5$ Alkylsulfonyl-Gruppe oder -N-($R^7$)$R^8$ (wobei $R^7$ und $R^8$, die gleich oder verschieden sein können, ein Wasserstoff-Atom oder eine $C_1$-$C_3$ Alkyl-Gruppe sind) darstellt, $R^4$ ein Wasserstoff-Atom darstellt, $R^5$ eine Hydroxy-Gruppe darstellt und $R^6$ eine $C_1$-$C_5$ Alkyl-Gruppe, $C_3$-$C_6$ Cycloalkyl-Gruppe oder Cycloalkyl-Gruppe mit Substituenten, die gleich oder verschieden sein können, ausgewählt aus der Gruppe bestehend aus einem Halogen-Atom und einer $C_1$-$C_3$ Alkyl-Gruppe darstellt.

**10.** Insektizid-Verfahren nach Anspruch 9, wobei in der Formel (IB) $R^1$ ist eine $C_1$-$C_5$ Alkyl-Gruppe darstellt, $R^2$ eine $C_1$-$C_5$ Alkyl-Gruppe darstellt, $R^3$ eine $C_1$-$C_5$ Alkyl-Gruppe, $C_1$-$C_5$ Alkylthio-Gruppe, $C_1$-$C_5$ Alkylsulfonyl-Gruppe oder - N($R^7$)$R^8$ (wobei $R^7$ und $R^8$, die gleich oder verschieden sein können, ein Wasserstoff-Atom oder eine $C_1$-$C_3$ Alkyl-Gruppe sind) darstellt, $R^4$ ein Wasserstoff-Atom darstellt, $R^5$ eine Hydroxy-Gruppe darstellt und $R^6$ eine $C_3$-$C_6$ Cyoloalkyl-Gruppe darstellt.

**11.** Insektizid-Verfahren nach Anspruch 8, 9 oder 10, wobei die pflanzenschädigen Insekten Schädlinge der Familie Hemiptera sind.

## Revendications

**1.** Dérivé de cyclohexane représenté par la formule (IA)

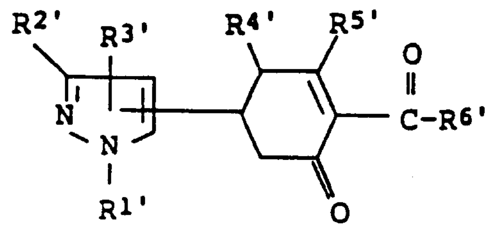

dans laquelle $R^{1'}$ représente un atome d'hydrogène, un groupe éthyle, un groupe n-propyle, un groupe n-butyle, un groupe isobutyle ou un groupe sec.-butyle, $R^{2'}$ représente un atome d'hydrogène, un atome d'halogène ou un groupe alkyle en $C_1$-$C_5$, $R^{3'}$ représente un atome d'halogène, un groupe alkyle en $C_1$-$C_5$, un groupe alcoxy en $C_1$-$C_5$, un groupe alkyl(en $C_1$-$C_5$)thio, un groupe alkyl(en $C_1$-$C_5$)-sulfynyle, un groupe alkyl(en $C_1$-$C_5$)sulfonyle ou encore -N($R^{7'}$)$R^{8'}$ (où chacun des radicaux $R^{7'}$ et $R^{8'}$, qui peuvent être identiques ou différents, représente un atome d'hydrogène ou un groupe alkyle en $C_1$-$C_3$, et $R^{7'}$ et $R^{8'}$ peuvent être liés l'un à l'autre pour former 4 à 6 groupes méthylène), $R^{4'}$ représente un atome d'hydrogène, un groupe alkyle en $C_1$-$C_5$ ou un groupe alcoxycarbonyle en $C_3$-$C_5$, $R^{5'}$ représente un groupe hydroxyle, un groupe phénylcarbonyloxy ou encore un groupe phénylcarbonyloxy dont le noyau phényle possède de 1 à 5 substituants, qui peuvent être identiques ou différents, choisis parmi le groupe constitué par un atome d'halogène, par un groupe alkyle en $C_1$-$C_3$ et par un groupe alcoxy-(en $C_1$-$C_3$), et $R^{6'}$ représente un groupe alkyle en $C_1$-$C_5$, un groupe cycloalkyle en $C_3$-$C_6$ ou encore un groupe cycloalkyle substitué par un groupe alkyle en $C_1$-$C_3$, à condition que, lorsque $R^{1'}$ représente un groupe éthyle, $R^{2'}$ représente un groupe méthyle, $R^{3'}$ représente un atome d'halogène ou un groupe méthylthio, $R^{4'}$ représente un atome d'hydrogène et $R^{5'}$ représente un groupe hydroxyle en même temps, $R^{6'}$ est un substituant différent d'un groupe éthyle et d'un groupe n-propyle, ou encore ses sels.

**2.** Dérivé de cyclohexane selon la revendication 1, dans lequel $R^{1'}$ représente un groupe éthyle ou un groupe n-propyle, $R^{2'}$ représente un atome d'hydrogène, un atome d'halogène ou un groupe alkyle en $C_1$-$C_5$, $R^{3'}$ représente un groupe alkyle en $C_1$-$C_5$, un groupe alkyl(en $C_1$-$C_5$)thio, un groupe alkyl(en $C_1$-$C_5$)sulfynyle, un groupe alkyl(en $C_1$-$C_5$)sulfonyle ou encore -N($R^{7'}$)$R^{8'}$ (où chacun des radicaux $R^{7'}$ et $R^{8'}$, qui peuvent être identiques ou différents, représente un atome d'hydrogène ou un groupe alkyle en $C_1$-$C_3$),$R^{4'}$ représente un atome d' hydrogène, $R^{5'}$ représente un groupe hydroxyle et $R^{6'}$ représente un groupe alkyle en $C_1$-$C_5$, un groupe cycloalkyle en $C_3$-$C_6$ ou encore un groupe cycloalkyle substitué par un groupe alkyle en $C_1$-$C_3$, à condition que, lorsque $R^{1'}$ représente un groupe éthyle, $R^{2'}$ représente un

groupe méthyle, $R^{3'}$ représente un groupe méthylthio, $R^{4'}$ représente un atome d'hydrogène et $R^{5'}$ représente un groupe hydroxyle en même temps, $R^{6'}$ est un substituant différent d'un groupe éthyle et d'un groupe n-propyle, ou ses sels.

**3.** Procédé pour préparer un dérivé de cyclohexane représenté par la formule

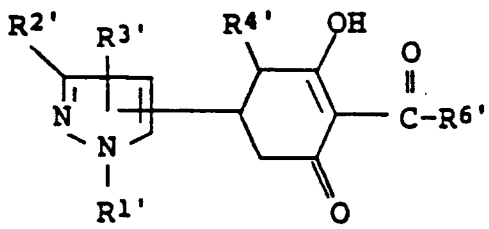

dans laquelle $R^{1'}$ représente un atome d'hydrogène, un groupe éthyle, un groupe n-propyle, un groupe n-butyle, un groupe isobutyle ou un groupe sec.-butyle, $R^{2'}$ représente un atome d'hydrogène, un atome d'halogène ou un groupe alkyle en $C_1$-$C_5$, $R^{3'}$ représente un atome d'halogène, un groupe alkyle en $C_1$-$C_5$, un groupe alcoxy en $C_1$-$C_5$, un groupe alkyl(en $C_1$-$C_5$)thio, un groupe alkyl(en $C_1$-$C_5$)-sulfynyle, un groupe alkyl(en $C_1$-$C_5$)sulfonyle ou encore -N($R^{7'}$)$R^{8'}$ (où chacun des radicaux $R^{7'}$ et $R^{8'}$, qui peuvent être identiques ou différents, représente un atome d'hydrogène ou un groupe alkyle en $C_1$-$C_3$, et $R^{7'}$ et $R^{8'}$ peuvent être liés l'un à l'autre pour former 4 à 6 groupes méthylène), $R^{4'}$ représente un atome d'hydrogène, un groupe alkyle en $C_1$-$C_5$ ou un groupe alcoxycarbonyle en $C_2$-$C_5$, $R^{6'}$ représente un groupe alkyle en $C_1$-$C_5$, un groupe cycloalkyle en $C_3$-$C_6$ ou encore un groupe cycloalkyle substitué par un groupe alkyle en $C_1$-$C_3$, à condition que, lorsque $R^{1'}$ représente un groupe éthyle, $R^{2'}$ représente un groupe méthyle, $R^{3'}$ représente un atome d'halogène ou un groupe méthylthio et $R^{4'}$ représente un atome d'hydrogène en même temps, $R^{6'}$ est un substituant différent d'un groupe éthyle et d'un groupe n-propyle, ou ses sels, qui comprend le fait de soumettre un composé de cyclohexane représenté par la formule

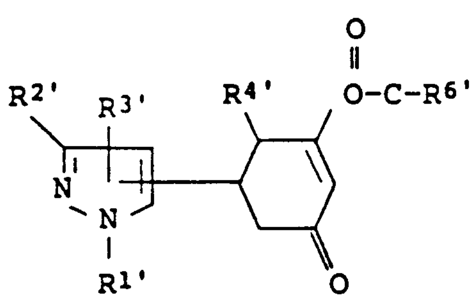

dans laquelle $R^{1'}$, $R^{2'}$, $R^{3'}$, $R^{4'}$ et $R^{6'}$ sont tels que définis ci-dessus, à une réaction de réarrangement en présence d'un catalyseur.

**4.** Utilisation, comme ingrédient actif d'un insecticide, d'un dérivé de cyclohexane représenté par la formule (IB)

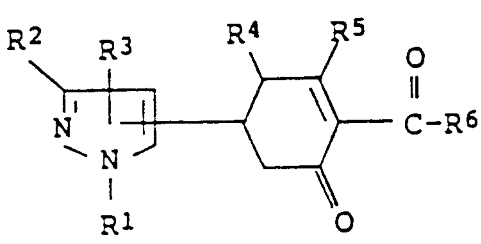

(IB)

dans laquelle $R^1$ représente un atome d'hydrogène, un groupe alkyle en $C_1$-$C_{16}$, un groupe halogénalkyle en $C_1$-$C_5$, un groupe alcényle en $C_2$-$C_5$, un groupe alcoxyalkyle en $C_2$-$C_5$, un groupe phényle, un groupe phényle possédant de 1 à 5 substituants, qui peuvent être identiques ou différents, choisis parmi le groupe constitué par un atome d'halogène, par un groupe alkyle en $C_1$-$C_3$ et par un groupe alcoxy en $C_1$-$C_3$, un groupe benzyle ou un groupe benzyle possédant de 1 à 5 substituants, qui peuvent être identiques ou différents, choisis parmi le groupe constitué par un atome d'halogène, par un groupe alkyle en $C_1$-$C_3$ et par un groupe alcoxy en $C_1$-$C_3$, $R^2$ représente un atome d'hydrogène, un atome d'halogène, un groupe alkyle en $C_1$-$C_5$, un groupe halogénalkyle en $C_1$-$C_5$, un groupe phényle ou un groupe phényle possédant de 1 à 5 substituants, qui peuvent être identiques ou différents, choisis parmi le groupe constitué par un atome d'halogène, par un groupe alkyle en $C_1$-$C_3$ et par un groupe alcoxy en $C_1$-$C_3$, $R^3$ représente un atome d'hydrogène, un atome d'halogène, un groupe alkyle en $C_1$-$C_5$, un groupe halogénalkyle en $C_1$-$C_5$, un groupe alcoxy en $C_1$-$C_5$, un groupe halogénalcoxy en $C_1$-$C_5$, un groupe alkyl(en $C_1$-$C_5$)thio, un groupe halogénalkyl(en $C_1$-$C_5$)thio, un groupe alkyl(en $C_1$-$C_5$)-sulfynyle, un groupe halogénalkyl(en $C_1$-$C_5$)sulfynyle, un groupe alkyl(en $C_1$-$C_5$)sulfonyle, un groupe halogénalkyl(en $C_1$-$C_5$)sulfonyle, un groupe phénoxy, un groupe phénoxy possédant de 1 à 5 substituants, qui peuvent être identiques ou différents, choisis parmi le groupe constitué par un atome d'halogène, par un groupe alkyle en $C_1$-$C_3$ et par un groupe alcoxy en $C_1$-$C_3$, un groupe phénylthio, un groupe phénylthio possédant de 1 à 5 substituants, qui peuvent être identiques ou différents, choisis parmi le groupe constitué par un atome d'halogène, par un groupe alkyle en $C_1$-$C_3$ et par un groupe alcoxy en $C_1$-$C_3$, $-N(R^7)R^8$ (où chacun des radicaux $R^7$ et $R^8$, qui peuvent être identiques ou différents, représente un atome d'hydrogène ou un groupe alkyle en $C_1$-$C_3$, et $R^7$ et $R^8$ peuvent être liés l'un à l'autre pour former 4 à 6 groupes méthylène), ou encore un groupe alkyl (en $C_2$-$C_5$) carbonyloxy, $R^4$ représente un atome d'hydrogène, un groupe alkyle en $C_1$-$C_5$ ou un groupe alcoxy(en $C_2$-$C_5$)carbonyle, $R^5$ représente un groupe hydroxyle, un atome d'halogène, un groupe alkyl(en $C_1$-$C_5$)thio, un groupe alcénylthio contenant jusqu'à 5 atomes de carbone, $-N(R^9)R^{10}$ (où chacun des radicaux $R^9$ et $R^{10}$, qui peuvent être identiques ou différents, représente un atome d'hydrogène, un groupe alkyle en $C_1$-$C_3$ ou encore un groupe alcényle contenant jusqu'à 3 atomes de carbone), un groupe alkyl(en $C_2$-$C_8$)-carbonyloxy, un groupe cycloalkyl(en $C_3$-$C_6$)carbonyloxy, un groupe phénylcarbonyloxy ou encore un groupe phénylcarbonyloxy dont le noyau phényle possède de 1 à 5 substituants, qui peuvent être identiques ou différents, choisis parmi le groupe constitué par un atome d'halogène, par un groupe alkyle en $C_1$-$C_3$ et par un groupe alcoxy en $C_1$-$C_3$, et $R^6$ représente un groupe alkyle en $C_1$-$C_{18}$, un groupe cycloalkyle en $C_3$-$C_6$, un groupe cycloalkyle possédant des substituants, qui peuvent être identiques ou différents, choisis parmi le groupe constitué par un atome d'halogène et par un groupe alkyle en $C_1$-$C_3$, un groupe alcényle en $C_2$-$C_{17}$, un groupe alcoxyalkyle en $C_2$-$C_8$, un groupe alkylthioalkyle en $C_2$-$C_8$, un groupe phénoxyalkyle ou un groupe phénoxyalkyle dont le noyau phényle possède de 1 à 5 substituants, qui peuvent être identiques ou différents, choisis parmi le groupe constitué par un atome d'halogène, un groupe alkyle en $C_1$-$C_3$ et un groupe alcoxy en $C_1$-$C_3$ à condition que, lorsque $R^1$ représente un atome d'hydrogène, $R^2$ représente un groupe méthyle, $R^3$ représente 5-Cl, $R^4$ représente un atome d'hydrogène et $R^5$ représente OH, alors $R^6$ peut représenter un groupe benzyle, ou de ses sels.

**5.** Insecticide selon la revendication 4, dans lequel, dans la formule (IB), $R^1$ représente un groupe alkyle en $C_1$-$C_5$, un groupe halogénalkyle en $C_1$-$C_5$ ou un groupe alcényle en $C_2$-$C_5$, $R^2$ représente un atome d'hydrogène, un atome d'halogène, un groupe alkyle en $C_1$-$C_5$ ou un groupe halogénalkyle en $C_1$-$C_5$, $R^3$ représente un groupe alkyle en $C_1$-$C_5$, un groupe alcoxy en $C_1$-$C_5$, un groupe alkyl(en $C_1$-$C_5$)thio, un groupe halogénalkyl(en $C_1$-$C_5$)thio, un groupe alkyl(en $C_1$-$C_5$)sulfynyle, un groupe alkyl(en $C_1$-$C_5$)-sulfonyle ou $-N(R^7)R^8$ (où chacun des radicaux $R^7$ et $R^8$, qui peuvent être identiques ou différents, représente un atome d'hydrogène ou un groupe alkyle en $C_1$-$C_3$), $R^4$ représente un atome d'hydrogène, $R^5$ représente un groupe hydroxyle et $R^6$ représente un groupe alkyle en $C_1$-$C_5$, un groupe cycloalkyle en $C_3$-$C_6$ ou un groupe cycloalkyle possédant des substituants, qui peuvent être identiques ou différents, choisis parmi le groupe constitué par un atome d'halogène et par un groupe alkyle en $C_1$-$C_3$.

**6.** Insecticide selon la revendication 5, dans lequel, dans la formule (IB), $R^1$ représente un groupe alkyle en $C_1$-$C_5$, $R^2$ représente un groupe alkyle en $C_1$-$C_5$, $R^3$ représente un groupe alkyle en $C_1$-$C_5$, un groupe alkyl(en $C_1$-$C_5$)thio, un groupe alkyl(en $C_1$-$C_5$)sulfynyle ou $-N(R^7)R^8$ (où chacun des radicaux $R^7$ et $R^8$, qui peuvent être identiques ou différents, représente un atome d'hydrogène, un groupe alkyle en $C_1$-$C_3$), $R^4$ représente un atome d'hydrogène, $R^5$ représente un groupe hydroxyle et $R^6$ représente

un groupe cycloalkyle en $C_3$-$C_6$.

7. Insecticide selon la revendication 4, 5 ou 6, à savoir un insecticide pour lutter contre les insectes nuisibles appartenant aux Hémiptères.

8. Procédé insecticide qui comprend le fait d'appliquer un insecticide contenant, comme ingrédient actif, un dérivé de cyclohexane représenté par la formule (IB)

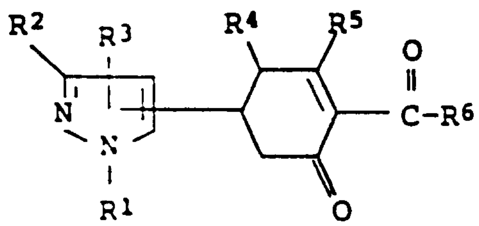

(IB)

dans laquelle $R^1$ représente un atome d'hydrogène, un groupe alkyle en $C_1$-$C_{16}$, un groupe halogénalkyle en $C_1$-$C_5$, un groupe alcényle en $C_2$-$C_5$, un groupe alcoxyalkyle en $C_2$-$C_5$, un groupe phényle, un groupe phényle possédant de 1 à 5 substituants, qui peuvent être identiques ou différents, choisis parmi le groupe constitué par un atome d'halogène, par un groupe alkyle en $C_1$-$C_3$ et par un groupe alcoxy en $C_1$-$C_3$, un groupe benzyle ou un groupe benzyle possédant de 1 à 5 substituants, qui peuvent être identiques ou différents, choisis parmi le groupe constitué par un atome d'halogène, par un groupe alkyle en $C_1$-$C_3$ et par un groupe alcoxy en $C_1$-$C_3$, $R^2$ représente un atome d'hydrogène, un atome d'halogène, un groupe alkyle en $C_1$-$C_5$, un groupe halogénalkyle en $C_1$-$C_5$, un groupe phényle ou un groupe phényle possédant de 1 à 5 substituants, qui peuvent être identiques ou différents, choisis parmi le groupe constitué par un atome d'halogène, par un groupe alkyle en $C_1$-$C_3$ et par un groupe alcoxy en $C_1$-$C_3$, $R^3$ représente un atome d'hydrogène, un atome d'halogène, un groupe alkyle en $C_1$-$C_5$, un groupe halogénalkyle en $C_1$-$C_5$, un groupe alcoxy en $C_1$-$C_5$, un groupe halogénalcoxy en $C_1$-$C_5$, un groupe alkyl(en $C_1$-$C_5$)thio, un groupe halogénalkyl(en $C_1$-$C_5$)thio, un groupe alkyl(en $C_1$-$C_5$)-sulfynyle, un groupe halogénalkyl(en $C_1$-$C_5$)sulfynyle, un groupe alkyl(en $C_1$-$C_5$)sulfonyle, un groupe halogénalkyl(en $C_1$-$C_5$)sulfonyle, un groupe phénoxy, un groupe phénoxy possédant de 1 à 5 substituants, qui peuvent être identiques ou différents, choisis parmi le groupe constitué par un atome d'halogène, par un groupe alkyle en $C_1$-$C_3$ et par un groupe alcoxy en $C_1$-$C_3$, un groupe phénylthio, un groupe phénylthio possédant de 1 à 5 substituants, qui peuvent être identiques ou différents, choisis parmi le groupe constitué par un atome d'halogène, par un groupe alkyle en $C_1$-$C_3$ et par un groupe alcoxy en $C_1$-$C_3$, $-N(R^7)R^8$ (où chacun des radicaux $R^7$ et $R^8$, qui peuvent être identiques ou différents, représente un atome d'hydrogène ou un groupe alkyle en $C_1$-$C_3$, et $R^7$ et $R^8$ peuvent être liés l'un à l'autre pour former 4 à 6 groupes méthylène), ou encore un groupe alkyl (en $C_1$-$C_5$) carbonyloxy, $R^4$ représente un atome d'hydrogène, un groupe alkyle en $C_1$-$C_5$ ou un groupe alcoxy(en $C_2$-$C_5$)carbonyle, $R^5$ représente un groupe hydroxyle, un atome d'halogène, un groupe alkyl(en $C_1$-$C_5$)thio, un groupe alcénylthio contenant jusqu'à 5 atomes de carbone, $-N(R^9)R^{10}$ (où chacun des radicaux $R^9$ et $R^{10}$, qui peuvent être identiques ou différents, représente un atome d'hydrogène, un groupe alkyle en $C_1$-$C_3$ ou encore un groupe alcényle contenant jusqu'à 3 atomes de carbone), un groupe alkyl(en $C_2$-$C_8$)-carbonyloxy, un groupe cycloalkyl(en $C_3$-$C_6$)carbonyloxy, un groupe phénylcarbonyloxy ou encore un groupe phénylcarbonyloxy dont le noyau phényle possède de 1 à 5 substituants, qui peuvent être identiques ou différents, choisis parmi le groupe constitué par un atome d'halogène, par un groupe alkyle en $C_1$-$C_3$ et par un groupe alcoxy en $C_1$-$C_3$, et $R^6$ représente un groupe alkyle en $C_1$-$C_{18}$, un groupe cycloalkyle en $C_3$-$C_6$, un groupe cycloalkyle possédant des substituants, qui peuvent être identiques ou différents, choisis parmi le groupe constitué par un atome d'halogène et par un groupe alkyle en $C_1$-$C_3$, un groupe alcényle en $C_2$-$C_{17}$, un groupe alcoxyalkyle en $C_2$-$C_8$, un groupe alkylthioalkyle en $C_2$-$C_8$, un groupe phénoxyalkyle ou un groupe phénoxyalkyle dont le noyau phényle possède de 1 à 5 substituants, qui peuvent être identiques ou différents, choisis parmi le groupe constitué par un atome d'halogène, par un groupe alkyle en $C_1$-$C_3$ et par un groupe alcoxy en $C_1$-$C_3$ à condition que, lorsque $R^1$ représente un atome d'hydrogène, $R^2$ représente un groupe méthyle, $R^3$ représente 5-Cl, $R^4$ représente un atome d'hydrogène et $R^5$ représente OH, alors $R^6$ peut représenter un groupe benzyle, ou ses sels, sur des plantes sur lesquelles les insectes nuisibles pour les plantes

se sont reproduits ou sur lesquelles on prévoit une reproduction des insectes, ou encore sur des endroits autour des plantes, en une quantité se situant dans le domaine de 1 g à 3 kg par 10 ares, comme quantité de l'ingrédient actif.

**9.** Procédé insecticide selon la revendication 8, dans lequel, dans la formule (IB), $R^1$ représente un groupe alkyle en $C_1$-$C_5$, un groupe halogénalkyle en $C_1$-$C_5$ ou $R^2$ représente un atome d'hydrogène, un atome d'halogène, un groupe alkyle en $C_1$-$C_5$ ou un groupe halogénalkyle en $C_1$-$C_5$, $R^3$ représente un groupe alkyle en $C_1$-$C_5$, un groupe alcoxy en $C_1$-$C_5$, un groupe alkyl(en $C_1$-$C_5$)thio, un groupe halogénalkyl(en $C_1$-$C_5$)thio, un groupe alkyl(en $C_1$-$C_5$)sulfynyle, un groupe alkyl(en $C_1$-$C_5$)sulfonyle ou -N($R^7$)$R^8$ (où chacun des radicaux $R^7$ et $R^8$, qui peuvent être identiques ou différents, représente un atome d'hydrogène ou un groupe alkyle en $C_1$-$C_3$),$R^4$ représente un atome d'hydrogène, $R^5$ représente un groupe hydroxyle et $R^6$ représente un groupe alkyle en $C_1$-$C_5$, un groupe cycloalkyle en $C_3$-$C_6$ ou un groupe cycloalkyle possédant des substituants, qui peuvent être identiques ou différents, choisis parmi le groupe constitué par un atome d'halogène et par un groupe alkyle en $C_1$-$C_3$.

**10.** Procédé insecticide selon la revendication 9, dans lequel, dans la formule (IB), $R^1$ représente un groupe alkyle en $C_1$-$C_5$, $R^2$ représente un groupe alkyle en $C_1$-$C_5$, $R^3$ représente un groupe alkyle en $C_1$-$C_5$, un groupe alkyl(en $C_1$-$C_5$)thio, un groupe alkyl(en $C_1$-$C_5$)sulfonyle ou -N($R^7$)$R^8$ (où chacun des radicaux $R^7$ et $R^8$, qui peuvent être identiques ou différents, représente un atome d'hydrogène ou un groupe alkyle en $C_1$-$C_3$), $R^4$ représente un atome d'hydrogène, $R^5$ représente un groupe hydroxyle et $R^6$ représente un groupe cycloalkyle en $C_3$-$C_6$.

**11.** Procédé insecticide selon la revendication 8, 9 ou 10, dans lequel les insectes nuisibles pour les plantes sont des insectes nuisibles appartenant aux Hémiptères.